# EUROPEAN PATENT SPECIFICATION

(11) **EP 1 075 467 B1**
(45) Date of publication and mention of the grant of the patent: **30.03.2005**
(21) Application number: 99923484.2
(22) Date of filing: 28.04.1999
(51) Int. Cl.: C07D 231/38, A61K 31/415, C07D 401/10, C07D 403/12, C07D 401/12, A61K 31/44

(54) **PYRAZOLE DERIVATIVES AS P-38 MAP KINASE INHIBITORS**
PYRAZOLDERIVATIVE ALS P-38 MAP KINASE INHIBITOREN
DERIVES DE PYRAZOLE TENANT LIEU D'INHIBITEURS DE P.38 MAP KINASE

(30) Priority: 05.05.1998 US 84250 P; 02.03.1999 US 122410 P
(43) Date of publication of application: 14.02.2001
(73) Proprietor: F. Hoffmann-La Roche AG, 4070 Basel (CH)
(72) Inventor: LABADIE, Sharada, Shenvi, Sunnyvale, CA 94087 (US); ROTSTEIN, David, Mark, Sunnyvale, CA 94087 (US); SJOGREN, Eric, Brian, Mountain View, CA 94043 (US); TALAMAS, Francisco, Xavier, San Carlos, CA 94070 (US)
(74) Representative: Löschner, Thomas
(86) International application number: PCT/EP1999/002879
(87) International publication number: WO 1999/057101

(56) References cited:
- WO-A-94/13643
- TUPPER, DAVID E. ET AL: "Steric and electronic control in the addition of hydrazine and phenylhydrazine to.alpha.-[(dimethylamino)methylene]-.beta .- oxoarylpropanenitriles" SYNTHESIS (1997), (3), 337-341 , XP002115275
- NISHIWAKI, TAROZAEMON ET AL: "Heterocyclic chemistry. XIX. Synthesis of 4-aroyl-1-arylpyrazoles from.alpha.-aroyl-.beta.-anilinoacrylonitr iles and photochemistry of 4-carbonyl substituted pyrazoles" J. CHEM. SOC., PERKIN TRANS. 1 (1974), (15), 1871-5 , XP002115276

## Description

This invention relates to certain pyrazole derivatives that inhibit p38 MAP kinase, pharmaceutical compositions containing them, their use, intermediates and processes for preparing these compounds.

TNF and IL-1 have been shown to be central players in the pathological processes underlying many chronic inflammatory and autoimmune diseases. IL-1 is implicated in mediating or exacerbating diseases such as rheumatoid arthritis ((*see.,* Arend, W. P. *Arthritis & Rheumatism* **38**(2): 151-160, (1995)), osteoarthritis, bone resorption, toxic shock syndrome, tuberculosis, atherosclerosis, diabetes, Hodgkin's disease (*see.*, Benharroch, D.; et. al. *Euro. Cytokine Network* **7**(1): 51-57) and Alzheimer's disease. Excessive or unregulated TNF production has been implicated in mediating or exacerbating diseases such as rheumatoid arthritis ((*see.,* Maini, R. N.; et. al. *APMIS.* **105**(4): 257-263, (1997); Feldmann, M., *J. of the Royal College of Physicians of London* **30**(6): 560-570, (1996); Lorenz, H. M.; et. al. *J. of Immunology* **156**(4): 1646-1653, (1996)) osteoarthritis, spondylitis, sepsis, septic shock ((*see.,* Abraham, E.; et. al. JAMA. **277**(19):1531-1538, (1997), adult respiratory distress syndrome, asthma ((*see*., Shah, A.; et. al. *Clin. & Exp. Allergy* 1038-1044, (1995) and Lassalle, P., et. al. *Clin. & Exp. Immunol.* **94**(1): 105-110, (1993)), bone resorption diseases, fever ((see., Cooper, A. L., et. al. *Am*. *J. of Physiology* **267**(6 Pt. 2): 1431-1436)), encephalomyelitis, demyelination ((*see.,* Klindert, W. E.; et al. *J. of Neuroimmunol*. **72**(2): 163-168, (1997)) and periodontal diseases.

Clinical trials with IL-1 and TNF receptor antagonists have shown that blocking the ability of these cytokines to signal through their receptors leads to significant improvement, in humans, in inflammatory diseases. Therefore, modulation of these inflammatory cytokines is considered one of the most effective strategies to block chronic inflammation and have positive therapeutic outcomes. It has also been shown that p38 MAP kinase plays an important role in the translational control of TNF and IL-1 and is also involved in the biochemical signaling of these molecules ((see., Lee, J. C., et al. *Nature.* **372** (6508): 739-46, (1994)). Compounds that bind to p38 MAP are effective in inhibiting bone resorption, inflammation, and other immune and inflammation-based pathologies. The characterization of the p38 MAP kinase and its central role in the biosynthesis of TNF and IL-1 have made this kinase an attractive target for the treatment of diseases mediated by these cytokines.

It would therefore be desirable to provide p38 MAP kinase inhibitors and thereby provide a means of combating diseases mediated by pro-inflammatory cytokines such as TNF and IL-1. This invention fulfills this and related needs.

In a first aspect, this invention provides compounds selected from the group of compounds represented by Formula (I): 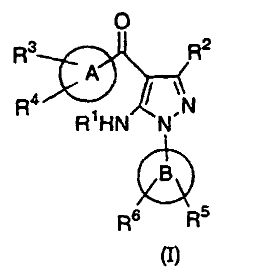 wherein:
R¹ is hydrogen, acyl or -P(O)(OH)₂;
R² is hydrogen;
A is an aryl, heteroaryl or a heterocyclyl ring optionally fused to a phenyl ring provided that the heterocyclyl ring is attached to the carbonyl group via a carbon ring atom;
B is an aryl or heteroaryl ring;
R³ is selected from the group consisting of:
   (a) amino, alkylamino or dialkylamino;
   (b) acylamino;
   (c) optionally substituted heterocyclyl;
   (d) optionally substituted aryl or heteroaryl;
   (e) heteroalkyl;
   (f) heteroalkenyl;
   (g) heteroalkynyl;
   (h) heteroalkoxy;
   (i) heteroalkylamino;
   (j) optionally substituted heterocyclylalkyl;
   (k) optionally substituted heterocyclylalkenyl;
   (l) optionally substituted heterocyclylalkynyl;
   (m) optionally substituted heterocyclylalkoxy or heterocyclyloxy;
   (n) optionally substituted heterocyclylalkylamino;
   (o) optionally substituted heterocyclylalkylcarbonyl;
   (p) heteroalkylcarbonyl;
   (q) -NHSO₂R⁶ where R⁶ is alkyl, heteroalkyl or optionally substituted heterocyclylalkyl;
   (r) -NHSO₂NR⁷R⁸ where R⁷ and R⁸ are, independently of each other, hydrogen, alkyl or heteroalkyl;
   (s) -Y-(alkylene)-R⁹ where:
      Y is a single bond, -O-, -NH- or -S(O)ₙ- (where n is an integer from 0 to 2); and
      R⁹ is cyano, optionally substituted heteroaryl, - COOH, -COR¹⁰, -COOR¹¹, -CONR¹²R¹³, -SO₂R¹⁴, -SO₂NR¹⁵R¹⁶, -NHSO₂R¹⁷ or -NHSO₂NR¹⁸R¹⁹, where R¹⁰ is alkyl or optionally substituted heterocycle, R¹¹ is alkyl, and R¹², R¹³, R¹⁴, R¹⁵, R¹⁶, R¹⁷, R¹⁸ and R¹⁹ are, independently of each other, hydrogen, alkyl or heteroalkyl;
   (t) -C(=NR²⁰)(NR²¹R²²) where R²⁰, R²¹ and R²² independently represent hydrogen, alkyl or hydroxy, or R²⁰ and R²¹ together are -(CH₂)ₙ- where n is 2 or 3 and R²² is hydrogen or alkyl;
   (u) -NHC(X)NR²³R²⁴ where X is -O- or -S-, and R²³ and R²⁴ are, independently of each other, hydrogen, alkyl or heteroalkyl;
   (v) -CONR²⁵R²⁶ where R²⁵ and R²⁶ independently represent hydrogen, alkyl, heteroalkyl or optionally substituted heterocyclylalkyl, or R²⁵ and R²⁶ together with the nitrogen to which they are attached form an optionally substituted heterocyclyl ring;
   (w) -S(O)ₙR²⁷ where n is an integer from 0 to 2, and R²⁷ is alkyl, heteroalkyl, optionally substituted heterocyclylalkyl or
      -NR²⁸R²⁹ where R²⁸ and R²⁹ are, independently of each other, hydrogen, alkyl or heteroalkyl;
   (x) cycloalkylalkyl, cycloalkylalkynyl and cycloalkylalkynyl, all optionally substituted with alkyl, halo, hydroxy or amino;
   (y) arylaminoalkylene or heteroarylaminoalkylene;
   (z) Z-alkylene-NR³⁰R³¹ or Z-alkylene-OR³² where Z is -NH-, -N(alkyl)- or -O-, and R³⁰, R³¹ and R³² are independently of each other, hydrogen, alkyl or heteroalkyl;
   (aa) -OC(O)-alkylene-CO₂H or -OC(O)-NR'R" (where R' and R" are independently hydrogen or alkyl); and
   (bb) heteroarylalkenylene or heteroarylalkynylene;
R⁴ is selected from the group consisting of:
   (a) hydrogen;
   (b) halo;
   (c) alkyl;
   (d) alkoxy; and
   (e) hydroxy;
R⁵ is selected from the group consisting of:
   (a) hydrogen;
   (b) halo;
   (c) alkyl;
   (d) haloalkyl;
   (e) thioalkyl;
   (f) hydroxy;
   (g) amino;
   (h) alkylamino;
   (i) dialkylamino;
   (j) heteroalkyl;
   (k) optionally substituted heterocycle;
   (l) optionally substituted heterocyclylalkyl;
   (m) optionally substituted heterocyclylalkoxy;
   (n) alkylsulfonyl;
   (o) aminosulfonyl, mono-alkylaminosulfonyl or di-alkylaminosulfonyl;
   (p) heteroalkoxy; and
   (q) carboxy;
R⁶ is selected from a group consisting of:
   (a) hydrogen;
   (b) halo;
   (c) alkyl; and
   (d) alkoxy;
individual isomers, mixtures of isomers and pharmaceutically acceptable salts thereof. In a second aspect, this invention provides processes for preparing compounds of Formula (I). In a third aspect this invention provides intermediates for preparing compounds of Formula (I).

In a fourth aspect, this invention provides medicaments or pharmaceutical compositions containing a therapeutically effective amount of a compound of Formula (I) or its pharmaceutically acceptable salt and a pharmaceutically acceptable excipient.

In a fifth aspect, this invention relates to the use of a compound of Formula (I).

Unless otherwise stated, the following terms used in the specification and claims have the meanings given below:
"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, pentyl, and the like.
"Alkylene" means a linear saturated divalent hydrocarbon radical of one to six carbon atoms or a branched saturated divalent hydrocarbon radical of three to six carbon atoms, e.g., methylene, ethylene, propylene, 2-methylpropylene, pentylene, and the like.
"Alkenyl" means a linear monovalent hydrocarbon radical of two to six carbon atoms or a branched monovalent hydrocarbon radical of three to six carbon atoms, containing at least one double bond, e.g., ethenyl, propenyl, and the like.
"Alkenylene" means a linear divalent hydrocarbon radical of two to six carbon atoms or a branched divalent hydrocarbon radical of three to six carbon atoms, containing at least one double bond, e.g., ethenylene, propenylene, and the like.
"Alkynyl" means a linear monovalent hydrocarbon radical of two to six carbon atoms or a branched divalent hydrocarbon radical of three to six carbon atoms, containing at least one triple bond, e.g., ethynyl, propynyl, and the like.
"Alkynylene" means a linear divalent hydrocarbon radical of two to six carbon atoms or a branched monovalent hydrocarbon radical of three to six carbon atoms, containing at least one triple bond, e.g., ethynylene, propynylene, and the like.
"Alkoxy" means a radical -OR where R is alkyl as defined above, e.g., methoxy, ethoxy, propoxy, 2-propoxy, the like.
"Acyl" means a radical -C(O)R where R is alkyl or haloalkyl e.g., acetyl, trifluoroacetyl, and the like.
"Acylamino" means a radical -NRC(O)R' where R is hydrogen or alkyl, and R' is alkyl, heteroalkyl or optionally substituted heterocyclylalkyl, e.g., acetylamino, 2-amino-2-methylpropionamide, and the like.
"Halo" means fluoro, chloro, bromo, or iodo, preferably fluoro and chloro.
"Haloalkyl" means alkyl substituted with one or more same or different halo atoms, e.g., -CH₂Cl, -CF₃, -CH₂CF₃, -CH₂CCl₃, and the like.
"Aryl" means a monovalent monocyclic or bicyclic aromatic hydrocarbon radical of 6 to 10 ring atoms e.g., phenyl, 1-naphthyl, 2-naphthyl, and the like. The aryl ring may optionally be fused to a 5-, 6- or 7- membered monocyclic saturated ring optionally containing 1 or 2 heteroatoms independently selected from oxygen, nitrogen or sulfur, the remaining ring atoms being C where one or two C atoms are optionally replaced by a carbonyl group. Representative aryl radicals with fused rings include, but are not limited to, 2,3-dihydrobenzo[1,4]dioxan, chroman, isochroman, 2,3-dihydrobenzofuran, 1,3-dihydroisobenzofuran, benzo[1,3]dioxole, 1,2,3,4-tetrahydroisoquinoline, 1,2,3,4-tetrahydroquinoline, 2,3-dihydro-1H-indole, 2,3-dihydro-1H-isoindole, benzimidazol-2-one, 3H-benzoxazol-2-one, and the like.
"Heteroaryl" means a monovalent monocyclic or bicyclic aromatic radical of 5 to 10 ring atoms containing one, two, or three ring heteroatoms selected from N, O, or S, the remaining ring atoms being C. The term also includes those radicals where a heteroatom within the ring has been oxidized or quaternized, such as, for example, to form an N-oxide or a quaternary salt. Representative examples include, but are not limited to, thienyl, benzothienyl, pyridyl, pyrazinyl, pyrimidinyl, pyridazinyl, quinolinyl, quinoxalinyl, imidazolyl, furanyl, benzofuranyl, thiazolyl, isoxazolyl, benzisoxazolyl, benzimidazolyl, triazolyl, pyrazolyl, pyrrolyl, indolyl, 2-pyridonyl, 4-pyridonyl, N-alkyl-2-pyridonyl, pyrazinonyl, pyridazinonyl, pyrimidinonyl, oxazolonyl, and their corresponding N-oxides, (e.g. pyridyl N-oxide, quinolinyl N-oxide), their quaternary salts and the like.
"Heterocycle" or "heterocyclyl" means a cyclic nonaromatic radical of 3 to 8 ring atoms in which one or two ring atoms are heteroatoms selected from N, O, or S(O)ₙ (where n is an integer from 0 to 2), the remaining ring atoms being C where one or two C atoms are optionally replaced by a carbonyl group. The term also includes those radicals where a ring nitrogen atom has been oxidized or quaternized, such as, for example, to form an N-oxide or a quaternary salt. Representative examples include, but are not limited to, tetrahydropyranyl, tetrahydrofuranyl, tetrahydrothiophenyl, piperidino, morpholino, piperazino, pyrrolidino, oxiranyl, dioxane, 1,3-dioxolanyl, 2,2-dimethyl-1,3-dioxalanyl, sulfolanyl, 2-oxazolidonyl, 2-imidazolidonyl, S,S-dioxo-thiomorpholino, and the like.
"Heterocycloamino" means a saturated monovalent cyclic group of 4 to 8 ring atoms, wherein at least one ring atom is N and optionally contains one additional ring atom selected from N or O, the remaining ring atoms being C. The term includes groups such as pyrrolidino, piperidino, morpholino, piperazino and the like.
"Optionally substituted aryl, heteroaryl or heterocyclyl" means an aryl, heteroaryl or heterocyclyl ring as defined above, which is optionally substituted independently with one or two substituents selected from alkyl, phenyl, benzyl, haloalkyl, heteroalkyl, halo, cyano, acyl, -OR (where R is hydrogen or alkyl), -NRR' (where R and R' are independently selected from hydrogen, alkyl or acyl), -NHCOR (where R is alkyl), -NRS(O)ₙR' (where R is hydrogen or alkyl, n is an integer from 0 to 2 and R' is hydrogen, alkyl or heteroalkyl), -NRS(O)ₙNR'R" (where R is hydrogen or alkyl, n is an integer from 0 to 2 and R' and R" are independently hydrogen, alkyl or heteroalkyl), -S(O)ₙR (where n is an integer from 0 to 2 and R is hydrogen, alkyl or heteroalkyl), -S(O)ₙNRR' (where n is an integer from 0 to 2 and R and R' are independently hydrogen, alkyl or heteroalkyl), -COOR, -(alkylene)COOR (where R is hydrogen or alkyl), -CONR'R" or -(alkylene)CONR'R" (where R' and R" are independently hydrogen or alkyl).
"Heteroalkyl" means an alkyl radical as defined above, carrying one, two or three substituents selected from -NR^{a}R^{b}, -OR^{c} wherein R^{a}, R^{b} and R^{c} are independently of each other hydrogen, alkyl or acyl, or R^{a} and R^{b} together form heterocycloamino group. Representative examples include, but are not limited to, hydroxymethyl, acetoxymethyl, 3-hydroxypropyl, 1,2-dihydroxyethyl, 2-methoxyethyl, 2-aminoethyl, 2-dimethylaminoethyl, 2-acetylaminoethyl, 3-[pyrrolidin-1-yl]ethyl and the like.
"Heteroalkenyl" means an alkenyl radical as defined above, carrying one or two substituents selected from -NR^{a}R^{b}, -OR^{c} or -S(O)ₙR^{d} wherein R^{a}, R^{b} and R^{c} are independently of each other hydrogen or alkyl, and R^{d} is alkyl or -NRR' (where R and R' are independently of each other hydrogen or alkyl. Representative examples include, but are not limited to, 3-hydroxy-1-propenyl, 3-aminoprop-1-enyl, 2-aminosulfonylethenyl, 2-methylsulfonylethenyl, and the like.
"Heteroalkynyl" means an alkynyl radical as defined above, carrying one or two substituents selected -NR^{a}R^{b}, -OR^{c}, -S(O)ₙR^{d} or -S(O)ₙNRR' (where R and R' are independently of each other hydrogen or alkyl) wherein R^{a}, R^{b} and R^{c} are independently of each other hydrogen or alkyl, and R^{d} is alkyl and n is an integer from zero to two. Representative examples include, but are not limited to, 3-hydroxy-1-propynyl, 3-dimethylaminoprop-1-ynyl and the like.
"Heteroalkoxy" means a radical -OR where R is heteroalkyl group as defined above, e.g., 2-hydroxyethoxy, 3-hydroxypropoxy, 2,3-dihydroxypropoxy, 2-aminoethoxy, and the like.
"Heteroalkylamino" means a radical -NR^{a}R^{b} where R^{a} is hydrogen or alkyl, and R^{b} is a heteroalkyl group as defined above, e.g., 2-hydroxyethylamino, 3-dimethylaminopropylamino, and the like.
" Optionally substituted heterocyclylalkyl" means a radical -R^{a}R^{b} where R^{a} is an alkylene group, and R^{b} is an optionally substituted heterocyclyl group as defined above e.g., 2-(morpholin-4-yl)ethyl, 3-(piperidin-1-yl)-2-methylpropyl, and the like.
" Optionally substituted heterocyclylalkenyl" means a radical -R^{a}R^{b} where R^{a} is an alkenylene group and R^{b} is an optionally substituted heterocyclyl group as defined above e.g., 3-(morpholin-4-yl)prop-1-enyll, 3-(piperidin-1-yl)prop-1-enyl, 3-(4-methylpiperazin-1-yl)prop-1-enyl, and the like.
" Optionally substituted heterocyclylalkynyl" means a radical -R^{a}R^{b} where R^{a} is an alkynyl group and R^{b} is an optionally substituted heterocyclyl group as defined above e.g., 3-(morpholin-4-yl)prop-1-ynyl, 3-(piperidin-1-yl)lprop-1-ynyl, and the like.
"Optionally substituted heterocyclylalkoxy" means a radical -OR where R is an optionally substituted heterocyclylalkyl group as defined above, e.g., 2-(morpholin-4-yl)-ethoxy, 3-(piperazin-1-yl)propoxy, 2-[2-oxopyrrolidin-1-yl]ethoxy, and the like.
"Optionally substituted heterocyclylalkylamino" means a radical -NR^{a}R^{b} where R^{a} is hydrogen or alkyl and R^{b} is an optionally substituted heterocyclylalkyl group as defined above, e.g., 2-(pyrrolidin-2-yl)ethylamino, 3-(piperidin-1-yl)propylamino, and the like.
"Optionally substituted heteroaralkyloxy means a radical -O-R^{a} where R^{a} is a heteroaralkyl radical e.g. 2-(pyridin-3-yl)ethoxy, 2-[3(2H)-pyridazon-1-yl]ethoxy and the like.
"Optional" or "optionally" means that the subsequently described event or circumstance may but need not occur, and that the description includes instances where the event or circumstance occurs and instances in which it does not. For example, "aryl group optionally mono- or disubstituted with an alkyl group" means that the alkyl may but need not be present, and the description includes situations where the aryl group is mono- or disubstituted with an alkyl group and situations where the heterocyclo group is not substituted with the alkyl group.
"Amino protecting group" refers to those organic groups intended to protect nitrogen atoms against undesirable reactions during synthetic procedures e.g., benzyl, benzyloxycarbonyl (CBZ), *tert*-butoxycarbonyl (Boc), trifluoroacetyl, and the like.

The compounds of this invention may possess one or more asymmetric centers; such compounds can therefore be produced as individual (*R*)- or (S)- stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 1992).

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. "A pharmaceutically acceptable excipient" as used in the specification and claims includes both one and more than one such excipient.

A "pharmaceutically acceptable salt" of a compound means a salt that is pharmaceutically acceptable and that possesses the desired pharmacological activity of the parent compound. Such salts include:
(1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-napthalenesulfonic acid, 4-toluenesulfonic acid, camphorsulfonic acid, 4-methylbicyclo[2.2.2]-oct-2-ene-1-carboxylic acid, glucoheptonic acid, 4,4'-methylenebis- (3-hydroxy-2-ene-1-carboxylic acid), 3-phenylpropionic acid, trimethylacetic acid, tertiary butylacetic acid, lauryl sulfuric acid, gluconic acid, glutamic acid, hydroxynapthoic acid, salicylic acid, stearic acid, muconic acid, and the like; or
(2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, *N*-methylglucamine, and the like.

"Pro-drugs" means any compound which releases an active parent drug according to Formula (I) *in vivo* when such prodrug is administered to a mammalian subject. Prodrugs of a compound of Formula (I) are prepared by modifying functional groups present in the compound of Formula (I) in such a way that the modifications may be cleaved *in vivo* to release the parent compound. Prodrugs include compounds of Formula (I) wherein a hydroxy, amino, or sulfhydryl group in compound (I) is bonded to any group that may be cleaved *in vivo* to regenerate the free hydroxyl, amino, or sulfhydryl group, respectively. Examples of prodrugs include, but are not limited to esters (e.g., acetate, formate, and benzoate derivatives), carbamates (e.g., N,N-dimethylaminocarbonyl) of hydroxy functional groups in compounds of Formula (I), and the like.

"Treating" or "treatment" of a disease includes:
(1) preventing the disease, i.e. causing the clinical symptoms of the disease not to develop in a mammal that may be exposed to or predisposed to the disease but does not yet experience or display symptoms of the disease,
(2) inhibiting the disease, i.e., arresting or reducing the development of the disease or its clinical symptoms, or
(3) relieving the disease, i.e., causing regression of the disease or its clinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

### NOMENCLATURE

The naming and numbering of the compounds of this invention is illustrated below.

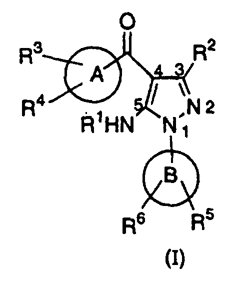

The nomenclature used in this application is generally based on the IUPAC recommendations, e.g., a compound of formula (I):
where R¹, R², R⁴, R⁶ are hydrogen, 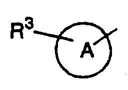 is 4-(3-hydroxypropyl)phenyl and 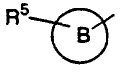 is 4-fluorophenyl is named 5-amino-1-(4-fluorophenyl)-4-[4-(3-hydroxypropyl)-benzoyl]pyrazole.
where R¹, R², R⁴, R⁶ are hydrogen, 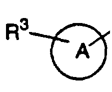 is 3-[3-(morpholin-4-yl)prop-1-ynyl]-phenyl and 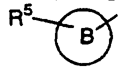 is 4-fluorophenyl is named 5-amino-1-(4-fluorophenyl)-4-[3-(3-morpholin-4-ylprop-1-ynyl)benzoyl]pyrazole.

### Representative compounds of this invention are as follows:

I. Compounds of Formula (I) where R¹, R² and R⁴ are hydrogen, B is phenyl and the other groups are as defined below are:
II.

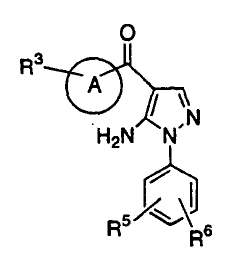

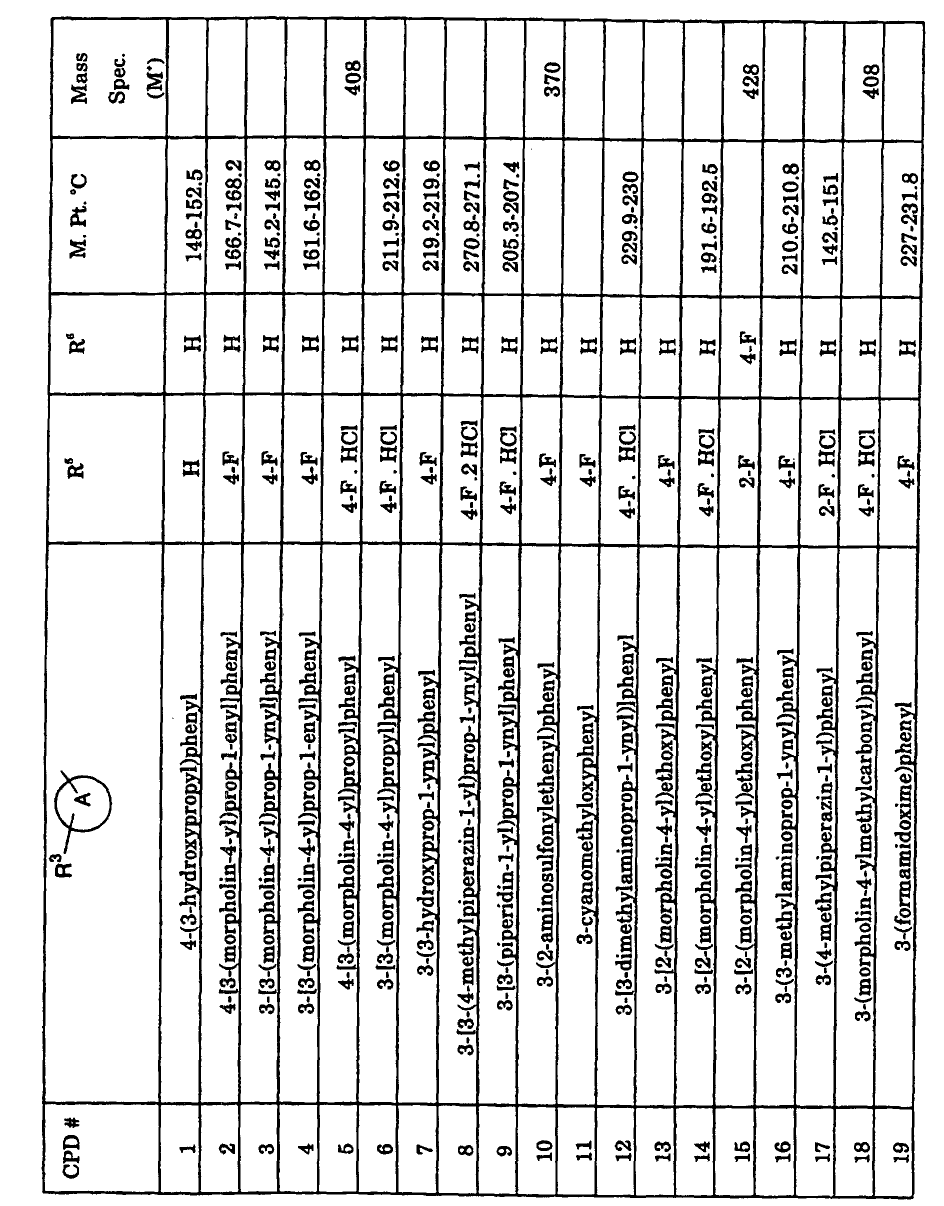

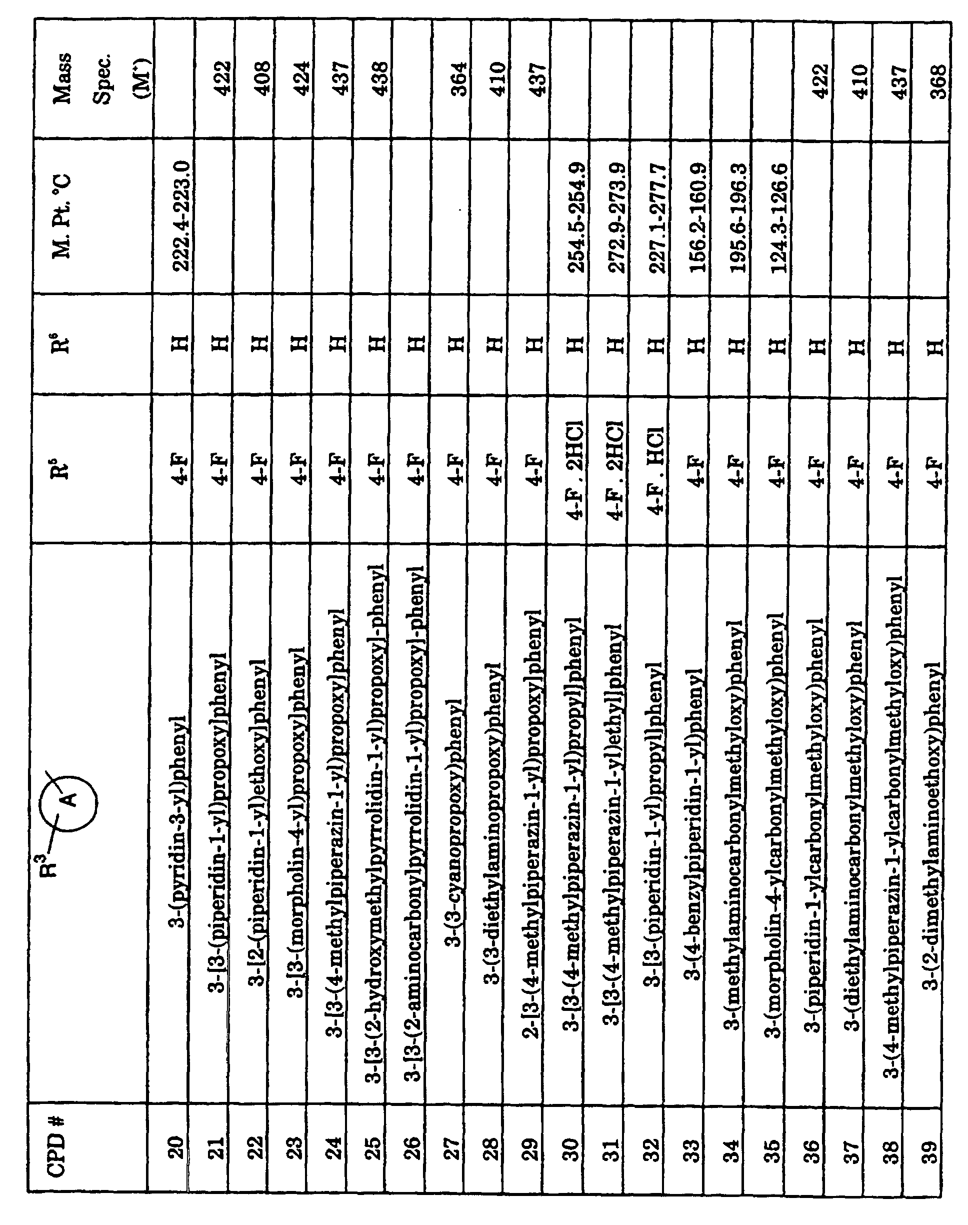

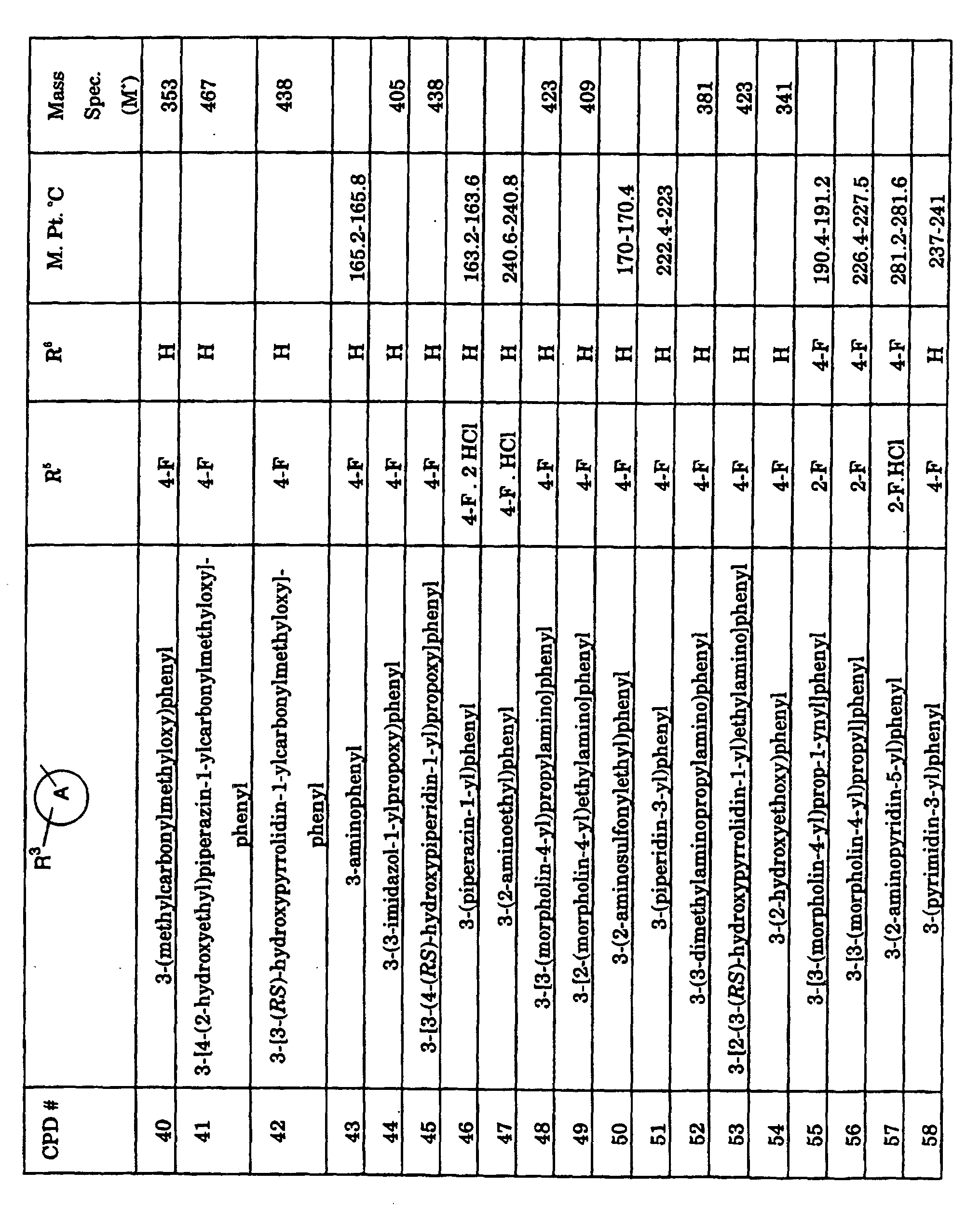

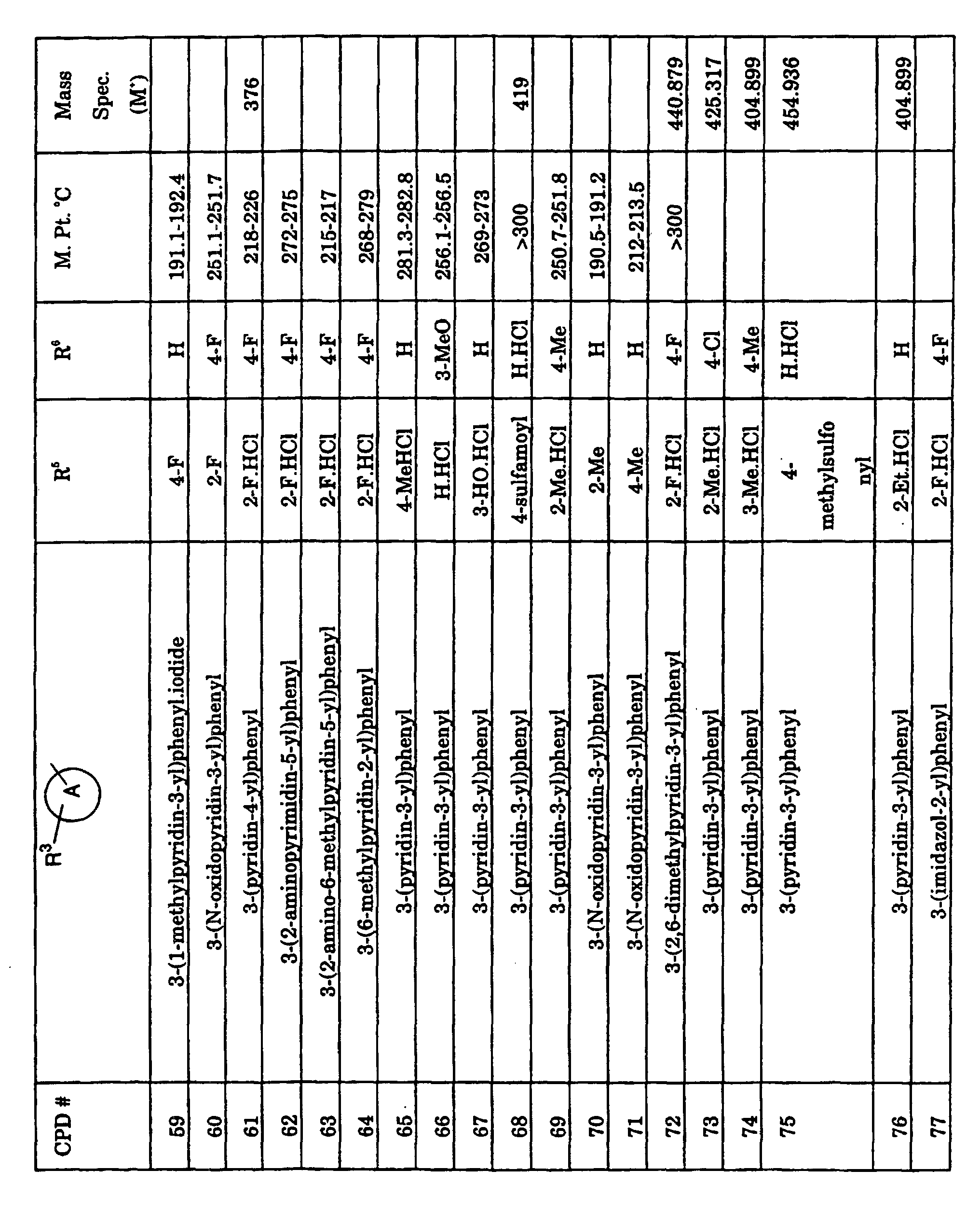

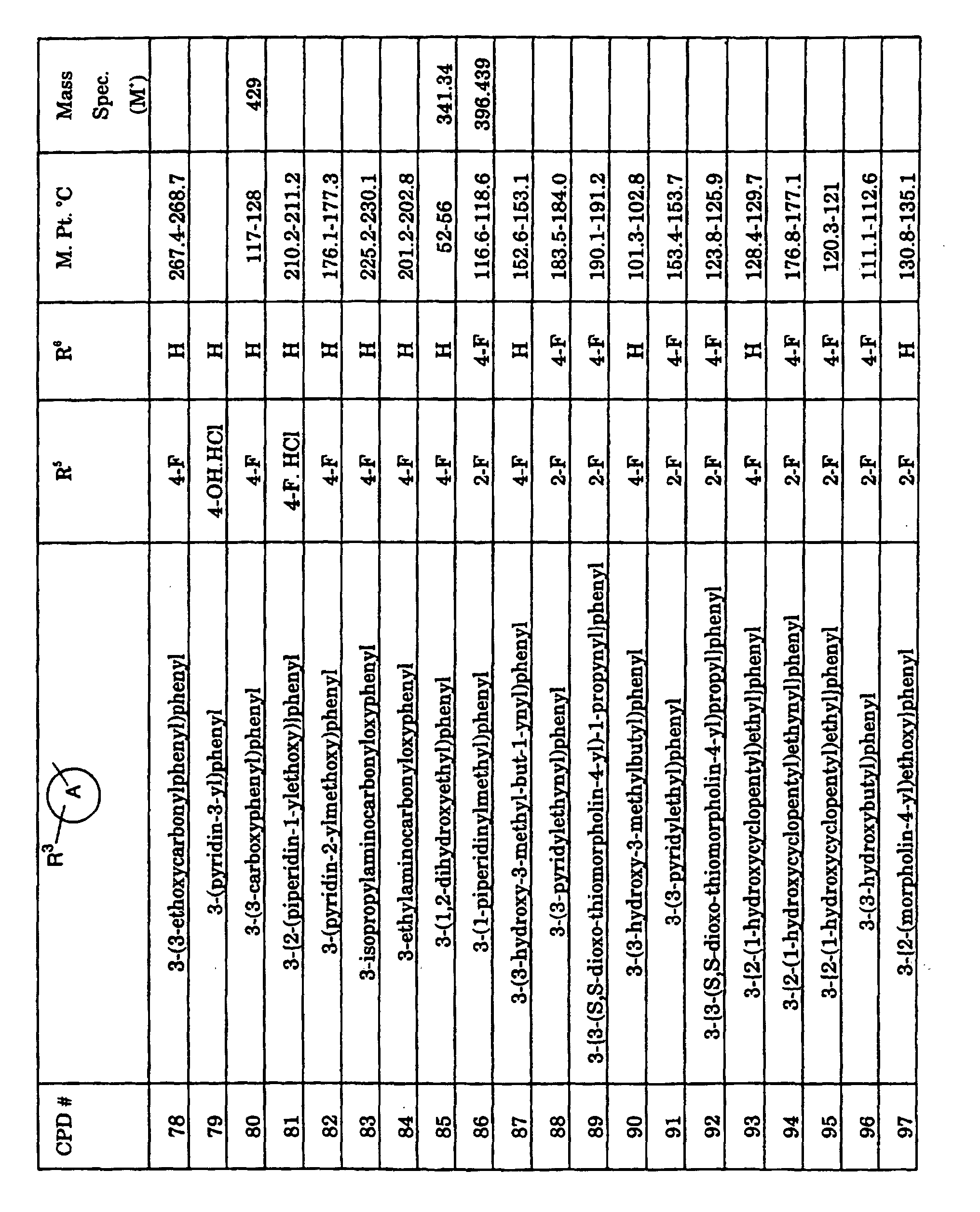

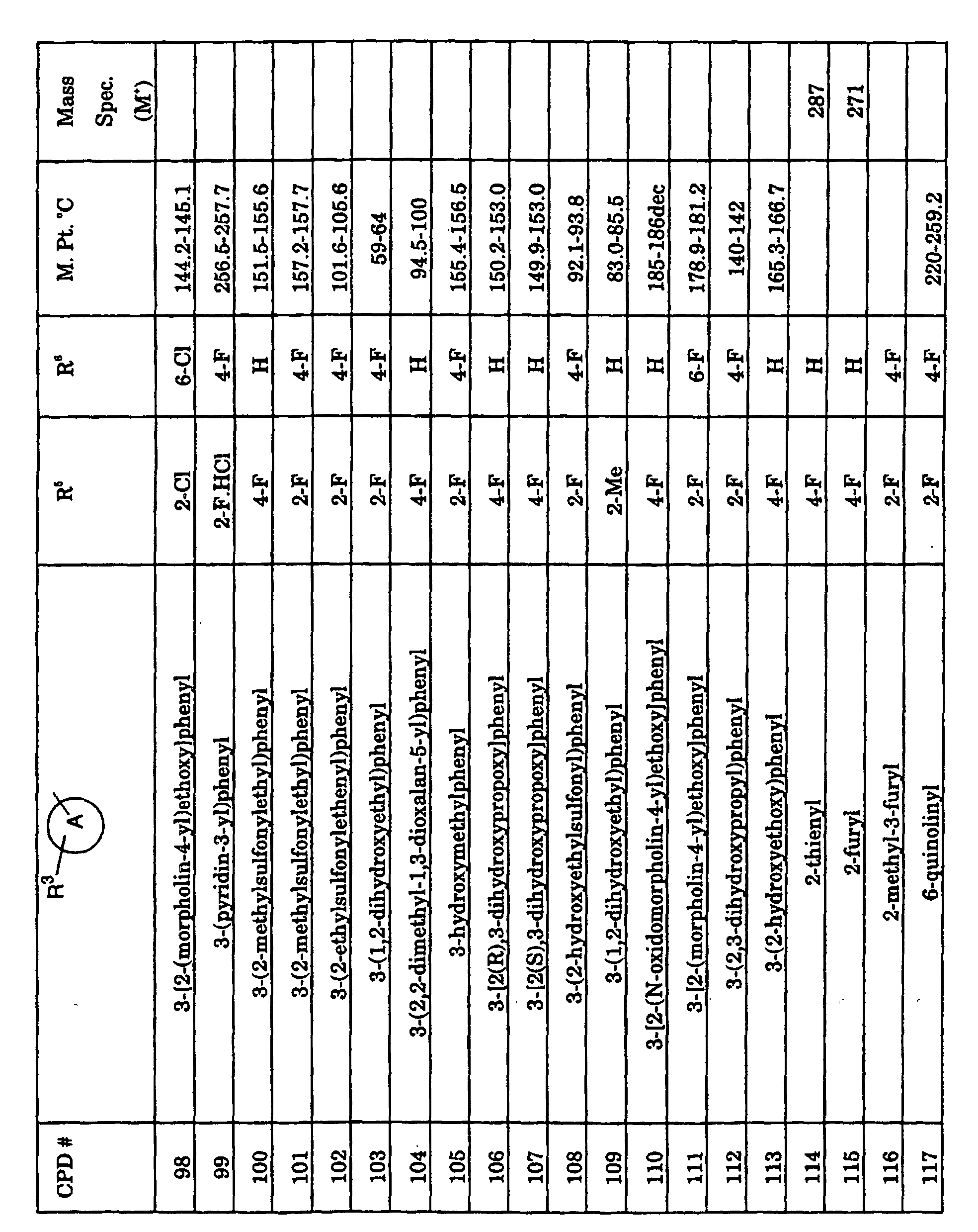

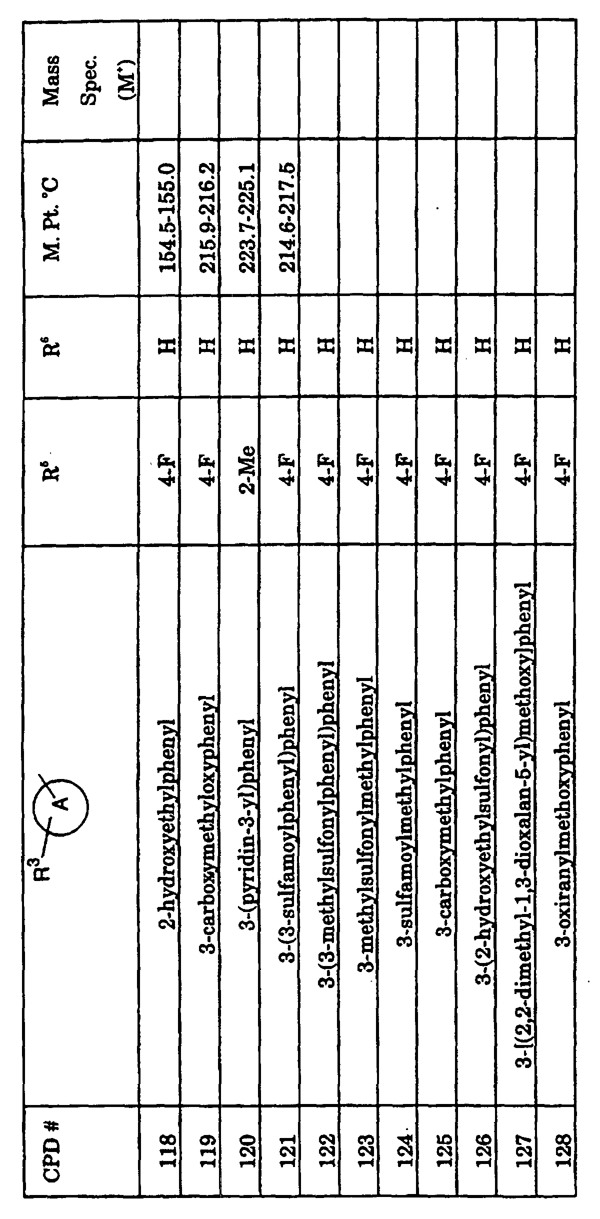

While the broadest definition of this invention is set forth above, certain compounds of Formula (I) are preferred.
I.1. A preferred group of compounds of formula (I) is wherein
   R¹ is hydrogen or acyl;
   A is an aryl or heteroaryl ring
   and the other residues are as defined above.
I.2. Another preferred group of compounds of Formula (I) is
   wherein
   R¹ is hydrogen, acyl or -P(O)(OH)₂;
   A is an aryl, heteroaryl or a heterocyclyl ring optionally fused to a phenyl ring provided that the heterocyclyl ring is attached to the carbonyl group via a carbon ring atom;
   B is an aryl or heteroaryl ring;
   R³ is selected from the group consisting of:
   (a) amino;
   (b) acylamino;
   (c) optionally substituted heterocycle;
   (d) heteroaryl optionally substituted with a substituent selected from halo, alkyl or alkoxy;
   (e) heteroalkyl;
   (f) heteroalkenyl;
   (g) heteroalkynyl;
   (h) heteroalkoxy;
   (i) heteroalkylamino;
   (j) optionally substituted heterocyclylalkyl;
   (k) optionally substituted heterocyclylalkenyl;
   (l) optionally substituted heterocyclylalkynyl;
   (m) optionally substituted heterocyclylalkoxy;
   (n) optionally substituted heterocyclylalkylamino;
   (o) optionally substituted heterocyclylalkylcarbonyl;
   (p) heteroalkylcarbonyl;
   (q) -NHSO₂R⁶ where R⁶ is alkyl, heteroalkyl or optionally substituted heterocyclylalkyl;
   (r) -NHSO₂NR⁷R⁸ where R⁷ and R⁸ are, independently of each other, hydrogen, alkyl or heteroalkyl;
   (s) -Y-(alkylene)-R⁹ where:
      Y is a single bond, -O-, -NH- or -S(O)ₙ- (where n is an integer from 0 to 2); and
      R⁹ is cyano, heteroaryl, -COOH, -COR¹⁰, -COOR¹¹ -CONR¹²R¹³, -SO₂R¹⁴, -SO₂NR¹⁵R¹⁶, -NHSO₂R¹⁷ or -NHSO₂NR¹⁸R¹⁹ where R¹⁰ is alkyl or optionally substituted heterocycle, R¹¹ is alkyl, and R¹², R¹³, R¹⁴, R¹⁵, R¹⁶, R¹⁷, R¹⁸ and R¹⁹ are, independently of each other, hydrogen, alkyl or heteroalkyl;
      (t) -C(=NR²⁰)(NR²¹R²²) where R²⁰, R²¹ and R²² independently represent hydrogen, alkyl or hydroxy, or R²⁰ and R²¹ together are -(CH₂)ₙ- where n is 2 or 3 and R²² is hydrogen or alkyl;
      (u) -NHC(X)NR²³R²⁴ where X is -O- or -S-, and R²³ and R²⁴ are, independently of each other, hydrogen, alkyl or heteroalkyl;
      (v) -CONR²⁵R²⁶ where R²⁵ and R²⁶ independently represent hydrogen, alkyl, heteroalkyl or optionally substituted heterocyclylalkyl, or R²⁵ and R²⁶ together with the nitrogen to which they are attached form an optionally substituted heterocyclyl ring;
      (w) -S(O)ₙR²⁷ where n is an integer from 0 to 2, and R²⁷ is alkyl, heteroalkyl, optionally substituted heterocyclylalkyl or
         -NR²⁸R²⁹ where R²⁸ and R²⁹ are, independently of each other, hydrogen, alkyl or heteroalkyl;
      R⁴ is selected from the group consisting of:
      (a) hydrogen;
      (b) halo;
      (c) alkyl; and
      (d) alkoxy;
      R⁵ is selected from the group consisting of :
      (a) hydrogen;
      (b) halo;
      (c) alkyl;
      (d) haloalkyl;
      (e) thioalkyl;
      (f) hydroxy;
      (g) amino;
      (h) alkylamino;
      (i) dialkylamino;
      (j) heteroalkyl;
      (k) optionally substituted heterocycle;
      (l) optionally substituted heterocyclylalkyl; and
      (m) optionally substituted heterocyclylalkoxy;
      R⁶ is selected from a group consisting of:
      (a) hydrogen;
      (b) halo;
      (c) alkyl; and
      (d) alkoxy
II.1. Within these groups and preferred groups a more preferred subgroup is wherein R³ is selected from:
   (a) optionally substituted heterocyclyl;
   (b) aryl or heteroaryl both optionally substituted with a substituent selected from halo, alkyl, amino, alkoxy, carboxy, lower alkoxy carbonyl, SO₂R' (where R' is alkyl) or SO₂NHR'R" (where R' and R" are independently hydrogen or alkyl);
   (c) heteroalkyl;
   (d) heteroalkenyl;
   (e) heteroalkylamino;
   (f) heteroalkoxy;
   (g) optionally substituted heterocyclylalkyl or heterocyclyloxy;
   (h) optionally substituted heterocyclylalkenyl;
   (i) optionally substituted heterocyclylalkynyl;
   (j) optionally substituted heterocyclylalkoxy;
   (k) optionally substituted heterocyclylalkylamino;
   (l) optionally substituted heterocyclylalkylcarbonyl;
   (s) -Y-(alkylene)-R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl;
   (x) cycloalkylalkyl, cycloalkylalkynyl and cycloalkylalkynyl, all optionally substituted with alkyl, halo, hydroxy or amino;
   (m) arylaminoalkylene or heteroarylaminoalkylene; or
   (n) Z-alkylene-NR³⁰R³¹ where Z is -NH-, -N(alkyl)- or -O-, and R³⁰ and R³¹ are independently of each other, hydrogen, alkyl or heteroalkyl.

   Within the above preferred group, more preferred groups of compounds are those wherein, A and B are aryl, preferably phenyl.
   Within the above preferred and more preferred groups, an even more preferred group of compounds is that wherein:
   R¹ is hydrogen;
   R⁴ is hydrogen, halo or alkyl, preferably hydrogen, chloro, fluoro or methyl, more preferably hydrogen.
II.1.1. Within the above preferred and more preferred groups, a particularly preferred group of compounds is that wherein R³ is at the 3-position and is optionally substituted heteroaryl, preferably pyridinyl, N-oxidopyridinyl or pyridonyl.
II.1.2. Another particularly preferred group of compounds is that wherein R³ is at the 3-position and is optionally substituted phenyl, preferably sulfamoylphenyl, alkylsulfamoylphenyl, carboxyphenyl, carboxamidophenyl, alkoxycarbonylphenyl, alkylaminocarbonylphenyl or dialkylaminocarbonylphenyl.
II.1.3. A third particularly preferred group of compounds is that wherein
   R³ is at the 3-position and is selected from:
   (a) heteroalkyl;
   (b) heteroalkoxy;
   (c) heteroalkylamino;
   (d) optionally substituted heterocyclylalkyl;
   (e) optionally substituted heterocyclylalkoxy;
   (f) optionally substituted heterocyclylalkylamino;
   (g) -Y-(alkylene)-R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl; or
   (h) Z-alkylene-NR³⁰R³¹ where Z is -NH-, -N(alkyl)- or -O-, and R³⁰ and R³¹ are independently of each other, hydrogen, alkyl or heteroalkyl.

   Preferred groups for R³ include amino, 3-dimethylaminopropoxy, 2-dimethylaminoethoxy, 2-dimethylaminoethyl, 3-dimethylaminopropyl, 4-dimethylaminobutyl, 2-dimethylaminoethylamino, 3-dimethylaminopropylamino, 3-dimethylaminoprop-1-enyl, 3-dimethylaminoprop-1-ynyl and 2-dimethylaminoethylcarbonyl, preferably amino.
   Another group of preferred groups for R³ is selected from 3-(morpholin-4-yl)propoxy, 2-(morpholin-4-yl)ethoxy, 3-(morpholin-4-yl)propyl, 2-(morpholin-4-yl)ethyl, 4-(morpholin-4-yl)butyl, 3-(morpholin-4-yl)propylamino, 2-(morpholin-4-yl)-ethylamino, 3-(morpholin-4-yl)-prop-1-enyl, 3-(morpholin-4-yl)prop-1-ynyl, 4-methylpiperazin-1-yl, piperazin-1-yl, pyridin-3-yl, morpholin-4-ylmethylcarbonyl, 3-dimethylaminoprop-1-enyl, 3-dimethylaminoprop-1-ynyl, 2-aminosulfonylethyl, 2-aminosulfonylethenyl, acetylamino and trifluoroacetylamino, preferably 2-(morpholin-4-yl)ethoxy and 3-(morpholin-4-yl)-propyl.
   A fourth group of particularly preferred compounds is that where R⁵ is halo or alkyl and R⁶ is hydrogen, halo or alkyl, preferably R⁵ is 4-F or 2-Me and R⁶ is hydrogen, or R⁵ is 2-F and R⁶ is 4-F.
III. Within the above preferred and more preferred I and II groups, an even more preferred group of compounds is that wherein R¹ is hydrogen and B is phenyl; particularly wherein A is phenyl; more particularly wherein R⁴ is hydrogen; and R⁵ is halo or alkyl, particularly wherein R⁵ is chloro, fluoro or methyl and R⁶ is hydrogen, chloro, fluoro, methyl or methoxy.
IV.1. Within the above preferred and more preferred R³ groups in I, II and III, a particularly preferred R³ group is that wherein R³ is optionally substituted heteroaryl, particularly wherein R³ is pyridin-2-yl, pyridin-3-yl, pyridin-4-yl, N-oxidopyridin-2-yl, N-oxidopyridin-3-yl, N-oxidopyridin-4-yl or pyridon-2-yl, all optionally substituted, especially wherein R³ is at the 3-position, particularly together with R⁵ and R⁶ wherein R⁵ is 4-F or 2-Me and R⁶ is hydrogen;
IV.2. Within the above preferred and even more preferred R³ groups I, II and III, another particular preferred R³ group is that wherein R³ is optionally substituted phenyl, particularly wherein R³ is 3-sulfamoylphenyl, 3-methylsulfonylphenyl, 3-carboxyphenyl or 3-ethoxycarbonylphenyl, particularly wherein R³ is at the 3-position, especially in combination wherein R⁵ is 4-F or 2-Me and R⁶ is hydrogen.
IV.3. Within the above preferred and even more preferred R³ groups in I, II and III, another particular preferred group of R³ groups are those wherein R³ is:
   (a) heteroalkyl;
   (b) heteroalkoxy;
   (c) heteroalkylamino;
   (d) optionally substituted heterocyclylalkyl;
   (e) optionally substituted heterocyclylalkoxy;
   (f) optionally substituted heterocyclylalkylamino;
   (g) -Y-(alkylene)-R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl; or
   (h) Z-alkylene-NR³⁰R³¹ where Z is -NH-, -N(alkyl)- or -O-, and R³⁰ and R³¹ are independently of each other, hydrogen, alkyl or heteroalkyl.
      a) A more preferred subgroup within these particular preferred R³ groups is that wherein R³ is heteroalkyl, especially wherein R³ is at the 3-position and is selected from the group consisting of 2-dimethylaminoethyl, 3-dimethylaminopropyl, 4-dimethylaminobutyl, 2-dimethylaminoethylamino, 3-dimethylaminopropylamino, hydroxymethyl, 1,2-dihydroxyethyl, 3-hydroxy-3-methyl-1-butyl or 3-hydroxybutyl, particularly in combination with R⁵ and R⁶, wherein R⁵ is 2-F and R⁶ is 4-F, or wherein R⁵ is 4-F and R⁶ is hydrogen or wherein R⁵ is 2-Me and R⁶ is hydrogen.
      b) Another more preferred subgroup within these particular preferred R³ groups is that wherein R³ is heteroalkoxy or heteroalkylamino, particularly wherein R³ is at the 3-position and is selected from the group consisting of 3-dimethylaminopropoxy, 2-dimethylaminoethoxy, 2-hydroxyethoxy, 2,3-dihydroxypropoxy, 2-dimethylaminoethylamino and 3-dimethylaminopropylamino, especially in combination with R⁵ and R⁶ wherein R⁵ is 4-F or 2-Me and R⁶ is hydrogen.
      c) Another preferred R³ subgroup within these particular preferred groups is that wherein R³ is optionally substituted heterocyclylalkyl, optionally substituted heterocyclylalkoxy or optionally substituted heterocyclylalkylamino, particularly wherein R³ is at the 3-position and is selected from the group consisting of 3-(morpholin-4-yl)propoxy, 2-(morpholin-4-yl)ethoxy, 2-(2-oxo-pyrrolidin-1-yl)ethoxy, 3-(morpholin-4-yl)propyl, 2-(morpholin-4-yl)ethyl, 4-(morpholin-4-yl)butyl, 3-(morpholin-4-yl)propylamino, 2-(morpholin-4-yl)ethylamino, 4-hydroxypiperidinylmethyl, 2-(S,S-dioxo-thiamorpholin-4-yl)ethyl, 3-(S,S-dioxo-thiamorpholin-4-yl)propyl and N-methylpiperazinylmethyl, especially in combination with R⁵ and R⁶ wherein R⁵ is 4-F or 2-Me and R⁶ is hydrogen.
      d) Another preferred R³ subgroup is that wherein R³ is -Y-(alkylene)- R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl, particularly wherein Y is a single bond and R⁹ is SO₂R¹⁴ or -SO₂NR¹⁵R¹⁶. Preferably R³ is methylsulfonylethyl or sulfamoylethyl and R³ is preferably combined with R⁵ and R⁶ wherein R⁵ is 4-F or 2-Me and R⁶ is hydrogen.
V. It has to be noted that in the embodiment I.2. of compounds of formula (I) wherein
   R¹ is hydrogen, acyl or -P(O)(OH)₂;
   A is an aryl, heteroaryl or a heterocyclyl ring optionally fused to a phenyl ring provided that the heterocyclyl ring is attached to the carbonyl group via a carbon ring atom;
   B is an aryl or heteroaryl ring;
   R³ is selected from the group consisting of:
   (a) amino;
   (b) acylamino;
   (c) optionally substituted heterocycle;
   (d) heteroaryl optionally substituted with a substituent selected from halo, alkyl or alkoxy;
   (e) heteroalkyl;
   (f) heteroalkenyl;
   (g) heteroalkynyl;
   (h) heteroalkoxy;
   (i) heteroalkylamino;
   (j) optionally substituted heterocyclylalkyl;
   (k) optionally substituted heterocyclylalkenyl;
   (l) optionally substituted heterocyclylalkynyl;
   (m) optionally substituted heterocyclylalkoxy;
   (n) optionally substituted heterocyclylalkylamino;
   (o) optionally substituted heterocyclylalkylcarbonyl;
   (p) heteroalkylcarbonyl; (q) -NHSO₂R⁶ where R⁶ is alkyl, heteroalkyl or optionally substituted heterocyclylalkyl;
   (r) -NHSO₂NR⁷R⁸ where R⁷ and R⁸ are, independently of each other, hydrogen, alkyl or heteroalkyl;
   (s) -Y-(alkylene)-R⁹ where:
      Y is a single bond, -O-, -NH- or -S(O)ₙ- (where n is an integer from 0 to 2); and
      R⁹ is cyano, heteroaryl, -COOH, -COR¹⁰, -COOR¹¹ -CONR¹²R¹³, -SO₂R¹⁴, -SO₂NR¹⁵R¹⁶, -NHSO₂R¹⁷ or -NHSO₂NR¹⁸R¹⁹ where R¹⁰ is alkyl or optionally substituted heterocycle, R¹¹ is alkyl, and R¹², R¹³, R¹⁴, R¹⁵, R¹⁶, R¹⁷, R¹⁸ and R¹⁹ are, independently of each other, hydrogen, alkyl or heteroalkyl;
   (t) -C(=NR²⁰)(NR²¹R²²) where R²⁰, R²¹ and R²² independently represent hydrogen, alkyl or hydroxy, or R²⁰ and R²¹ together are -(CH₂)ₙ- where n is 2 or 3 and R²² is hydrogen or alkyl;
   (u) -NHC(X)NR²³R²⁴ where X is -O- or -S-, and R²³ and R²⁴ are, independently of each other, hydrogen, alkyl or heteroalkyl;
   (v) -CONR²⁵R²⁶ where R²⁵ and R²⁶ independently represent hydrogen, alkyl, heteroalkyl or optionally substituted heterocyclylalkyl, or R²⁵ and R²⁶ together with the nitrogen to which they are attached form an optionally substituted heterocyclyl ring;
   (w) -S(O)ₙR²⁷ where n is an integer from 0 to 2, and R²⁷ is alkyl, heteroalkyl, optionally substituted heterocyclylalkyl or
      -NR²⁸R²⁹ where R²⁸ and R²⁹ are, independently of each other, hydrogen, alkyl or heteroalkyl;
   R⁴ is selected from the group consisting of:
   (a) hydrogen;
   (b) halo;
   (c) alkyl; and
   (d) alkoxy;
   R⁵ is selected from the group consisting of :
   (a) hydrogen;
   (b) halo;
   (c) alkyl;
   (d) haloalkyl;
   (e) thioalkyl;
   (f) hydroxy;
   (g) amino;
   (h) alkylamino;
   (i) dialkylamino;
   (j) heteroalkyl;
   (k) optionally substituted heterocycle;
   (l) optionally substituted heterocyclylalkyl; and
   (m) optionally substituted heterocyclylalkoxy; and
   R⁶ is selected from a group consisting of:
   (a) hydrogen;
   (b) halo;
   (c) alkyl; and
   (d) alkoxy;
   further preferred embodiments exist.
V.1. In one preferred embodiment a preferred subgroup is that wherein R³ is selected from:
   (a) optionally substituted heterocycle;
   (b) heteroaryl optionally substituted with a substituent selected from halo, alkyl or alkoxy;
   (c) optionally substituted heterocyclylalkyl;
   (d) optionally substituted heterocyclylalkenyl;
   (e) optionally substituted heterocyclylalkynyl;
   (f) optionally substituted heterocyclylalkoxy;
   (g) optionally substituted heterocyclylalkylamino;
   (h) optionally substituted heterocyclylalkylcarbonyl;
   (i) heteroalkenyl;
   (j) heteroalkynyl;
   (k) -Y-(alkylene)-R⁹ where Y is a single bond, -O- or -NH- and R⁹ is -CONR¹²R¹³ or -SO₂NR¹⁵R¹⁶ where R¹², R¹³, R¹⁵ and R¹⁶ are independently of each other hydrogen, alkyl or heteroalkyl; and
   (l) acylamino,
   particularly wherein B is aryl; particularly wherein R⁴ is hydrogen, halo or alkyl, R⁵ is hydrogen, halo, alkyl, hydroxy or heteroalkyl and R⁶ is hydrogen, halo or alkyl; particularly wherein A is aryl; B is phenyl; particularly wherein A is phenyl; particularly wherein R¹ is hydrogen, R⁴ is hydrogen, chloro, fluoro or methyl, R⁵ is hydrogen, fluoro, methyl, hydroxy, amino, hydroxymethyl or aminomethyl and R⁶ is hydrogen or fluoro;
   particularly wherein R³ is at the 3-position and is selected from 3-(morpholin-4-yl)propoxy, 2-(morpholin-4-yl)ethoxy, 3-(morpholin-4-yl)propyl, 2-(morpholin-4-yl)ethyl, 4-(morpholin-4-yl)butyl, 3-(morpholin-4-yl)propylamino, 2-(morpholin-4-yl)ethylamino, 3-(morpholin-4-yl)prop-1-enyl, 3-(morpholin-4-yl)prop-1-ynyl, 4-methylpiperazin-1-yl, piperazin-1-yl, pyridin-3-yl, morpholin-4-ylmethyl-carbonyl, 3-dimethylaminoprop-1-enyl, 3-dimethylaminoprop-1-ynyl, 2-amino-sulfonylethyl, 2-aminosulfonylethenyl, acetylamino and trifluoroacetylamino.
V.2. Another preferred subgroup of R³ is that wherein R³ is selected from:
   (a) amino;
   (b) heteroalkyl;
   (c) heteroalkoxy;
   (d) heteroalkylamino; and
   (e) heteroalkylcarbonyl;
   particularly wherein B is aryl; particularly wherein R⁴ is hydrogen, halo or alkyl, R⁵ is hydrogen, halo, alkyl, hydroxy or heteroalkyl and R⁶ is hydrogen, halo or alkyl; particularly wherein A is aryl and B is phenyl; particularly wherein A is phenyl; particularly wherein R¹ is hydrogen, R⁴ is hydrogen, chloro, fluoro or methyl, R⁵ is hydrogen, fluoro, methyl, hydroxy, amino, hydroxymethyl or aminomethyl and R⁶ is hydrogen or fluoro;
   particularly wherein R³ is at the 3-position and is selected from amino, 3-dimethylaminopropoxy, 2-dimethylaminoethoxy, 2-dimethylaminoethyl, 3-dimethylaminopropyl, 4-dimethylaminobutyl, 2-dimethylaminoethylamino, 3-dimethylaminopropylamino and 2-dimethylaminoethylcarbonyl.
V.3. In another preferred group of compounds of formula (I) is that
   wherein A is a phenyl ring fused to a 5 or 6 membered monocyclic saturated ring optionally containing 1 or 2 heteroatoms independently selected from nitrogen or oxygen the remaining ring atoms being carbon, where one or two carbon atoms are optionally replaced by a carbonyl group; particularly wherein B is aryl; particularly wherein R⁴ is hydrogen, halo or alkyl, R⁵ is hydrogen, halo, alkyl, hydroxy or heteroalkyl and R⁶ is hydrogen, halo or alkyl; particularly wherein R² is hydrogen and B is phenyl;
   particularly wherein R³ is selected from:
   (a) optionally substituted heterocycle;
   (b) heteroaryl optionally substituted with a substituent selected from halo, alkyl or alkoxy;
   (c) optionally substituted heterocyclylalkyl;
   (d) optionally substituted heterocyclylalkenyl;
   (e) optionally substituted heterocyclylalkynyl.
   (f) optionally substituted heterocyclylalkoxy;
   (g) optionally substituted heterocyclylalkylamino;
   (h) optionally substituted heterocyclylalkylcarbonyl;
   (i) heteroalkenyl;
   (j) heteroalkynyl;
   (k) -Y-(alkylene)-R⁹ where Y is a single bond, -O- or - NH- and R⁹ is -CONR¹²R¹³ or -SO₂NR¹⁵R¹⁶ where R¹², R¹³, R¹⁵ and R¹⁶ are independently of each other hydrogen, alkyl or heteroalkyl; and
   (l) acylamino or
   wherein R³ is selected from:
   (a) amino;
   (b heteroalkyl;
   (c) heteroalkoxy;
   (d) heteroalkylamino; and
   (f) heteroalkylcarbonyl;
   more particularly wherein A is 2,3-dihydrobenzo[1,4]dioxane, chroman, isochroman, 2,3-dihydrobenzofuran, 1,3-dihydroisobenzofuran, benzo[1,3]dioxole, 1,2,3,4-tetrahydroisoquinoline, 1,2,3,4-tetrahydroquinoline, 2,3-dihydro-1H-indole, 2,3-dihydro-1H-isoindole, benzimidazol-2-one or 3H-benzoxazolin-2-one.
V.4. Within the embodiment of the compounds of formula (I) wherein R³ is selected from:
   (a) optionally substituted heterocycle;
   (b) heteroaryl optionally substituted with a substituent selected from halo, alkyl or alkoxy;
   (c) optionally substituted heterocyclylalkyl;
   (d) optionally substituted heterocyclylalkenyl;
   (e) optionally substituted heterocyclylalkynyl;
   (f) optionally substituted heterocyclylalkoxy;
   (g) optionally substituted heterocyclylalkylamino;
   (h) optionally substituted heterocyclylalkylcarbonyl;
   (i) heteroalkenyl;
   (j) heteroalkynyl;
   (k) -Y-(alkylene)-R⁹ where Y is a single bond, -O- or -NH- and R⁹ is -CONR¹²R¹³ or -SO₂NR¹⁵R¹⁶ where R¹², R¹³, R¹⁵ and R¹⁶ are independently of each other hydrogen, alkyl or heteroalkyl; and
   (l) acylamino;
   and wherein B is aryl, and
   wherein R⁴ is hydrogen, halo or alkyl, R⁵ is hydrogen, halo, alkyl, hydroxy or heteroalkyl and R⁶ is hydrogen, halo or alkyl, another preferred group is that
   wherein A is heteroaryl and B is phenyl; particularly
   wherein A is furanyl, thienyl or pyridyl; particularly
   wherein R¹ is hydrogen, R⁴ is hydrogen, chloro, fluoro or methyl, R⁵ is hydrogen, fluoro, methyl, hydroxy, amino, hydroxymethyl or aminomethyl and R⁶ is hydrogen or fluoro; particularly
   wherein R³ is selected from 3-(morpholin-4-yl)propoxy, 2-(morpholin-4-yl)ethoxy, 3-(morpholin-4-yl)propyl, 2-(morpholin-4-yl)ethyl, 4-(morpholin-4-yl)butyl, 3-(morpholin-4-yl)propylamino, 2-(morpholin-4-yl)ethylamino, 3-(morpholin-4-yl)prop-1-enyl, 3-(morpholin-4-yl)prop-1-ynyl, 4-methylpiperazin-1-yl, piperazin-1-yl, pyridin-3-yl, morpholin-4-ylmethylcarbonyl, 3-dimethylaminoprop-1-enyl, 3-dimethylaminoprop-1-ynyl, 2-aminosulfonylethyl, 2-aminosulfonylethenyl, acetylamino and trifluoroacetylamino.
V.5. Another group of compounds of formula (I) wherein R³ is selected from:
   (a) amino;
   (b) heteroalkyl;
   (c) heteroalkoxy;
   (d) heteroalkylamino; and
   (e) heteroalkylcarbonyl, and
   wherein B is aryl, and wherein R⁴ is hydrogen, halo or alkyl, R⁵ is hydrogen, halo, alkyl, hydroxy or heteroalkyl and R⁶ is hydrogen, halo or alkyl, also such are preferred wherein A is heteroaryl and B is phenyl; particularly wherein A is furanyl, thienyl or pyridyl; particularly
   wherein R¹ is hydrogen, R⁴ is hydrogen, chloro, fluoro or methyl, R⁵ is hydrogen, fluoro, methyl, hydroxy, amino, hydroxymethyl or aminomethyl and R⁶ is hydrogen or fluoro; particularly wherein R³ is selected from amino, 3-dimethylamino-propoxy, 2-dimethylaminoethoxy, 2-dimethylaminoethyl, 3-dimethylaminopropyl, 4-dimethylaminobutyl, 2-dimethylaminoethylamino, 3-dimethylaminopropylamino and 2-dimethylaminoethylcarbonyl.
V.6. Another group of compounds of formula (I), wherein R³ is selected from:
   (a) optionally substituted heterocycle;
   (b) heteroaryl optionally substituted with a substituent selected from halo, alkyl or alkoxy;
   (c) optionally substituted heterocyclylalkyl;
   (d) optionally substituted heterocyclylalkenyl;
   (e) optionally substituted heterocyclylalkynyl;
   (f) optionally substituted heterocyclylalkoxy;
   (g) optionally substituted heterocyclylalkylamino;
   (h) optionally substituted heterocyclylalkylcarbonyl;
   (i) heteroalkenyl;
   (j) heteroalkynyl;
   (k) -Y-(alkylene)-R⁹ where Y is a single bond, -O- or -NH- and R⁹ is -CONR¹²R¹³ or -SO₂NR¹⁵R¹⁶ where R¹², R¹³, R¹⁵ and R¹⁶ are independently of each other hydrogen, alkyl or heteroalkyl; and
   (l) acylamino; and
   B is aryl and R⁴ is hydrogen, halo or alkyl, R⁵ is hydrogen, halo, alkyl, hydroxy or heteroalkyl and R⁶ is hydrogen, halo or alkyl; is that,
   wherein A is heterocyclyl ring and B is phenyl; particularly
   wherein A is tetrahydrofuran, tetrahydropyran, dioxane or dioxolane, particularly, wherein R¹ is hydrogen, R⁴ is hydrogen, chloro, fluoro or methyl, R⁵ is hydrogen, fluoro, methyl, hydroxy, amino, hydroxymethyl or aminomethyl and R⁶ is hydrogen or fluoro; particularly, wherein R³ is selected from amino, 3-dimethylaminopropoxy, 2-dimethylaminoethoxy, 2-dimethylaminoethyl, 3-dimethylamino-propyl, 4-dimethylaminobutyl, 2-dimethylaminoethylamino, 3-dimethylaminopropyl-amino and 2-dimethylaminoethylcarbonyl.

Preferred compounds of the invention are selected from the group consisting of:
5-amino-1-(4-fluorophenyl)-4-[3-(2-morpholin-4-ylethoxy)benzoyl]pyrazole,
5-amino-1-(2,4-difluorophenyl)-4-[3-(3-morpholin-4-ylpropyl)benzoyl]-pyrazole,
5-amino-4-(3-aminobenzoyl)-1-(4-fluorophenyl)pyrazole,
5-amino-1-(4-fluorophenyl)-4-[3-(3-morpholin-4-ylpropyl)benzoyl]pyrazole,
5-amino-4-[3-(2-aminosulfonylethenyl)benzoyl]-1-(4-fluorophenyl)pyrazole,
5-amino-4-(3-acetylaminobenzoyl)-1-phenylpyrazole,
5-amino-4-[3-(2-aminoethyl)benzoyl]-1-(4-fluorophenyl)pyrazole,
5-amino-1-(4-fluorophenyl)-4-[3-(3-morpholin-4-ylpropylamino)benzoyl]pyrazole,
5-amino-4-[3-(2-aminosulfonylethyl)benzoyl]-1-(4-fluorophenyl)pyrazole,
5-amino-1-(4-fluorophenyl)-4-(3-pyridin-3-ylbenzoyl)pyrazole, and

Other preferred compounds of the invention are selected from the group consisting of:
5-amino-1-(2-methylphenyl)-4-[3-pyridin-3-yl)benzoyl]pyrazole,
5-amino-1-(2-methylphenyl)-4-[3-(N-oxidopyridin-3-yl)benzoyl]pyrazole,
5-amino-4-[3-(2,3-dihydroxypropoxy)benzoyl]-1-(4-fluorophenyl)pyrazole,
5-amino-4-[3-(1,2-dihydroxyethyl)benzoyl]-1-(4-fluorophenyl)pyrazole,
5-amino-1-(4-fluorophenyl)-4-[3-(sulfamoylbenzoyl]pyrazole,
5-amino-1-(4-fluorophenyl)-4-[3-(3-hydroxy-3-methylbutyl)benzoyl]pyrazole,
5-amino-1-(4-fluorophenyl)-4-[3-(2-(1-hydroxycyclopentyl)ethyl)benzoyl]pyrazole,
5-amino-4-[3-(2-methylsulfonylethyl)benzoyl]-1-(4-fluorophenyl)pyrazole, and
5-amino-1-(2,4-difluorophenyl)-4-[3-(2-hydroxyethylsulfonyl)benzoyl]pyrazole.

In a second aspect the present invention relates to processes for preparing compounds of formula (I). In one embodiment the compounds of formula (I) can be prepared by a process comprising
(i) reacting a 2-keto-3-phenylaminoacrylonitrile of Formula 1: 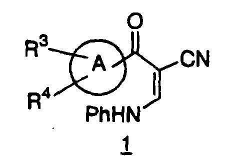 with a hydrazine of Formula 2: 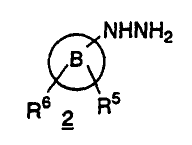 where R³, R⁴ R⁵ and R⁶ are as defined above to provide a compound of Formula (I) where R¹ is hydrogen; or
(ii) reacting a 2-keto-3-phenylaminoacrylonitrile of formula 3: 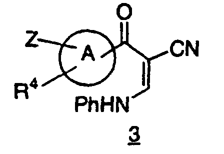 where Z is either hydroxy, nitro or halo group and R⁴ is as defined above with a hydrazine of formula 2 to provide a compound of formula 4: 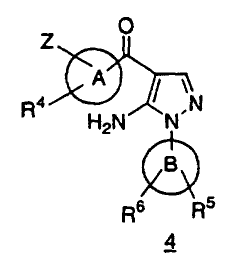 followed by conversion of the Z group to the desired R³ group to provide a compound of Formula (I) where R¹ is hydrogen;
(iii) optionally modifying any of the R¹, R³, R⁴, R⁵ or R⁶ groups;
(iv) optionally converting the compound of Formula (I) prepared in Steps (i), (ii) or (iii) above, to the corresponding acid addition salt by treatment with an acid;
(v) optionally converting the compound of Formula (I) prepared in Steps (i), (ii) or (iii) above, to the corresponding free base by treatment with a base; and
(vi) optionally separating a mixture of stereoisomers of a compound of Formula (I) prepared in Steps (i) - (v) above, to give a single stereoisomer.

In another embodiment the compounds can be prepared by a process comprising reacting a compound of Formula 5: 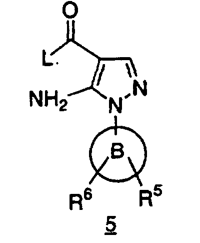 where R⁵ and R⁶ are as defined above and L is a leaving group under organometallic displacement reaction conditions
with an organometallic reagent of formula 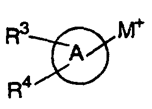 where R³ and R⁴ are as defined above and M is a metallic moiety to provide a compound of Formula (I) where R¹ is hydrogen;
(ii) optionally modifying any of the R¹, R³, R⁴, R⁵ or R⁶ groups;
(iii) optionally converting the compound of Formula (I) prepared in Steps (i) or (ii) above, to the corresponding acid addition salt by treatment with an acid;
(iv) optionally converting the compound of Formula (I) prepared in Steps (i) or (ii) above, to the corresponding free base by treatment with a base; and
(v) optionally separating a mixture of stereoisomers of a compound of Formula (I) prepared in Steps (i) or (iv) above, to give a single stereoisomer.

More specifically, the compounds of this invention can be made by the methods depicted in the reaction schemes shown below.

The starting materials and reagents used in preparing these compounds are either available from commercial suppliers such as Aldrich Chemical Co., (Milwaukee, Wisconsin, USA), Bachem (Torrance, California, USA), Emka-Chemie, or Sigma (St. Louis, Missouri, USA) or are prepared by methods known to those skilled in the art following procedures set forth in references such as *Fieser and Fieser's Reagents for Organic Synthesis*, Volumes 1-17 (John Wiley and Sons, 1991); *Rodd's Chemistry of Carbon Compounds,* Volumes 1-5 and Supplementals (Elsevier Science Publishers, 1989), *Organic Reactions*, Volumes 1-40 (John Wiley and Sons, 1991), *March's* Advanced Organic Chemistry, (John Wiley and Sons, 4th Edition), and *Larock's* Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure.

The starting materials and the intermediates of the reaction may be isolated and purified if desired using conventional techniques, including but not limited to filtration, distillation, crystallization, chromatography, and the like. Such materials may be characterized using conventional means, including physical constants and spectral data.

### Preparation of compounds of Formula (I)

Schemes A, B and C describe methods to generate the compounds of Formula (I).

### Scheme A

Compounds of Formula (I) where groups are as defined in the Summary of the Invention are prepared as described below.

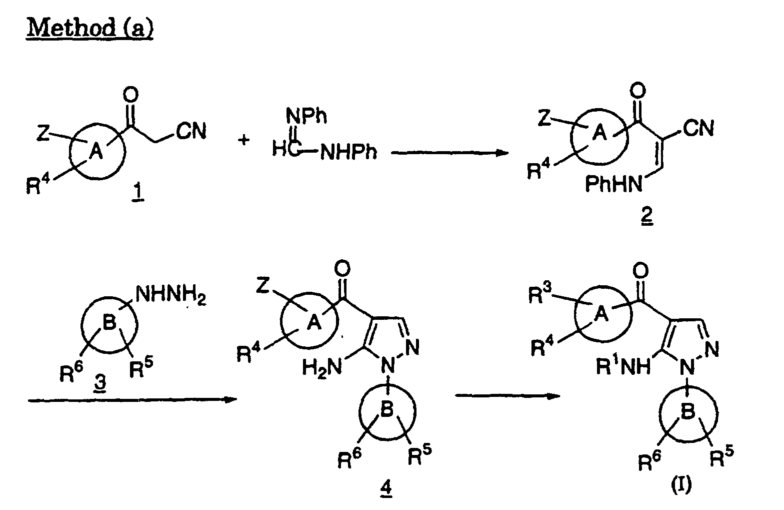

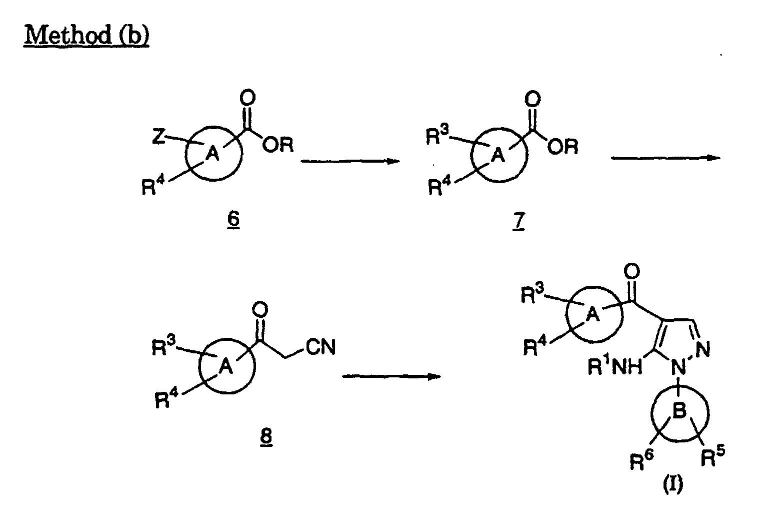

In general, compounds of Formula (I) can be prepared by following either method (a) or (b) as described below.

### Method (a)

Reaction of a 2-ketoacetonitrile of formula 1 [where Z is halo (e.g., bromo or iodo), alkoxy, nitro or acetylamino] with N,N-diphenylformamidine gives a 2-keto-3-phenylamino-acrylonitrile of formula 2. The reaction occurs upon heating in a high boiling aromatic hydrocarbon such as toluene, xylene, and the like.

In general, compounds of formula 1 are either commercially available or they can be prepared by methods well known in the art. For example, 2-aroylacetonitriles of formula 1 such as 4-methoxybenzoylacetonitrile, 3-nitrobenzoylacetonitrile are commercially available. Others can be prepared by treating acetonitrile with a base such as *n*-butyllithium followed by reaction of the formed acetonitrile anion with an aroyl/heteroaroyl halide or an aryl/heteroaryl ester as described in Sjogren, E. B., et al., *J. Med. Chem*, 34, 3295, (1991).

Reaction of the 2-keto-3-phenylaminoacrylonitrile of formula 2 with a hydrazine of formula 3 provides a 5-amino-4-ketopyrazole of formula 4. This reaction is generally carried out in a polar solvent such as ethanol, isopropanol, and the like. Aryl/heteroaryl hydrazines of formula 2 such as 2-or 3-chlorophenylhydrazine, 2-,3-, or 4-fluorophenylhydrazine, phenylhydrazine, 2-hydrazinopyridine, 2-hydrazinobenzothiazole, 2-hydrazinoquinoline etc., are commercially available.

Compound 4 is then converted to a compound of Formula (I) where R¹ is hydrogen and R³ is as defined in the Summary of the Invention by methods well known in the art. Some such procedures are described below.
(i) A compound of Formula (I) where R³ is heterocyclylalkyloxy can be prepared from a compound of formula 4 where Z is alkoxy as shown below: 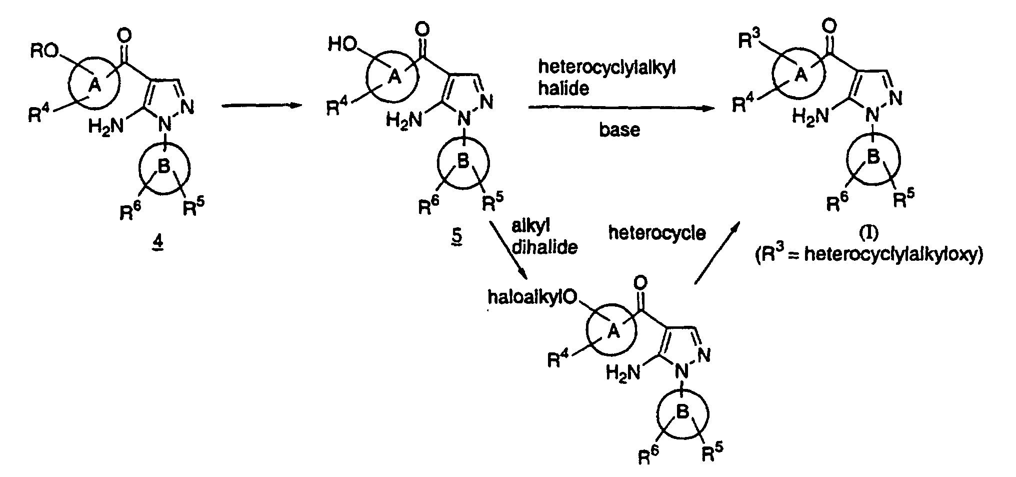
   A compound of Formula (I) where R³ is heterocyclylalkyloxy can be prepared from a compound of formula 4 where Z is alkoxy by first dealkylating the alkoxy group to give the corresponding compound of formula 5 where Z is hydroxy followed by reaction with a heterocyclylalkyl halide [e.g., 4-(2-chloroethyl)morpholine, 1-(2-chloroethyl)pyrrolidine, and the like]. The de-alkylation reaction is carried out either with boron tribromide in a halogenated organic solvent such as dichloromethane or by heating 4 in neat pyridinium hydrochloride. The alkylation is carried out in the presence of a base (such as potassium carbonate, cesium carbonate, and the like) in a polar organic solvent such as acetonitrile, dimethylformamide, acetone, and the like.
   Alternatively, a heterocyclylalkyl group can be attached by reacting 5 with an alkyl dihalide followed by the reaction of the resulting haloalkyloxy intermediate with a heterocyclyl group (e.g., piperazine, morpholine, pyrrolidine, and the like) under the reaction conditions described above. Alkyl dihalides such as 1-bromo-2-chloroethane, 1-chloro-3-iodopropane, and the like, are commercially available.
(ii) A compound of Formula (I) where R³ is -O-(alkylene)-R⁹ (where R⁹ is -COOH, -COR¹⁰, -COOR¹¹ or -CONR¹²R¹³) can be prepared from a compound of formula 5 as shown below: 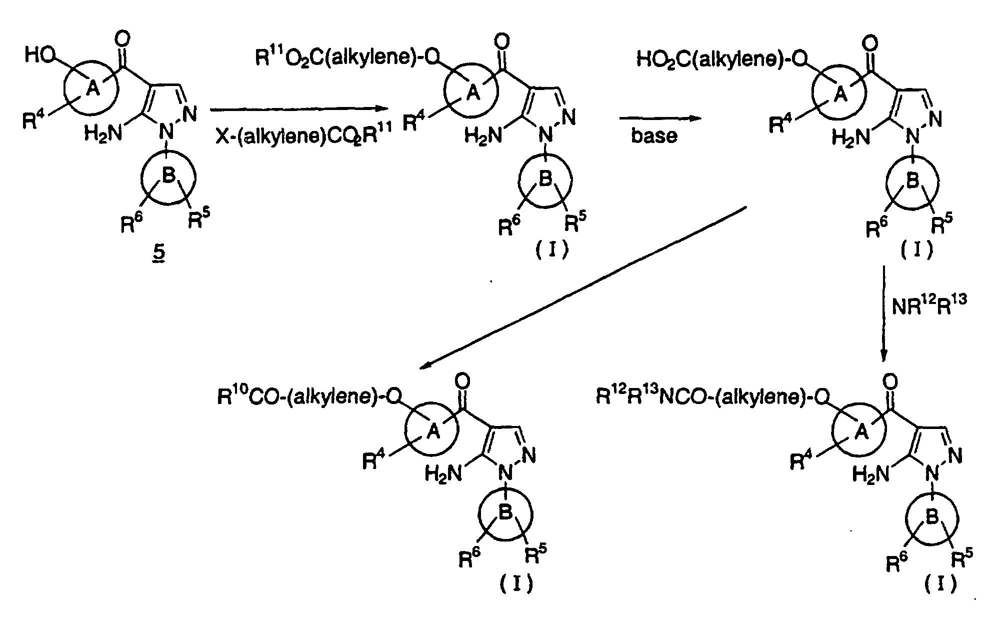
   A compound of Formula (I) where R³ is -O-(alkylene)-COOR¹¹ is prepared by reacting a compound of formula 5 with an alkylating agent of formula X-(alkylene)-CO₂R¹¹ where X is a halo group. Hydrolysis of the ester group provides the free acid (R⁹ is -COOH) which can be converted to a compound of Formula (I) where R⁹ = -CONR¹²R¹³, if desired, by treating the acid with an amine of formula NR¹²R¹³ (where R¹² and R¹³ are as defined in the Summary of the Invention) in the presence of a suitable coupling agent (e.g., carbonyl diimidazole, N,N-dicyclohexylcarbodiimide and the like).
   A compound of Formula (I) where R⁹ is -COR¹⁰ can be prepared from a compound of Formula (I) where R⁹ is -COOH by first converting the acid to a Weinreb amide followed by treatment with either a Grignard reagent or organolithium reagent of formula R¹⁰MgBr or R¹⁰Li, respectively.
(iii) A compound of Formula (I) where R³ is -NH-(alkylene)-R⁹ where R⁹ is -COOH, -COR¹⁰, -COOR¹¹, -CONR¹²R¹³ or heterocyclylalkylamino can be prepared from a compound of formula 4 where Z is a nitro group by reducing the nitro group to the amino group and then following the procedures described above.
(iv) A compound of Formula (I) where R³ is heteroalkenyl, heteroalkynyl, heterocyclyl-alkenyl, heterocyclylalkynyl, heteroalkyl or heterocyclylalkyl can be prepared as shown below. 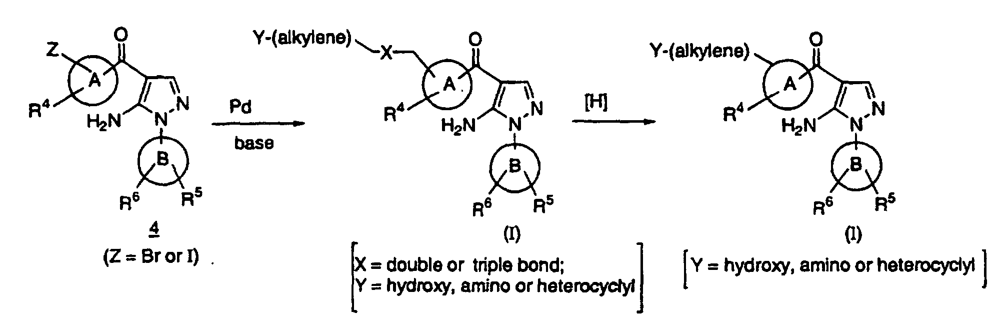
   A compound of Formula (I) where R³ is heteroalkenyl, heteroalkynyl, heterocyclylalkenyl or heterocyclylalkynyl can be prepared by reacting a compound of formula 4 where Z is halo with a heteroalkene, heteroalkyne, heterocyclylalkene or heterocyclylalkyne respectively in the presence of a palladium (II) catalyst such as dichlorobis(triphenylphosphine)-palladium (II) in an organic base such as diisopropylamine, and the like.
   Heteroalkenes, heteroalkynes such as allyl alcohol, propargyl alcohol, 3-butyn-1-ol, propargylamine are commercially available. Heterocyclylalkyne can be prepared by reacting an alkynyl halide with a heterocycle. For example, 2-morpholin-1-ylprop-1-yne can be prepared by reacting propargyl bromide with morpholine. Reduction of the double or triple bond under catalytic hydrogenation reaction conditions provides the corresponding compound of Formula (I) where R³ is a heterocyclylalkyl or heteroalkyl group.
(v) A compound of Formula (I) where R³ is -NHSO₂R⁶, -NHSO₂NR⁷R⁸ or NHC(X)R²³R²⁴ (where X is -O- or -S-) can be prepared from a compound of Formula (I) where R³ is amino by following the synthetic procedures described in PCT Application No. WO 97/46524.

A compound of Formula (I) where R¹ is an acyl group can be prepared by reacting the the corresponding compound of Formula (I) where R¹ is hydrogen with an acylating reagent of formula R¹COL where L is a leaving group under acylating reaction conditions such as halo. The reaction is carried out in the presence of a base such as sodium hydroxide, cesium carbonate, and the like.

### Method (b)

Alternatively, a compound of Formula (I) can be prepared from an ester of formula 6 where Z is as defined above, by first converting the Z group in compound 6 to the desired R³ group utilizing the reaction conditions described in method (a)(i-v) above. Condensation of 7 with acetonitrile anion gives a 2-ketoacetonitrile of formula 8 which is then converted to a compound of Formula (I) utilizing the reaction conditions described in method (a) above.

Compounds of Formula (I) where R² is thioalkyl or alkyl can be prepared by following the procedures described in U. S. Pat. No. 5,712,303.

### Scheme B

An alternate synthesis of compounds of Formula (I) where groups are as defined in the Summary of the Invention is described below.

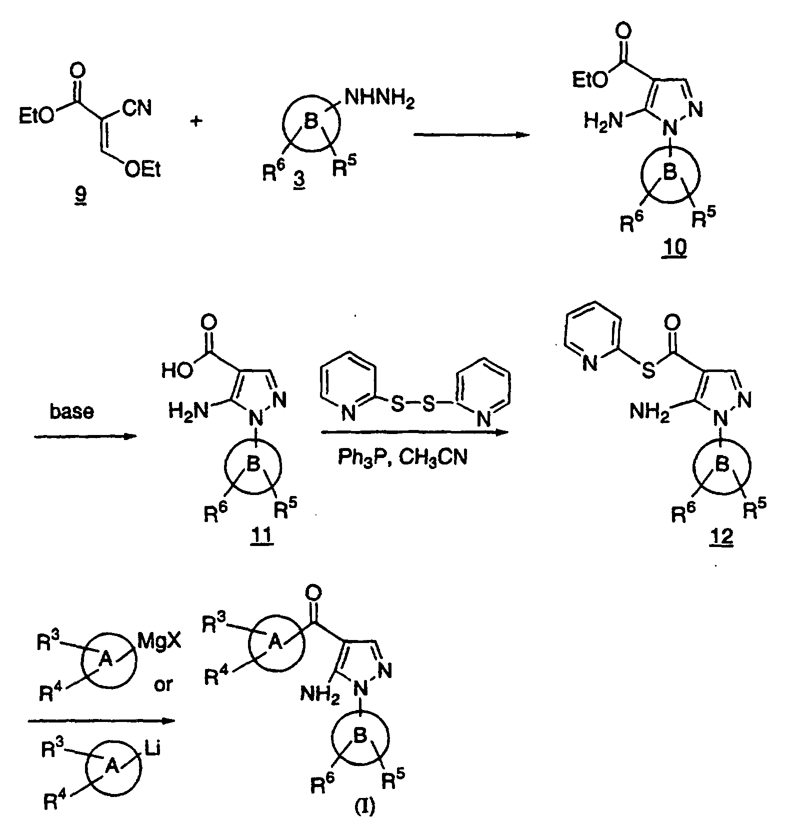

Condensation of 2-cyano-3-ethoxyacrylate of formula 9 with a hydrazine of formula 3 provides a 5-amino-4-ethoxycarbonyl pyrazole of formula 10. The condensation reaction is carried out in a suitable polar organic solvent such as ethanol, isopropanol, and the like. Hydrolysis of 10 with an aqueous base (e.g., sodium hydroxide, lithium hydroxide, and the like) in an alcoholic organic solvent (e.g., methanol, ethanol, and the like) provides the corresponding 5-amino-4-carboxypyrazole of formula 11. Treatment of 11 with dipyridyldisulfide followed by reaction of the resulting thiopyridyl ester derivative 12 with an organometallic reagent such as a Grignard reagent or an organolithium reagent shown above provides a compound of Formula (I).

### Scheme C

Another alternate synthesis of compounds of Formula (I) where groups are as defined above is described below.

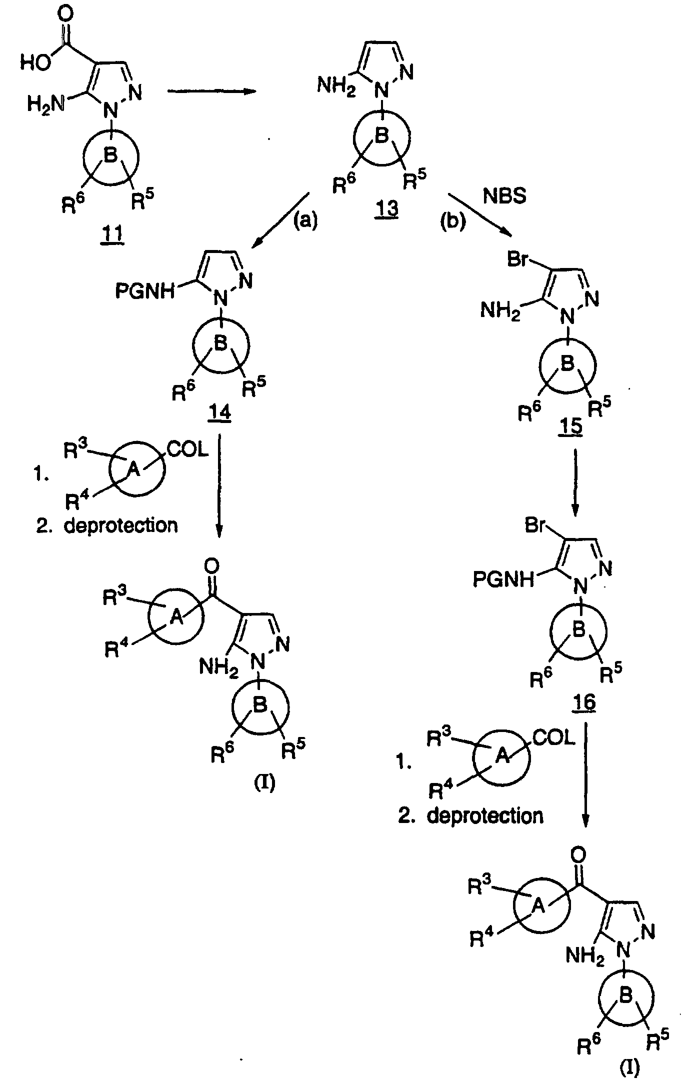

Thermal decarboxylation of a 5-amino-4-carboxypyrazole of formula 11 gives the corresponding 5-aminopyrazole of formula 13. Compound 13 is then converted to a compound of Formula (I) as shown in method (a) or (b) above.

In method (a), a compound of formula 13 is converted to a compound of Formula (I) by first protecting the amino group in compound 13 with a suitable amino protecting group (e.g., *tert*-butoxycarbonyl, and the like) to give the corresponding amino-protected compound of formula 14. Treatment of 14 with an acid derivative of formula R³COL where L is a leaving group under organometallic displacement reaction conditions [e.g., alkoxy (preferably methoxy or ethoxy), dialkylamino, or preferably N,O-dimethylhydroxylamino] followed by the removal of the amino protecting group then provides a compound of Formula (I). The nucleophilic substitution is carried out in the presence of 2 equivalents of an alkyllithium (e.g., *tert*-butyllithium, and the like) and in an aprotic organic solvent such as tetrahydrofuran. The reaction conditions employed for the removal of the amino protecting group depends on the nature of the protecting group. For example, if *tert*-butoxycarbonyl is the protecting group, it is removed by treatment with an acid such as trifluroacetic acid, hydrochloric acid, and the like.

Acid derivatives of formula R³COL can be prepared by methods well known in the field of organic chemistry. For example, an acid derivative where L is a N,O-dimethylhydroxylamino group can be prepared from its corresponding acid by first converting the acid to the acid chloride with a suitable chlorinating agent such as oxalyl chloride, followed by treatment with N,O-dimethylhydroxylamine hydrochloride in the presence of an organic base such as triethylamine.

In method (b), a compound of formula 10 is brominated to give the 5-amino-4-bromo-pyrazole of formula 15. The bromination reaction is carried out with a suitable brominating agent such as N-bromosuccinimide in a suitable polar organic solvent such as dimethylformamide. Compound 15 is then converted to a compound of Formula (I) utilizing the reaction conditions described in Scheme C, method (a) above.

In a third aspect the present invention provides intermediates of formula 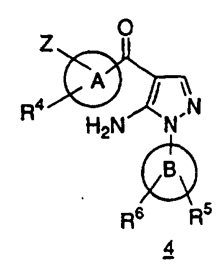 wherein Z is either hydroxy, nitro or halo group and A, B, R⁴, R⁵ and R⁶ are as defined above.

In a fourth embodiment, the present invention provides medicaments or pharmaceutical compositions containing a compound of formula (I) and a pharmaceutically acceptable excipient.

### Utility

The compounds of Formula (I) are p38 MAP kinase inhibitors and therefore compounds of Formula (I) and compositions containing them are useful in the treatment of diseases such as rheumatoid arthritis, osteoarthritis, spondylitis, bone resorption diseases, sepsis, septic shock, toxic shock syndrome, endotoxic shock, tuberculosis, atherosclerosis, diabetes, adult respiratory distress syndrome, chronic pulmonary inflammatory disease, fever, periodontal diseases, ulcerative colitis, pyresis, Alzheimer's and Parkinson's diseases. Preferably, the compounds and medicaments may be used in the treatment of inflammatory diseases, preferably arthritis.

### Testing

The ability of the compounds of Formula (I) to inhibit p38 MAP kinase was demonstrated by the *in vitro* assay described in Example 15. The ability of the compounds of Formula (I) to inhibit the release of TNF-α was demonstrated by the *in vitro* and the *in vivo* assays described in detail in Examples 16 and 17, respectively. The anti-inflammatory activity of the compounds of this invention was determined utilizing adjuvant induced arthritis in rats assay described in Example 18.

### Administration and Pharmaceutical Compositions

In general, the compounds of this invention will be administered in a therapeutically effective amount by any of the accepted modes of administration for agents that serve similar utilities. The actual amount of the compound of this invention, i.e., the active ingredient, will depend upon numerous factors such as the severity of the disease to be treated, the age and relative health of the subject, the potency of the compound used, the route and form of administration, and other factors.

Therapeutically effective amounts of compounds of Formula (I) may range from approximately 0.1-50 mg per kilogram body weight of the recipient per day; preferably about 1-30 mg/kg/day. Thus, for administration to a 70 kg person, the dosage range would most preferably be about 70 mg to 2.1g per day.

In general, compounds of this invention will be administered as pharmaceutical compositions by any one of the following routes: oral, systemic (e.g., transdermal, intranasal or by suppository), or parenteral (e.g., intramuscular, intravenous or subcutaneous) administration. The preferred manner of administration is oral using a convenient daily dosage regimen which can be adjusted according to the degree of affliction. Compositions can take the form of tablets, pills, capsules, semisolids, powders, sustained release formulations, solutions, suspensions, elixirs, aerosols, or any other appropriate compositions.

The choice of formulation depends on various factors such as the mode of drug administration (e.g., for oral administration, formulations in the form of tablets, pills or capsules are preferred) and the bioavailability of the drug substance. Recently, pharmaceutical formulations have been developed especially for drugs that show poor bioavailability based upon the principle that bioavailability can be increased by increasing the surface area i.e., decreasing particle size. For example, U.S. Pat. No. 4,107,288 describes a pharmaceutical formulation having particles in the size range from 10 to 1,000 nm in which the active material is supported on a crosslinked matrix of macromolecules. U.S. Pat. No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

The compositions are comprised of in general, a compound of Formula (I) in combination with at least one pharmaceutically acceptable excipient. Acceptable excipients are non-toxic, aid administration, and do not adversely affect the therapeutic benefit of the compound of Formula (I). Such excipient may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. Preferred liquid carriers, particularly for injectable solutions, include water, saline, aqueous dextrose, and glycols.

Compressed gases may be used to disperse a compound of this invention in aerosol form. Inert gases suitable for this purpose are nitrogen, carbon dioxide, etc.

Other suitable pharmaceutical excipients and their formulations are described in *Remington's Pharmaceutical Sciences,* edited by E. W. Martin (Mack Publishing Company, 18th ed., 1990).

The amount of the compound in a formulation can vary within the full range employed by those skilled in the art. Typically, the formulation will contain, on a weight percent (wt%) basis, from about 0.01-99.99 wt% of a compound of Formula (I) based on the total formulation, with the balance being one or more suitable pharmaceutical excipients. Preferably, the compound is present at a level of about 1-80 wt%. Representative pharmaceutical formulations containing a compound of Formula (I) are described in Example 14.

### EXAMPLES

The following preparations and examples are given to enable those skilled in the art to more clearly understand and to practice the present invention. They should not be considered as limiting the scope of the invention, but merely as being illustrative and representative thereof. Numbers in brackets refer to the CPD # in Table I.

### Example 1

### 5-Amino-1-(4-fluorophenyl)-4-[3-{3-(morpholin-4-yl)prop-1-ynyl}benzoyl]pyrazole (3)

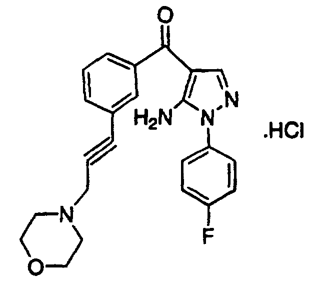

### Step 1

*n*-Butyllithium (214 ml, 340 mmol, 1.6 M solution in hexane) was added dropwise to a solution of acetonitrile (23.8 ml, 460 mmol) in dry tetrahydrofuran (1000 ml) at -78 °C. After stirring the reaction mixture for 20 min., a solution of 4-bromobenzoyl chloride in dry tetrahydrofuran (50 ml) was added dropwise over 20 min. After 1 h, saturated ammonium chloride was added (200 ml) and the reaction mixture was allowed to warm to room temperature. The product was extracted into ether and washed with 1N hydrochloric acid (400 ml). The organics were removed *in vacuo* and the residue was redissolved in ethyl acetate. Ammonium hydroxide was added to give a solid which was filtered, redissolved in ethyl acetate and washed with 2 N hydrochloric acid. The organic layer was washed with brine, dried over sodium sulfate and concentrated *in vacuo* to give 2-(3-bromobenzoyl)- acetonitrile (16.6 g) as a solid.

### Step 2

A mixture of 2-(3-bromobenzoyl)acetonitrile (16.5 g, 73.6 mmol) and N,N-diphenylformamidine (14.5 g, 73.6 mmol) in xylene (100 ml) was heated at reflux under a nitrogen atmosphere. After 3 h, the reaction mixture was cooled to room temperature and diluted with ether to give 2-(3-bromobenzoyl)-3-phenylaminoacrylonitrile (17.9 g) as a solid.

### Step 3

A mixture of 4-fluorophenylhydrazine (4.25 g, 33.7 mmol) and 2-(3-bromobenzoyl)-3-phenylaminoacrylonitrile (10.0 g, 30.7 mmol) in ethanol (100 ml) was heated at reflux under a nitrogen atmosphere. After 4 h, the reaction mixture was cooled to room temperature, diluted with hexane to give 5-amino-4-(3-bromobenzoyl)-1-(4-fluorophenyl)pyrazole (9.7 g) as a solid.

Replacing 4-fluorophenylhydrazine with 2,4-difluorophenylhydrazine in step 3 above gave 5-amino-4-(3-bromobenzoyl)-1-(2,4-difluorophenyl)pyrazole.

### Step 4

A mixture of 5-amino-4-(3-bromobenzoyl)-1-(4-fluorophenyl)pyrazole (1.3 g, 4.16 mmol), 4-(prop-2-ynyl)morpholine (2.1 g, 16.6 mmol) [prepared by adding to a solution of morpholine (14.7 ml, 168 mmol) in ether (50 ml), propargyl bromide (7.5 ml, 84 mmol) in ether (50 ml) dropwise over 30 min. and heating the reaction mixture to reflux. After 16 h, the reaction mixture was cooled to room temperature and filtered through a Buchner funnel. The filtrate was concentrated and purified by flash chromatography (gradient elution, 20-100% EtOAc/hexane) to give 4-(prop-2-ynyl)morpholine (5.0 g)], bis(triphenylphosphine)-palladium chloride (0.29 g, 0.42 mmol) and copper iodide (0.079 g, 0.42 mmol) in diisopropylamine (60 ml) was heated at 70 °C under argon. After 10 h, the reaction mixture was cooled to room temperature, diluted with ethyl acetate, washed with brine and dried over sodium sulfate. The organics were removed *in vacuo*. The crude product was purified by flash chromatography (elution gradient, EtOAc-5% MeOH/EtOAc with 0.2 % NH₄OH) to give 5-amino-1-(4-fluorophenyl)-4-[3-(3-morpholin-4-ylprop-1-ynyl)benzoyl]- pyrazole which was converted to the hydrochloride salt and recrystallized from a mixture of methanol/ethyl acetate/hexane to give (1.4 g) of the pure product.

Proceeding as described in Example 1 above but substituting 4-(prop-2-ynyl)-morpholine in Step 4 with:
1-(prop-2-ynyl)-4-methylpiperazine,
1-(prop-2-ynyl)piperidine,
2-propyn-1-ol,
1-dimethylamino-2-propyne, and
2-methyl-3-butyn-2-ol; gave 5-amino-1-(4-fluorophenyl)-4-{3-[3-(4-methylpiperazin-1-yl)prop-1-ynyl]benzoyl}pyrazole.2HCl (8),
5-amino-1-(4-fluorophenyl)-4-[3-{3-(piperidin-1-yl)prop-1-ynyl}benzoyl]pyrazole.HCl (9),
5-amino-1-(4-fluorophenyl)-4-[3-(3-hydroxyprop-1-ynyl)benzoyl]pyrazole (7),
5-amino-4-[3-(3-dimethylaminoprop-1-ynyl)benzoyl]-1-(4-fluorophenyl)pyrazole.HCl (12), and
5-amino-1-(4-fluorophenyl)-4-[3-(3-hydroxy-3-methyl-but-1-ynyl)benzoyl]pyrazole (87), respectively.

Proceeding as described in Example 1 above but substituting 4-fluorophenylhydrazine in Step 3 with 2,4-difluorophenylhydrazine, and 4-(prop-2-ynyl)morpholine in Step 4:
with 3-ethynylpyridine gave
   5-amino-1-(2,4-difluorophenyl)-4-[3-(3-pyridylethynyl)benzoyl]pyrazole (88),
with 3-(S,S-dioxo-thiomorpholin-4-yl)-1-propyne gave
   5-amino-1-(2,4-difluorophenyl)-4-[3-{3-(S,S-dioxo-thiomorpholin-4-yl)-1-propynyl} benzoyl]pyrazole (89), and
with 1-ethynylcyclopentanol gave
   5-amino-1-(2,4-difluorophenyl)-4-[3-{2-(1-hydroxycyclopentyl)ethynyl}benzoyl]pyrazole (94).

### Example 2

### 5-Amino-1-(4-fluorophenyl)-4-[3-(3-morpholin-4-ylpropyl)benzoyl]pyrazole hydrochloride (6)

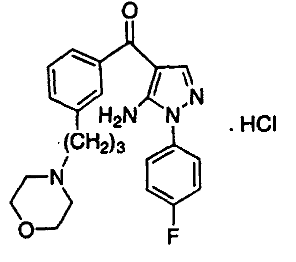

A mixture of 5-amino-1-(4-fluorophenyl)-4-[3-(3-morpholin-4-ylprop-1-ynyl)-benzoyl]pyrazole (0.45 g, 1.0 mmol) [prepared as described in Example 1] and 5 % Pd/C (0.07 g) in ethanol (20 ml) was stirred under hydrogen atmosphere. After 16 h, the reaction mixture was filtered through Celite® and the filtrate was concentrated *in vacuo*. The crude product was purified by flash chromatography (elution gradient, EtOAc-15 % MeOH/EtOAc with 0.2 % NH₄OH). The product was converted to the hydrochloride salt and recrystallized from a mixture of methanol/ethyl acetate to give 5-amino-1-(4-fluorophenyl)-4-[3-(3-morpholin-4-ylpropyl)benzoyl]pyrazole.HCl (0.3 g, mpt. 211.9-212.6 °C) as a solid.

Proceeding as described in Example 2 above, but substituting of 5-amino-1-(4-fluorophenyl)-4-[3-(3-morpholin-4-ylprop-1-ynyl)benzoyl]pyrazole with:
5-amino-1-(4-fluorophenyl)-4-{3-[3-(4-methylpiperazin-1-yl)prop-1-ynyl]benzoyl}pyrazole,
5-amino-1-(4-fluorophenyl)-4-[3-{3-(piperidin-1-yl)prop-1-ynyl}benzoyl]pyrazole,
5-amino-1-(4-fluorophenyl)-4-[3-(3-hydroxyprop-1-ynyl)benzoyl]pyrazole,
5-amino-4-[3-(3-dimethylaminoprop-1-ynyl)benzoyl]-1-(4-fluorophenyl)pyrazole,
5-amino-1-(4-fluorophenyl)-4-[3-(3-hydroxy-3-methyl-1-butynyl)benzoyl]pyrazole,
5-amino-1-(2,4-difluorophenyl)-4-[3-(3-pyridylethynyl)benzoyl]pyrazole,
5-amino-1-(2,4-difluorophenyl)-4-[3-{3-(S,S-dioxo-thiomorpholin-4-yl)-1-propynyl}benzoyl]pyrazole,
5-amino-1-(4-fluorophenyl)-4-[3-{2-(1-hydroxycyclopentyl)ethynyl}benzoyl]pyrazole, and
5-amino-1-(2,4-difluorophenyl)-4-[3-{2-(1-hydroxycyclopentyl)ethynyl}benzoyl]pyrazole gave
5-amino-1-(4-fluorophenyl)-4-{3-[3-(4-methylpiperazin-1-yl)propyl]benzoyl}pyrazole (30);
5-amino-1-(4-fluorophenyl)-4-[3-(3-piperidin-1-ylpropyl)benzoyl]pyrazole (32);
5-amino-1-(4-fluorophenyl)-4-[3-(3-hydroxypropyl)benzoyl]pyrazole;
5-amino-4-[3-(3-dimethylaminopropyl)benzoyl]-1-(4-fluorophenyl)pyrazole;
5-amino-1-(4-fluorophenyl)-4-[3-(3-hydroxy-3-methylbutyl)benzoyl]pyrazole (90),
5-amino-1-(2,4-difluorophenyl)-4-[3-(3-pyridylethyl)benzoyl]pyrazole (91),
5-amino-1-(2,4-difluorophenyl)-4-[3-{3-(S,S-dioxo-thiomorpholin-4-yl)propyl}benzoyl]pyrazole (92),
5-amino-1-(4-fluorophenyl)-4-[3-{2-(1-hydroxycyclopentyl)ethyl}benzoyl]pyrazole (93), and
5-amino-1-(2,4-difluorophenyl)-4-[3-{2-(1-hydroxycyclopentyl)ethyl}benzoyl]pyrazole (94) respectively.

### Example 3

### 5-Amino-1-(4-fluorophenyl)-4-[3-{2-(marpholin-4-yl)ethoxy}benzoyl]-pyrazole hydrochloride (14)

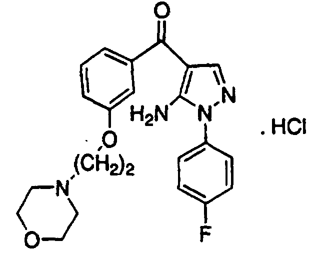

### Step 1

A mixture of methyl 3-hydroxybenzoate (8.0 g, 56 mmol) and 4-(2-chloroethyl)-morpholine hydrochloride (15.7 g, 84 mmol) and potassium carbonate (11.5 g, 83 mmol) in toluene (50 ml) was heated at reflux. After 4 days, the reaction mixture was cooled to room temperature and diluted with ethyl acetate. The organic layer was washed with water and then extracted with dilute hydrochloric acid. The acidic layer was separated, basified with 5 N sodium hydroxide and the product was extracted into ethyl acetate. The organics were removed *in vacuo* and the residue was purified by flash chromatography (elution gradient 3 % acetone/methylene chloride) to give methyl 3-(2-morpholin-4-ylethoxy)benzoate (9.0 g) as an oil.

### Step 2

Lithium diisopropylamide (18.8 ml, 37 mmol, 2.0 M solution in heptane/ tetrahydrofuran/ethylbenzene) was added dropwise to a solution of acetonitrile (1.58 g, 37 mmol) in dry tetrahydrofuran (50 ml) at -78 °C. After stirring the reaction mixture for 30 min., a solution of methyl 3-(2-morpholin-4-ylethoxy)benzoate in dry tetrahydrofuran (50 ml) was added dropwise over 10 min. After 15 min., water was added and the reaction mixture was allowed to warm to room temperature. The aqueous layer was separated and acidified with dilute hydrochloric acid to pH 7. The product was extracted into ethyl acetate and washed with water and brine and dried over magnesium sulfate. The organics were removed *in vacuo* to give 2-[3-(2-morpholin-4-ylethoxy)phenyl]acetonitrile (5.0 g) as an oil which was used in the next step without further purification.

### Step 3

A mixture of 2-[3-(2-morpholin-4-ylethoxy)phenyl]acetonitrile (5.0 g) and N,N-diphenylformamidine (5.0 g, 25.5 mmol) in xylene (150 ml) was heated at 100 °C under a nitrogen atmosphere. After 3 h, the reaction mixture was cooled to room temperature and diluted with hexane to give 2-[3-(2-morpholin-4-ylethoxy)benzoyl]-3-phenylamino-acrylonitrile (5.0 g) as a solid.

### Step 4

A mixture of 4-fluorophenylhydrazine (1.0 g, 6.8 mmol) and 2-[3-(2-morpholin-4-ylethoxy)-benzoyl]-3-phenylaminoacrylonitrile (2.0 g, 5.3 mmol) in ethanol (30 ml) was heated at reflux under a nitrogen atmosphere. After 6 h, the reaction mixture was cooled to room temperature and diluted with water. The product was extracted into ethyl acetate and the organic layer was washed with brine, dried over sodium sulfate and concentrated *in vacuo.* Purification by flash chromatography (elution gradient: CH₂Cl₂-3%MeOH/CH₂Cl₂) gave 5-amino-1-(4-fluorophenyl)-4-[3-(2-morpholin-4-ylethoxy)benzoyl]pyrazole which was converted to the hydrochloride salt (0.7 g, mpt. 191.6-192.5 °C).

Replacing 4-fluorophenylhydrazine in Step 4 above with:
2-fluorophenylhydrazine, and
2,6-dichlorophenylhydrazine, respectively were obtained:
5-amino-1-(2-fluorophenyl)-4-[3-(2-morpholin-4-ylethoxy)benzoyl]pyrazole (97), and
5-amino-1-(2,6-dichlorophenyl)-4-[3-(2-morpholin-4-ylethoxy)benzoyl]pyrazole (98).

### Example 4

### 5-Amino-1-(4-fluorophenyl)-4-[3-(pyridin-3-yl)benzoyl]pyrazole (20)

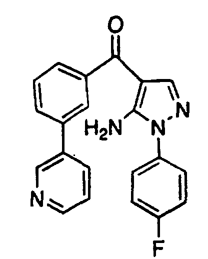

### Step 1

A mixture of 5-amino-4-(3-bromobenzoyl)-1-(4-fluorophenyl)pyrazole (0.9 g, 2.5 mmol) [prepared as described in Example 1 above], pyridine-3-boronic acid, 1,3-propanediol cyclic ester (0.5 g, 3 mmol), potassium phosphate (0.8 g, 3.73 mmol) and tetrakistriphosphine palladium (0.3 g, 0.25 mmol) in dioxane (20 ml) was heated at 85 °C under argon. After 16 h, the reaction mixture was cooled to room temperature and poured into brine. The product was extracted into ethyl acetate, dried over sodium sulfate and filtered. The organic layer was removed *in vacuo* and the residue was purified by flash chromatography (elution gradient: 40-80% ethyl acetate/hexane) to give 5-amino-1-(4-fluorophenyl)-4-[3-(pyridin-3-yl)benzoyl]- pyrazole (0.50 g) which was recrystallized from ethyl acetate (mpt. 222.2-223.0).

Treatment of 5-amino-1-(4-fluorophenyl)-4-[3-(pyridin-3-yl)benzoyl]pyrazole with methyl iodide in ethyl acetate gave 5-amino-1-(4-fluorophenyl)-4-[3-(N-methylpyridinium-3-yl)benzoyl]pyrazole iodide(59).

Substitution of 5-amino-4-(3-bromobenzoyl)-1-(4-fluorophenyl)pyrazole with 5-amino-4-(3-bromobenzoyl)-1-(2,4-difluorophenyl)pyrazole in Step 1 above followed by conversion to the hydrochloride salt gave 5-amino-1-(2,4-difluorophenyl)-4-[3-(pyridin-3-yl)benzoyl]pyrazole.HCl (99).

### Example 5

### 5-Amino-4-[3-(2-aminosulfonylethyl)benzoyl]-1-(4-fluorophenyl)pyrazole(50)

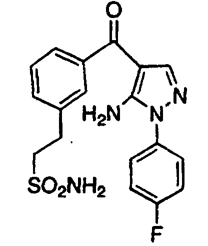

### Step 1

A mixture of 5-amino-4-(3-bromobenzoyl)-1-(4-fluorophenyl)pyrazole (1.5 g, 4.14 mmol) [prepared as described in Example 1 above], vinylsulfonamide (1.33 g, 12.4 mmol), bis(triphenylphosphine)palladium chloride (0.3 g, 0.42 mmol) and triethylamine (6 ml, 43 mmol) in dimethylformamide (18 ml) was heated at 100 °C under argon. After 16 h, the reaction mixture was cooled to room temperature and poured into 1 N hydrochloric acid. The product was extracted into ethyl acetate, washed with brine, dried over sodium sulfate and filtered. The organic layer was removed *in vacuo* and the residue was purified by flash chromatography (elution gradient: 40-80% ethyl acetate/hexane) to give 5-amino-4-[3-(2-aminosulfonylethenyl)benzoyl]-1-(4-fluorophenyl)pyrazole which was recrystallized from a mixture of methanol/ethyl acetate/hexane to give 0.78 g of the desired product.

### Step 2

A mixture of 5-amino-4-[3-(2-aminosulfonylethenyl)benzoyl]-1-(4-fluorophenyl)-pyrazole (2.1 g, 5.43 mmol) and palladium hydroxide (0.6 g) in methanol (150 ml) was shaken in a Parr apparatus under hydrogen atmosphere at 50 psi. After 4 days, the reaction mixture was filtered through Celite® and the filtrate was concentrated. The residue was purified by flash chromatography (elution gradient: 40-100% ethyl acetate/hexane) to give a crude product which was recrystallized from methanol/ethyl acetate/hexane to give 5-amino-4-[3-(2-aminosulfonylethyl)benzoyl]-1-(4-fluorophenyl)pyrazole (0.95 g, mpt. 170-170.4 °C) as a solid.

Replacement of vinylsulfonamide in Step 1 above with vinylmethylsulfone gave:
5-amino-4-[3-(2-methylsulfonylethyl)benzoyl]-1-(4-fluorophenyl)pyrazole (100).

### Example 6

### 5-Amino-1-(4-fluorophenyl)-4-[3-(morpholin-4-ylmethylcarbonyl)benzoyl]pyrazole(18)

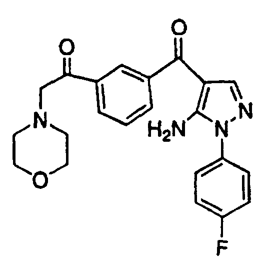

### Step 1

A mixture of 5-amino-4-(3-bromobenzoyl)-1-(4-fluorophenyl)pyrazole (3.5 g, 9.7 mmol) [prepared as described in Example 1 above], tributyl-(1-ethoxyvinyl)tin (4.3 ml, 12.36 mmol) and tetrakis(triphenylphosphine)palladium (1.0 g, 0.87 mmol) in dimethylformamide (25 ml) was heated at 95 °C under argon. After 16 h, the reaction mixture was cooled to room temperature and 10% aqueous hydrochloric acid (25 ml) was slowly added. After 30 min., the reaction mixture was diluted with ethyl acetate and filtered through Celite®. The organic layer was separated and washed with brine, dried over sodium sulfate and concentrated *in vacuo*. The residue was purified by flash chromatography (elution gradient: 10-60% ethyl acetate/hexane) to give 5-amino-4-[3-(1-ethoxyvinyl)benzoyl]-1-(4-fluorophenyl)pyrazole which was dissolved in tetrahydrofuran (50 ml). 1 N hydrochloric acid (20 ml) was added and the reaction mixture was stirred at room temperature for 16 h. The organic layer was separated, washed with brine, dried over sodium sulfate and concentrated *in vacuo.* The crude product was purified by flash chromatography (elution gradient: 20-50% ethyl acetate/hexane) and then was recrystallized from a mixture of ethyl acetate/hexane to give 5-amino-4-[3-acetylbenzoyl]-1-(4-fluorophenyl)pyrazole (2.0 g).

### Step 2

To a suspension of copper bromide (2.2 g, 9.85 mmol) in a (1:1) mixture of ethyl acetate/methylene chloride (100 ml) at reflux was added a solution of 5-amino-4-[3-acetylbenzoyl]-1-(4-fluorophenyl)pyrazole (1.6 g, 4.95 mmol) in methylene chloride (25 ml) under nitrogen. After 16 h, the reaction mixture was concentrated and the residue was partitioned between aqueous sodium bisulfite and ethyl acetate. The organic layer was separated, washed with brine, dried over sodium sulfate and concentrated *in vacuo*. The residue was purified by flash chromatography (elution gradient: 10-40% ethyl acetate/hexane) to give 5-amino-4-[3-(2-bromoacetyl)benzoyl]-1-(4-fluorophenyl)pyrazole (0.47 g) as a solid.

### Step 3

To a solution of morpholine (0.25 ml, 2.79 mmol) in dimethylformamide (5 ml) was added a solution of 5-amino-4-[3-(2-bromoacetyl)benzoyl]-1-(4-fluorophenyl)pyrazole (0.22 g, 0.56 mmol) in dimethylformamide (5 ml). After 16 h, the reaction mixture was poured into brine and the product was extracted into ethyl acetate. The organic layer was separated, washed with brine, dried over sodium sulfate and concentrated *in vacuo*. The residue was purified by flash chromatography (elution gradient: ethyl acetate-10% methanol/ethyl acetate) to give 5-amino-1-(4-fluorophenyl)-4-[3-(morpholin-4-ylmethylcarbonyl)benzoyl]pyrazole (0.05 g) as a solid.

### Example 7

### 5-amino-1-(4-fluorophenyl)-4-[3-(2-hydroxyethyl)benzoyl]pyrazole (118)

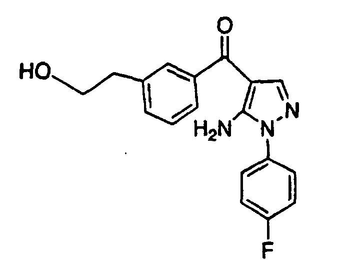

### Step 1

To a solution of 3-bromophenylacetic acid (10 g, 46.5 mmol) in tetrahydrofuran (100 ml) at 0 °C was added diborane (70 ml, 1.0 M solution in tetrahydrofuran). The reaction mixture was allowed to warm to room temperature. After 16 h, the reaction mixture was cooled to 0 °C and water was added dropwise (50 ml). The organic layer was separated and washed with brine, dried over sodium sulfate and concentrated *in vacuo*. The residue was purified by flash chromatography (elution gradient: 40-60% ethyl acetate/hexane) to give 3-(2-hydroxyethyl)bromobenzene (9.0 g).

### Step 2

To a solution of 3-(2-hydroxyethyl)bromobenzene (4.0 g, 20 mmol) in methylene chloride (100 ml) at 0 °C was added a solution of *tert*-butyldimethylsilyl chloride (3.6 g, 24 mmol), dimethylaminopyridine (0.61 g, 5 mmol) and triethylamine (3.6 ml, 25.9 mmol). After 1 h, the reaction mixture was washed with brine, saturated ammonium chloride, dried over sodium sulfate and concentrated *in vacuo.* The residue was purified by flash chromatography (elution gradient: 0-10% hexane/ethyl acetate) to give 3-(2-*tert*-butyl-dimethylsiloxyethyl)bromobenzene (6.0 g).

### Step 3

A mixture of ethyl (ethoxymethylene)cyanoacetate (26 ml, 154 mmol) and 4-fluorophenyl hydrazine (19.4 g, 154 mmol) in ethanol (125 ml) was heated at reflux. After 16 h, the reaction mixture was cooled to room temperature. The solid was filtered off and dried to give 5-amino-4-ethylcarboxy-1-(4-fluorophenyl)pyrazole (28 g) which was suspended in a mixture of 1 N lithium hydroxide (100 ml) and methanol (250 ml). The reaction mixture was heated at reflux. After 16 h, the reaction mixture was filtered through a sinter funnel and the filtrate was acidified with 2 N hydrochloric acid (65 ml). The solid was filtered off and dried to give 5-amino-4-carboxy-1-(4-fluorophenyl)pyrazole (21 g).

### Step 4

A mixture of 5-amino-4-carboxy-1-(4-fluorophenyl)pyrazole (15 g, 68 mmol), aldrathiol-2 (14.9 g, 68 mmol) and triphenylphosphine (17.8 g, 68 mmol) in acetonitrile (2 l) was stirred at room temperature. After 16 h, the product was filtered off and dried to give 5-amino-1-(4-fluorophenyl)-4-(2-pyridylthiocarboxy)pyrazole (14 g).

### Step 5

Into an oven dried flask containing magnesium turnings (0.386 g, 15.9 mmol) and tetrahydrofuran (10 ml) was added 3-(2-*tert*-butyldimethylsiloxyethyl)bromobenzene (5.0 g, 15.9 mmol) and the reaction mixture was heated at reflux. After 3 h, the reaction mixture was cooled to room temperature and 5-amino-1-(4-fluorophenyl)-4-(2-pyridylthiocarboxy)pyrazole (2.37 g, 7.6 mmol) was added and the stirring was continued for 16 h. The reaction mixture was concentrated *in vacuo*. The residue was dissolved in ethyl acetate and washed with aqueous ammonium chloride and brine and dried over sodium sulfate. The organics were removed *in vacuo* and the residue was purified by flash chromatography (elution gradient: 10-30% ethyl acetate/hexane) to give 5-amino-1-(4-fluorophenyl)-4-[3-(2-*tert*-butyldimethylsiloxyethyl)benzoyl]pyrazole (1.20 g).

### Step 6

To a solution of 5-amino-1-(4-fluorophenyl)-4-[3-(2-*tert*-butyldimethylsiloxyethyl)-benzoyl]pyrazole (1.2 g, 3.0 mmol) in tetrahydrofuran (25 ml) was added tetrabutyl-ammonium fluoride (3.6 ml, 3.6 mmol, 1 M solution in tetrahydrofuran). After 1 h, the reaction mixture was poured into brine and the product was extracted into ethyl acetate. The organic layer was dried over sodium sulfate, filtered and concentrated *in vacuo*. The residue was purified by flash chromatography (elution gradient: 40-100 % ethyl acetate/hexane) to give 5-amino-1-(4-fluorophenyl)-4-[3-(2-hydroxyethyl)benzoyl]pyrazole (0.8 g).

### Example 8

Synthesis of 5-amino-1-(4-fluorophenyl)- 4-(3-[4-methylpiperazin-1-yl)ethyl)-benzoyl]pyrazole dihydrochloride (31)

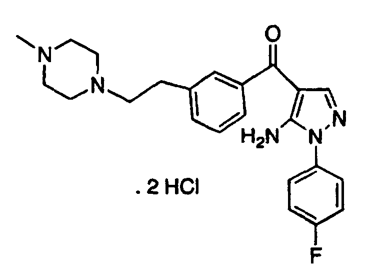

### Step 1

To a solution of 5-amino-1-(4-fluorophenyl)-4-[3-(2-hydroxyethyl)benzoyl]pyrazole (0.8 g, 2.5 mmol) in pyridine (10 ml) was added methanesulfonyl chloride (0.29 ml, 3.7 mmol). After 2 h, the reaction mixture was poured into 2 N hydrochloric acid (40 ml) and the product was extracted into ethyl acetate. The organic layer was washed with brine, dried over sodium sulfate, filtered and concentrated *in vacuo*. The residue was purified by flash chromatography (elution gradient: 40-100 % ethyl acetate/hexane) to give 5-amino-1-(4-fluorophenyl)-4-[3-(2-methanesulfonyloxyethyl)benzoyl]pyrazole (0.87 g).

### Step 2

A mixture of 5-amino-1-(4-fluorophenyl)-4-[3-(2-methanesulfonyloxyethyl)-benzoyl]pyrazole (0.22 g, 0.55 mmol), N-methylpiperazine (0.18 ml, 1.64 mmol) and potassium carbonate (0.22 g, 1.64 mmol) in dimethylformamide (10 ml) was heated at 70 °C. After 4 h, the reaction mixture was cooled to room temperature, poured into water and the product was extracted into ethyl acetate. The organic layer was washed with brine, dried over sodium sulfate, filtered and concentrated *in vacuo*. The residue was purified by flash chromatography (elution gradient: ethyl acetate- 20% methanol/ethyl acetate) to 5-amino-1-(4-fluorophenyl)-4-{3-[4-methylpiperazin-1-yl)ethyl)benzoyl]pyrazole which was converted to the hydrochloride salt (mpt. 272.9-273.9).

### Example 9

### 5-Amino-4-[3-(2-aminoethyl)benzoyl]-1-(4-fluorophenyl)pyrazole hydrochloride (47)

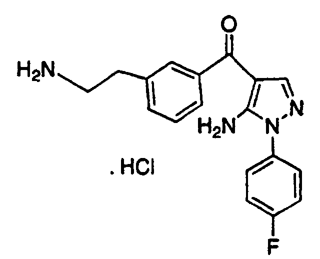

### Step 1

A mixture of 5-amino-1-(4-fluorophenyl)-4-[3-(2-methanesulfonyloxyethyl)-benzoyl]pyrazole (0.40 g, 0.99 mmol), sodium azide (0.19 ml, 2.97 mmol) and potassium carbonate (0.41 g, 2.97 mmol) in dimethylformamide (15 ml) was stirred at room temperature. After 16 h, the reaction mixture was poured into brine and the product was extracted into ethyl acetate. The organic layer was dried over sodium sulfate, filtered and concentrated *in vacuo*. The residue was purified by flash chromatography (elution gradient: 20-50 % ethyl acetate/hexane) to 5-amino-1-(4-fluorophenyl)-4-[3-(2-azidoethyl)benzoyl]pyrazole (0.32 g).

### Step 2

To a solution of 5-amino-1-(4-fluorophenyl)-4-[3-(2-azidoethyl)benzoyl]pyrazole (0.31 g, 0.9 mmol) in tetrahydrofuran (15 ml) was added triphenylphosphine (3.55 g, 1.36 mmol). After 48 h, the reaction mixture concentrated *in vacuo*. The residue was dissolved in 2 N sodium hydroxide and the product was extracted into ethyl acetate. The organic layer was dried over sodium sulfate, filtered and concentrated *in vacuo*. The product was converted to its hydrochloride salt and recrystallized from a mixture of methanol-ethyl acetate to give 5-amino-4-[3-(2-aminoethyl)benzoyl]-1-(4-fluorophenyl)pyrazole hydrochloride salt (0.22 g).

### Example 10

### 5-Amino-4-[3-(tert-butoxycarbonylmethyloxy)benzoyl]-1-(4-fluorophenyl)pyrazole

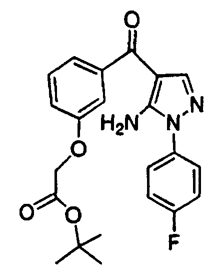

### Step 1

Into an oven dried flask containing magnesium turnings (0.408 g, 17 mmol) and tetrahydrofuran (10 ml) was added 3-bromoanisole (3.1 g, 17 mmol ) and the reaction mixture was heated at reflux. After 2 h, the reaction mixture was cooled to room temperature and 5-amino-1-(4-fluorophenyl)-4-(2-pyridylthiocarboxy)pyrazole (1.5g, 4.8 mmol) was added and the stirring was continued for 1 h. The reaction mixture was quenched with water and the product was extracted into ethyl acetate. The organic layer was washed with aqueous ammonium chloride and brine and dried over sodium sulfate. The organics were removed *in vacuo* and the residue was filtered and washed with hexane to give 5-amino-1-(4-fluoro-phenyl)-4-(3-methoxybenzoyl)pyrazole (1.20 g).

### Step 2

To an ice-cooled solution of 5-amino-1-(4-fluorophenyl)-4-(3-methoxybenzoyl)-pyrazole (3.0 g, 10.0 mmol) in methylene chloride (25 ml) was added boron tribromide (51 ml, 51 mmol, 1 M solution in methylene chloride). After 1 h, the reaction mixture was poured into brine and the product was extracted into ethyl acetate. The organic layer was dried over sodium sulfate, filtered and concentrated *in vacuo* to give 5-amino-1-(4-fluorophenyl)-4-[3-hydroxybenzoyl]pyrazole (2.4 g).

### Step 3

A mixture of 5-amino-1-(4-fluorophenyl)-4-[3-hydroxybenzoyl]pyrazole (1.0 g, 3.3 mmol), *tert*-butyl bromoacetate (1.4 g, 7.2 mmol) and potassium carbonate (1 g, 7.2 mmol ) in acetonitrile was heated at 70 °C overnight. The reaction mixture was cooled, diluted with ethyl acetate and filtered. The filtrate was concentrated *in vacuo* and the residue was purified by flash chromatography (elution gradient: 10% acetone/hexane) to give 5-amino-4-[3-(*tert*-butoxycarbonylmethyloxy)benzoyl]-1-(4-fluorophenyl)pyrazole (1.2 g) as a solid.

### Example 11

### 5-Amino-4-[3-carboxymethyloxy)benzoyl]-1-(4-fluorophenyl)-pyrazole (119)

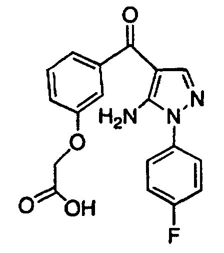

### Step1

A mixture of 5-amino-4-[3-(*tert*-butoxycarbonylmethyloxy)benzoyl]-1-(4-fluorophenyl)pyrazole (1.0 g, 3.3 mmol) and trifluoroacetic acid (15 ml, 194 mmol ) in methylene chloride (15 ml) was stirred overnight at room temperature. The organics were removed *in vacuo* and the residue was dissolved in toluene. The solution was concentrated and the residue was triturated between ethyl acetate and hexane to give 5-amino-4-[3-(carboxymethyloxy)benzoyl]-1-(4-fluorophenyl)pyrazole (0.8 g) as a solid.

### Example 12

### 5-Amino-1-(4-fluorophenyl)-4-[3-(methylaminocarbonylmethyloxy)-benzoyl]pyrazole (34)

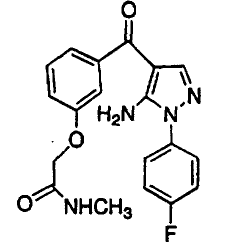

### Step1

To a solution of 5-amino-4-[3-(carboxymethyloxy)benzoyl]-1-(4-fluorophenyl)-pyrazole (0.5 g, 1.43 mmol) in tetrahydrofuran (10 ml) was added carbonyl diimidazole (0.3 g, 1.85 mmol) and the reaction mixture was heated at 60 °C. After 1 h, methylamine (10 ml, 5 mmol, 0.5 M solution in tetrahydrofuran) was added and reaction was continued at 60 °C overnight. The reaction mixture was cooled and diluted with ethyl acetate. The organic layer was separated and washed with brine and dried over sodium sulfate. The organics were removed *in vacuo* and the residue was purified by flash chromatography (elution gradient: 20-30% acetone/hexane) to give 5-amino-1-(4-fluorophenyl)-4-[3-(methylaminocarbonyl-methyloxy)benzoyl]pyrazole (0.25 g, mpt. 195.6-196.3 °C) as a solid.

Proceeding as described in Example 12 above, but substituting methylamine with:
morpholine gave 5-amino-1-(4-fluorophenyl)-4-[3-(morpholin-4-ylcarbonylmethyloxy)-benzoyl]pyrazole (35).

### Example 13

### 5-ammo-1-(4-fluorophenyl)-4-[3-{3-(morpholin-4-yl)propylamino}benzoyl]pyrazole (48)

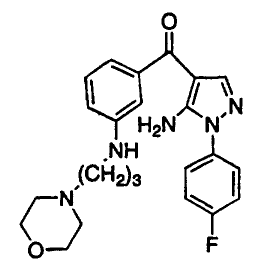

### Step 1

Benzoylacetonitrile (14.5 g, 10 mmol) was added to cold fuming nitric acid (50 ml) portionwise over 10 min. The reaction mixture was stirred for 15 min., and then poured into ice. The solid was filtered off and recrystallised from ethanol to give 2-(3-nitrobenzoyl)-acetonitrile (5.4 g) as a brown solid.

### Step 2

A mixture of 2-(3-nitrobenzoyl)acetonitrile (13.75 g, 72.3 mmol) and N,N-diphenylformamidine (14.2 g, 72.3 mmol) in xylene (200 ml) was heated at reflux under nitrogen atmosphere. After 3 h, the reaction mixture was cooled to room temperature and diluted with xylenes to give 2-(3-nitrobenzoyl)-3-phenylaminoacrylonitrile (15.7 g) as a yellow solid.

### Step 3

A mixture of 4-fluorophenylhydrazine (2.24 g, 15.57 mmol) and 2-(3-nitrobenzoyl)-3-phenylaminoacrylonitrile (4.15 g, 14.16 mmol) in ethanol (50 ml) was heated at reflux under nitrogen atmosphere. After 1 h, the reaction mixture was cooled to room temperature and stirred for an additional 3 h. The solid was filtered and dried to give 5-amino-1-(4-fluorophenyl)-4-(3-nitrobenzoyl)pyrazole (4.5 g) as a solid.

### Step 4

A mixture of 5-amino-1-(4-fluorophenyl)-4-(3-nitrobenzoyl)pyrazole (4.0 g, 24.52), Fe powder (3.84 g, 68 mmol) and ammonium chloride (3.84, 71.78 mmol) in ethanol (135 ml) and water (64 ml) was heated at reflux under nitrogen atmosphere. After 1 h, the reaction mixture was cooled to room temperature and stirred overnight. The reaction mixture was filtered through Celite® and the filtrate was concentrated *in vacuo*. The residue was partitioned between water and ethyl acetate. The organic layer was separated and washed with brine, dried over sodium sulfate and concentrated *in vacuo* to give 5-amino-4-(3-amino-benzoyl)-1-(4-fluorophenyl)pyrazole (3.5 g) as a solid.

### Step 5

5-amino-4-(3-aminobenzoyl)-1-(4-fluorophenyl)pyrazole (0.5 g, 1.6 mmol), 1-bromo-3-chloropropane (0.26 g, 1.6 mmol) and cesium carbonate (0.52 g, 1.6 mmol) in dimethylformamide (25 ml) was heated at 80 °C. After 2 days, the reaction mixture was cooled to room temperature and diluted with ethyl acetate. The organic layer was washed with brine, dried over sodium sulfate and concentrated *in vacuo*. The residue was purified by flash chromatography (elution gradient: 20 % acetone/hexanes) to give 5-amino-4-[3-(3-chloropropylamino)benzoyl]-1-(4-fluorophenyl)pyrazole (0.2 g) as a solid.

### Step 6

A mixture of 5-amino-4-[3-(3-chloropropylamino)benzoyl]-1-(4-fluorophenyl)-pyrazole (0.05 g, 0.13 mmol), morpholine (0.1 ml, 1.1 mmol), potassium carbonate (0.1 g) and potassium iodide (0.1 g) in acetonitrile (3 ml) was heated at reflux. After 2 days, the reaction mixture was poured into brine and the product was extracted into ethyl acetate. The organic layer was separated, washed with brine, dried over sodium sulfate and concentrated *in vacuo*. The residue was purified by flash chromatography (elution gradient: 3 % MeOH/CH₂Cl₂) to give 5-amino-1-(4-fluorophenyl)-4-[3-{3-(morpholin-4-yl)propylamino}-benzoyl]pyrazole as a solid.

### Example 14

### 5-amino-1-(4-fluorophenyl)-4-[3-{2-(piperidin-1-yl)ethoxy}benzoyl]pyrazole HCl salt (81)

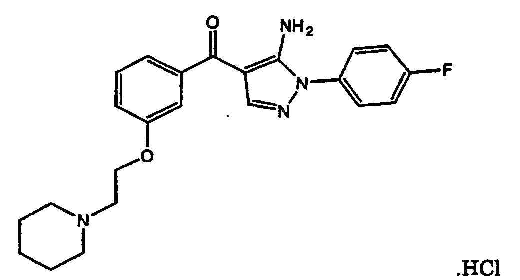

### Step 1

5-Amino-1-(4-fluorophenyl)-4-[3-hydroxybenzoyl]pyrazole, from Example 10, step 2, 1.5g, 5.05mmol) was combined with toluene (50mL). 2-bromoethanol (1.79 mL, 25.23 mmol) was added and then the reaction mixture was cooled to 0°C. Triphenylphosphine (5.425g, 20.69mmol) and diethyl azodicarboxylate (3.26 mL, 20.69 mmol) were then added. The reaction was allowed to warm to room temperature. After stirring for 16 hours, the reaction was quenched with a saturated aqueous solution of NH₄Cl, extracted with ethyl acetate, dried (MgSO₄), filtered, and concentrated under vacuum. The product (5-amino-1-(4-fluorophenyl)-4-[3-(2-bromoethoxy)benzoyl]pyrazole) was purified by column chromatography on silica gel using 40:1 CH₂Cl₂/MeOH then stirred with ether for 20 minutes, filtered and dried to give 0.785g of product.

### Step 2

5-amino-1-(4-fluorophenyl)-4-[3-(2-bromoethoxy)benzoyl]pyrazole (0.6g, 1.48 mmol) was combined with piperidine (1.47 mL, 14.8 mmol) and ethanol (10mL) and heated at reflux for 16 hrs. The reaction mixture was concentrated under vacuum. The resulting residue was partitioned between a saturated aqueous solution of NaHCO₃ and ethyl acetate and extracted three times with ethyl acetate. The organic extracts were dried (MgSO₄), filtered, concentrated under vacuum and purified by column chromatography on silica gel using 16:1 CH₂Cl₂/MeOH. Dissolving the product in ethyl acetate then adding hydrochloric acid (1.0M, 1.0 equivalent) formed the hydrochloric salt which was filtered and dried to give 0.413g of 5-amino-1-(4-fluorophenyl)-4-[3-{2-(piperidin-1-yl)ethoxy}benzoyl]pyrazole.HCl (mpt 210.2-211.2°C).

Proceeding as in Step 2 but replacing piperidine with diethanolamine, dimethylamine, N-methylpiperazine, 2-aminoethanol, bis(2-methoxyethyl)amine, diethylamine, methylamine, ammonia, and 3-oxopyridazine the following compounds were obtained.

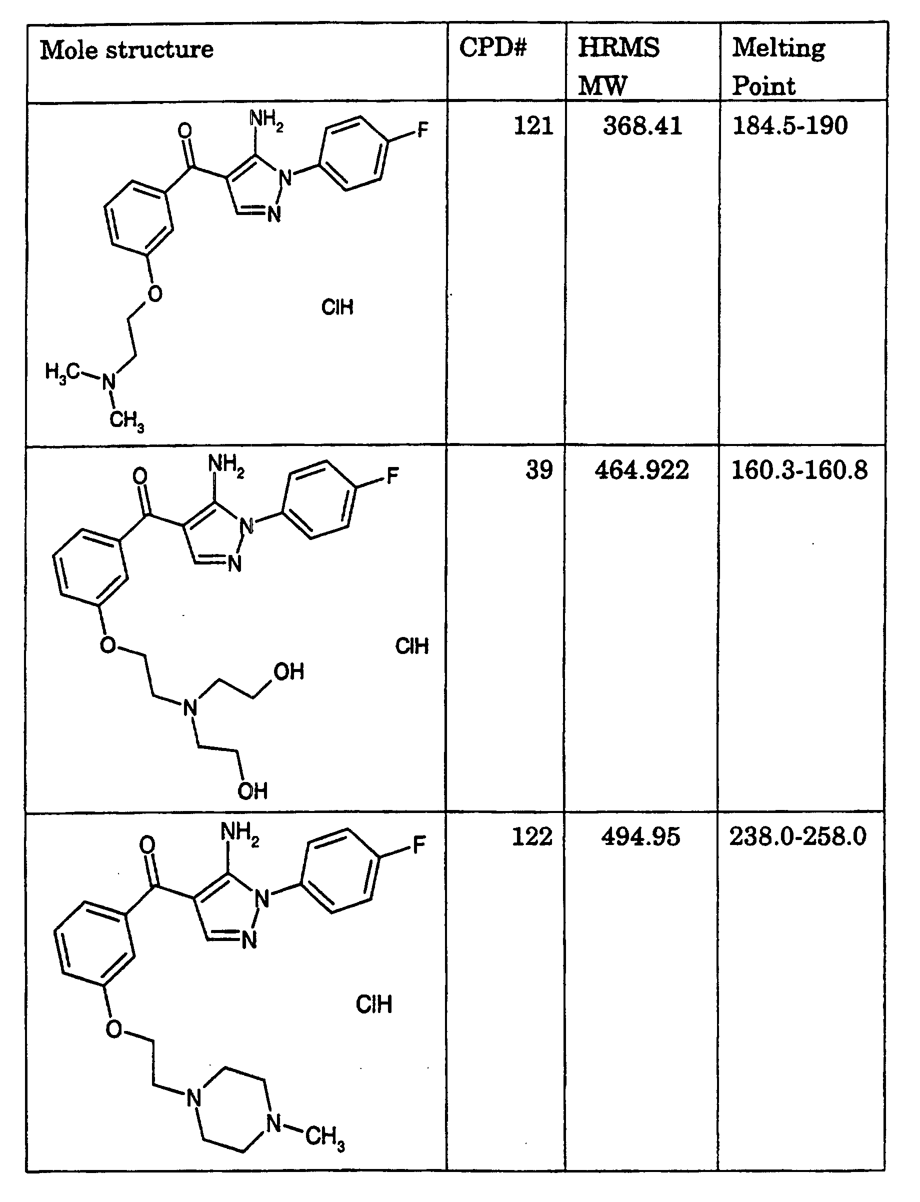

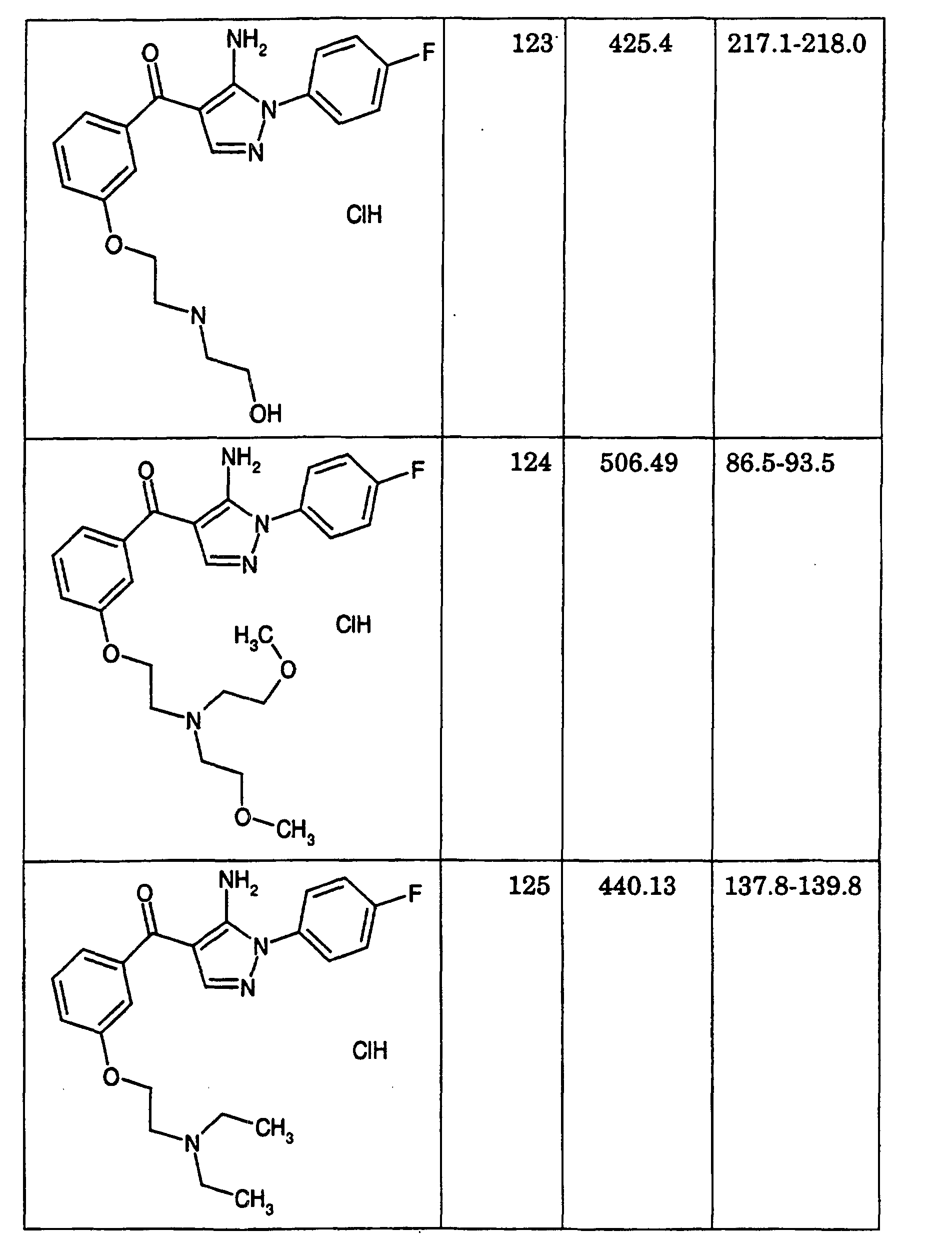

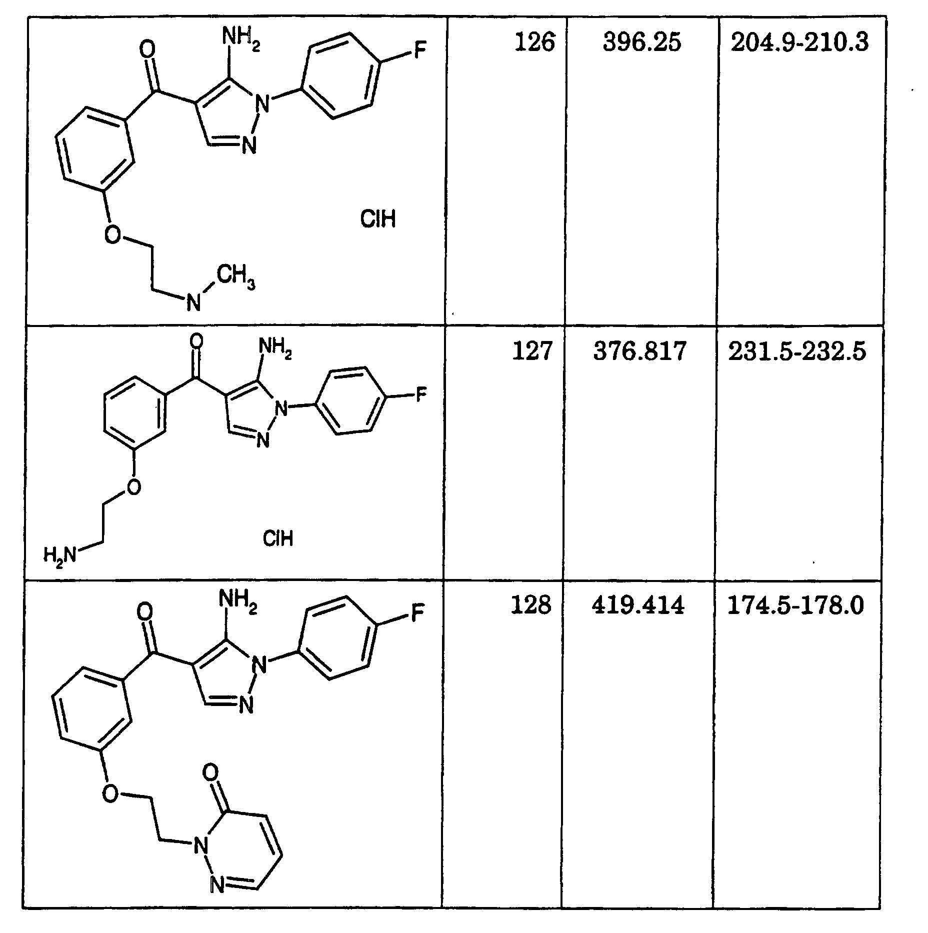

### Example 15

### 5-amino-1-(4-fluorophenyl)-4-[3-(pyridin-2-ylmethoxy)benzoyl]pyrazole (82)

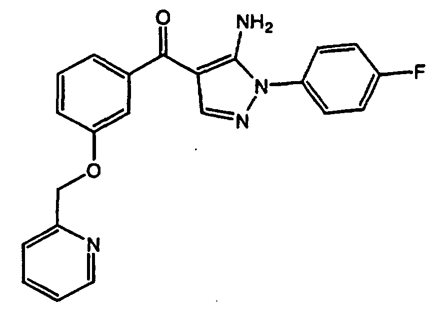

5-Amino-1-(4-fluorophenyl)-4-[3-hydroxybenzoyl]pyrazole, from Example 10, step 2, (0.5g, 1.68 mmol), 2-pyridylcarbinol (0.81 mL, 8.41 mmol), triphenylphospine (1.81g, 6.9 mmol), and diethylazodicarboxylate (1.09 mL, 6.9 mmol)) were combined in toluene (50mL). The reaction mixture was stirred for 16 hours then quenched with a saturated aqueous solution of NH₄Cl and extracted three times with ethyl acetate. The product was then extracted from the ethyl acetate into a 10% aqueous solution of HCl. The aqueous layer was then neutralized with NaOH and extracted with ethyl acetate. The organic extracts were dried (MgSO₄), filtered, and concentrated under vacuum. The residue was purified by column chromatography on silica gel using 1:1 hexane/ethyl acetate to give 0.165g of 5-amino-1-(4-fluorophenyl)-4-[3-(pyridin-2-ylmethoxy)benzoyl]pyrazole (mpt. 176.1-177.3°C).

Replacing 2-pyridylcarbinol with glycolic acid, 1-(2-hydroxyethyl)-2-pyrrolidinone and 4-hydroxypiperidine gave the following compounds.

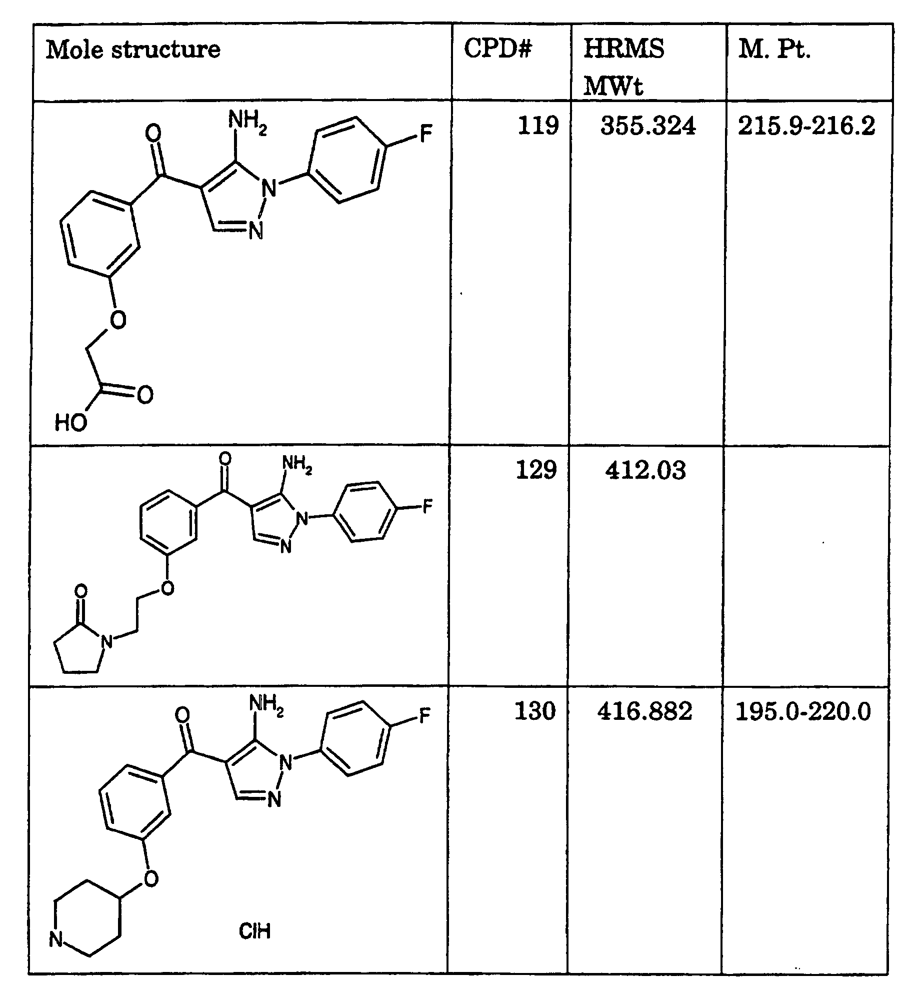

### Example 16

### 5-amino-1-(4-fluorophenyl)-4-[3-isopropylaminocarbonyloxybenzoyl]pyrazole (83)

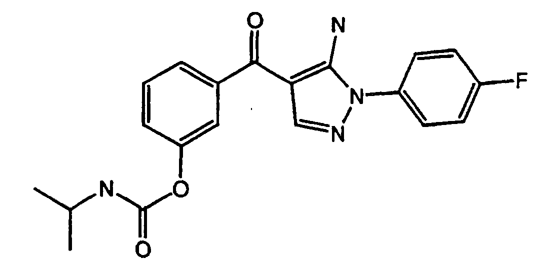

5-Amino-1-(4-fluorophenyl)-4-[3-hydroxybenzoyl]pyrazole, from Example 10, step 2, (0.30g, 1.01 mmol) was combined with K₂CO₃ (0.418g, 3.03 mmol) and THF (6mL). The mixture was cooled in an ice bath and then isopropyl isocyanate (0.12 mL, 1.21 mmol) was added. The reaction was allowed to warm to room temperature and stirred for 16 hours. The reaction mixture was quenched with water, extracted into ethyl acetate, dried (MgSO₄), filtered, and concentrated to dryness. The residue was stirred in methanol and dichloromethane for one hour then filtered to give 0.118g of 5-amino-1-(4-fluorophenyl)-4-[3-isopropylaminocarbonyloxybenzoyl]pyrazole (mpt. 225.2-230.1°C).

Replacing isopropyl isocyanate with ethyl isocyanate was made 5-amino-1-(4-fluorophenyl)-4-[3-ethylaminocarbonyloxybenzoyl]pyrazole (84). Mpt. 201.2-202.8°C.

### Example 17

### 5-Amino-1-(4-fluorophenyl)-4-[3-iodo benzoyl]pyrazole

### Step 1

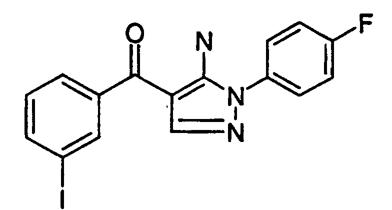

*n*-Butyllithium (30.5 ml, 76 mmol, 2.5 M solution in hexane) was added dropwise to a cooled (0°C) solution of diisopropylamine (10.6 ml, 76 mmol) in 10 ml dry tetrahydrofuran. Once addition was complete, the solution was kept at 0 °C for 10 minutes and was then cooled to -50 °C. This cold LDA solution was then added to a -50 °C solution of acetonitrile (2.37 ml, 45.3 mmol) and ethyl 4-iodobenzoate (10.0g, 36.2 mmol) in dry tetrahydrofuran (18 ml). Once addition was complete, the reaction was stirred at -50 °C for 3 hours and was subsequently warmed to 0°C. Saturated ammonium chloride was added (20 ml) and the reaction mixture was allowed to warm to room temperature. The product was extracted into ether and washed with 1N hydrochloric acid (50 ml). The organics were washed with brine (50 ml), dried over MgSO₄ and then concentrated *in vacuo* to a red oil. The oil was purified through a small plug of silica gel using 3:1-2:1 hexanes/ethyl acetate as eluent. Concentration of the column fractions *in vacuo* gave 2-(3-iodobenzoyl)- acetonitrile (8.3 g) as a yellow oil.

### Step 2

A mixture of 2-(3-iodobenzoyl)acetonitrile (36.2 g, 133.5 mmol) and N,N-diphenylformamidine (26.2 g, 133.5 mmol) in toluene (200 ml) was heated at reflux under a nitrogen atmosphere. After 8 h, the reaction mixture was cooled to room temperature and diluted with ether (200 ml) to give 2-(3-iodobenzoyl)-3-phenylaminoacrylonitrile (31.2 g) as a solid.

### Step 3

A mixture of 4-fluorophenylhydrazine (26.6 g, 211 mmol) and 2-(3-iodobenzoyl)-3-phenylaminoacrylonitrile (79 g, 211 mmol) in ethanol (400 ml) was heated at reflux under a nitrogen atmosphere. After 30 minutes, the reaction mixture was cooled to room temperature, diluted with hexane to give 5-amino-4-(3-iodobenzoyl)-1-(4-fluorophenyl)pyrazole (75.1 g) as a solid.

Replacing 4-fluorophenylhydrazine with 4-methylphenyllhydrazine, 3-methoxyphenylhydrazine, 4-sulfamoylphenylhydrazine, 2,4-dimethylphenylhydrazine, 2-methylphenylhydrazine, 4-chloro-2-methylphenylhydrazine, 4-methylsulfonylphenylhydrazine, 2-ethylphenylhydrazine, and 2,4-difluorophenylhydrazine in Step 3 above gave respectively:
5-amino-4-(3-iodobenzoyl)-1-(4-methylphenyl)pyrazole,
5-amino-4-(3-iodobenzoyl)-1-(3-methoxyphenyl)pyrazole,
5-amino-4-(3-iodobenzoyl)-1-(4-sulfamoylphenyl)pyrazole,
5-amino-4-(3-iodobenzoyl)-1-(2,4-dimethylphenyl)pyrazole,
5-amino-4-(3-iodobenzoyl)-1-(2-methylphenyl)pyrazole,
5-amino-4-(3-iodobenzoyl)-1-(4-chloro-2-methylphenyl)pyrazole,
5-amino-4-(3-iodobenzoyl)-1-(4-methylsulfonylphenyl)pyrazole,
5-amino-4-(3-iodobenzoyl)-1-(2-ethylphenyl)pyrazol, and
5-amino-4-(3-iodobenzoyl)-1-(2,4-dinuorophenyl)pyrazole.

### Example 18

### 5-amino-1-(4-fluorophenyl)-4-[3-(1,2-dihydroxyethyl)benzoyl] pyrazole (85)

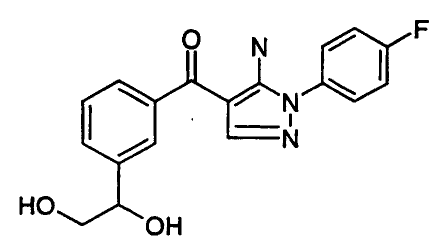

### Step 1

To a solution of 5-amino-1-(4-fluorophenyl)-4-[3-iodobenzoyl]pyrazole (10g, 24.6 mmol) in 100 ml dimethylformamide was added vinyltributytin (8.57g, 27.0 mmol) and tetrakistriphenylphosphine palladium (0) (1.42g, 1.23 mmol). The resulting solution was degassed with argon and subsequently warmed to 100°C for 12 hours.

The reaction was cooled to room temperature and was poured into 500 ml distilled water and was extracted 3x 100 ml 1:1 ether/ethyl acetate. The organics were washed with brine (150 ml), dried over MgSO₄ and then concentrated *in vacuo* to a brown oil. The oil was purified by flash column chromatography using 5:1-4:1 hexanes/ethyl acetate to remove impurities and 3:1-2:1 hexanes/ethyl acetate to elute the desired product. Concentration of the column fractions *in vacuo* gave 5-amino-1-(4-fluorophenyl)-4-[3-vinylbenzoyl] pyrazole (4.48 g) as a white solid.

### Step 2

To a suspension of 5-amino-1-(4-fluorophenyl)-4-[3-vinylbenzoyl] pyrazole (4.48g, 13.95 mmol) in 50 ml t-butanol was added N-methylmorpholine N-oxide (1.79g, 15.35 mmol) in 50 ml distilled water. To this mixture at room temperature was added a solution of 2.5% osmium tetraoxide in t-butanol (5.25 ml, 0.42 mmol). After 5 hours, the homogenous reaction was diluted with ethyl acetate (25 ml) and the organics were separated and washed with brine (25 ml), dried over MgSO₄ and then concentrated *in vacuo* to a brown oil. The oil was purified by flash column chromatography using 1:1 hexanes/ethyl acetate to remove impurities and ethyl acetate to elute the desired product. Concentration of the column fractions *in vacuo* gave 5-amino-1-(4-fluorophenyl)-4-[3-(1,2-dihydroxyethyl)benzoyl] pyrazole (4.48 g) as a white foam. The foam was triturated to a solid from hexanes (2.36 g).

Replacing 5-amino-1-(4-fluorophenyl)-4-[3-iodobenzoyl]pyrazole in Step 1 above with:
5-amino-1-(2,4-difluorophenyl)-4-[3-iodobenzoyl]pyrazole and
5-amino-1-(2-methylphenyl)-4-[3-iodobenzoyl]pyrazole, gave respectively
5-amino-1-(2,4-difluorophenyl)-4-[3-(1,2-dihydroxyethyl)benzoyl] pyrazole (103) and
5-amino-1-(2-methylphenyl)-4-[3-(1,2-dihydrozyethyl)benzoyl] pyrazole (109).

### Example 19

### 5-Amino-1-(2,4-difluorophenyl)-4-[3-(1-piperidinylmethyl)-benzoyl]pyrazole (86)

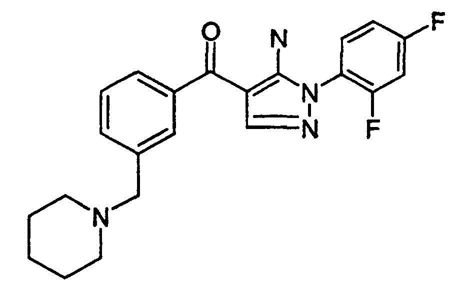

### Step 1:

To a suspension of 5-amino-1-(2,4-difluorophenyl)-4-[3-(1,2-dihydroxyethane)benzoyl] pyrazole (10.1g, 28 mmol) in 100 ml t-butanol was added 100 ml distilled water and sodium periodate (18.06g, 84 mmol). After 2 hours, the solid precipitate was collected by vacuum filtration and was washed with 300 ml distilled water and dried in vacuo to give 8.28 g of 5-amino-1-(2,4-difluorophenyl)-4-[3-formylbenzoyl] pyrazole as a white solid.

### Step 2:

To a solution of 5-amino-1-(2,4-difluorophenyl)-4-[3-formylbenzoyl] pyrazole (0.3 g, 0.92 mmol), piperidine (0.1 ml, 1.0 mmol), acetic acid (0.05 ml) in 1,2-dichloroethane (5 ml) was added sodium triacetoxyborohydride (0.29g, 1.37 mmol). After stirring at room temperature for 12 hours, the reaction was diluted with 10% hydrochloric acid and ethyl acetate (10 ml). The aqueous layer was separated and neutralized to pH 9 with sodium hydroxide and was then extracted with ethyl acetate. The combined organics were separated and washed with brine (25 ml), dried over MgSO₄ and then concentrated *in vacuo* to a brown oil. The oil was purified by flash column chromatography using 1:1 hexanes/ethyl acetate to remove impurities and ethyl acetate to elute the desired product. Concentration of the column fractions *in vacuo* gave 5-amino-1-(4-fluorophenyl)-4-[3-(1-piperidinylmethyl)-benzoyl] pyrazole as an oil (0.211g). The compound was triturated to a solid from hexanes/ethyl acetate.

Replacing piperidine in Step1 above with:
morpholine,
N-methylpiperazine,
4-hydroxypiperidine,
2-aminopyridine,
3-aminopyridine,
4-methylimidazole,
3-aminopyrazole, and
2-methylimidazole;
the following compounds were obtained

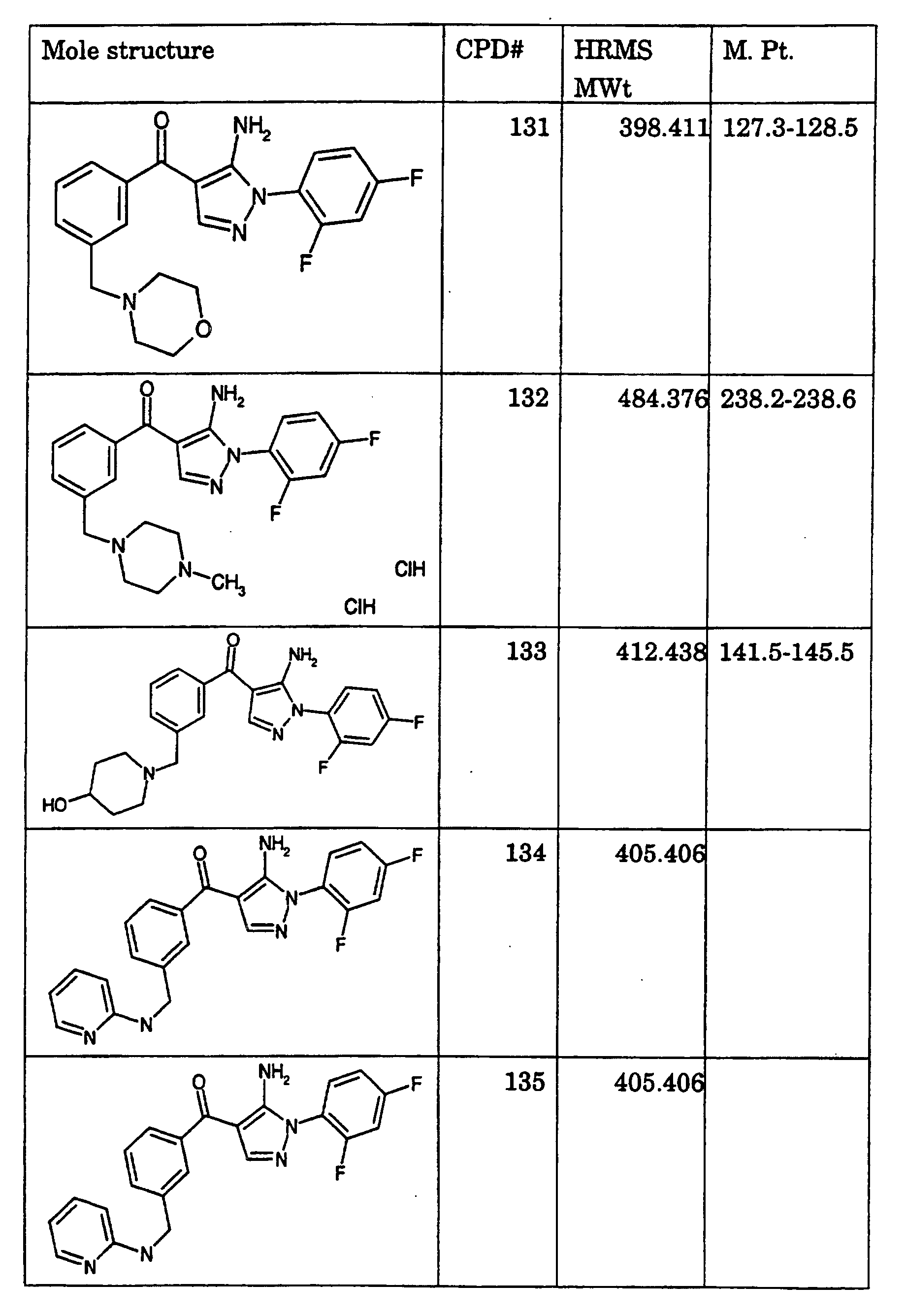

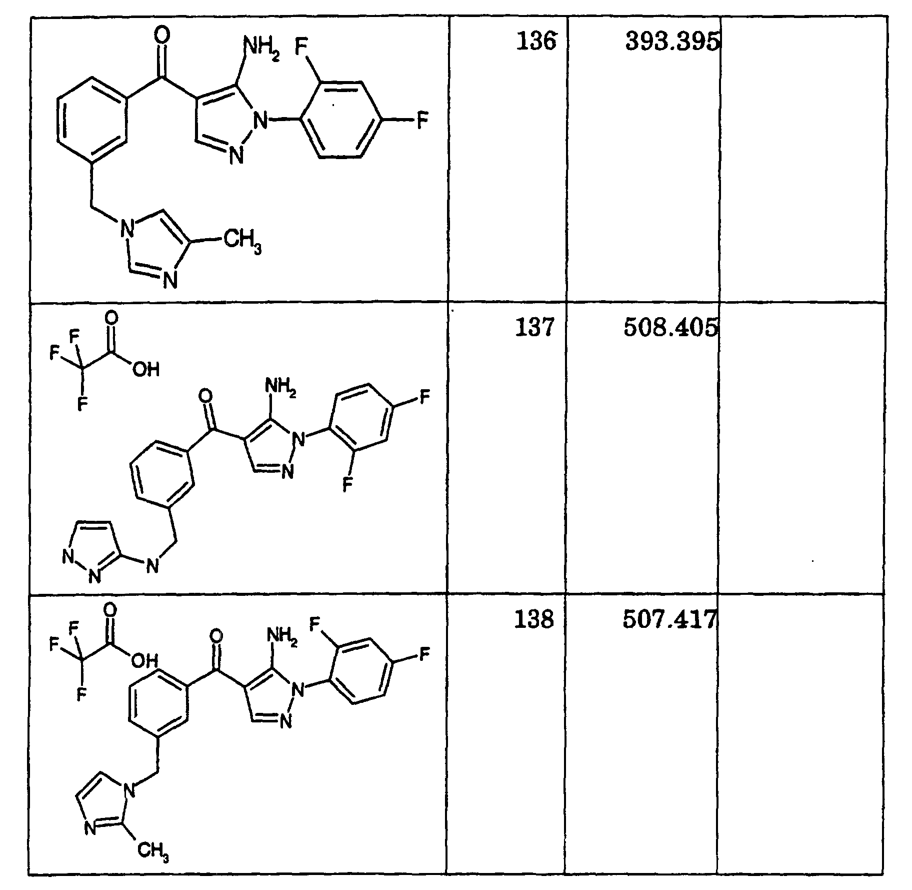

### Example 20

### 5-amino-1-(3-methylphenyl)-4-[3-{N-oxidopyridin-3-yl)}benzoyl]pyrazole (70)

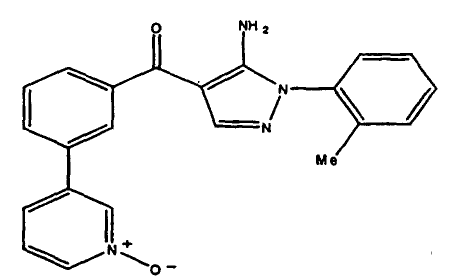

A mixture of 5-amino-4-(3-iodobenzoyl)-1-(2-methylphenyl)pyrazole, from Example 17 (0.98 g, 2.4 mmol), pinacol diboron (0.68 g, 2.7 mmol), [1,1'-bis(diphenylphoshino)ferrocene]dichloropalladium (0.2 g,.24mmol) and potassium acetate (0.72 g, 7.3mmol) in DMF (10 ml) was heated at 80 degrees, under argon. After 2 h, the reaction mixture was cooled to room temperature and 3-bromopyridine N-oxide (.47 g, 2.7 mmol), [1,1'-bis(diphenylphosphino)ferrocene]dichloropalladium (0.2 g, .24 mmol) and 2 M sodium carbonate (6.1 ml, 12.2 mmol) was added and heated to 80 degrees. After 16 h, the reaction mixture was cooled to room temperature, poured into brine and the product extracted into ethyl acetate. The organic layer was dried over sodium sulfate, filtered and then the solution was evaporated to dryness. The residue was purified by flash chromatography (gradient elution: ethyl acetate to 20 % methanol/ethyl acetate) to give, after recrystallization from methanol/ethyl acetate/hexane, 5-amino-1-(3-methylphenyl)-4-[3-(N-oxidopyridin-3-yl)benzoyl]pyrazole (0.57 g, mpt. 190.5-191.2).

Replacing 5-amino-4-(3-iodobenzoyl)-1-(2-methylphenyl)pyrazole/3-bromopyridine N-oxide with:
5-amino-4-(3-iodobenzoyl)-1-(4-methylphenyl)pyrazole/3-bromopyridine,
5-amino-4-(3-iodobenzoyl)-1-(3-methoxyphenyl)pyrazole/3-bromopyridine,
5-amino-4-(3-iodobenzoyl)-1-(4-sulfamoylphenyl)pyrazole/3-bromopyridine,
5-amino-4-(3-iodobenzoyl)-1-(2,4-dimethylphenyl)pyrazole/3-bromopyridine,
5-amino-4-(3-iodobenzoyl)-1-(2-methylphenyl)pyrazole/3-bromopyridine-N-oxide,
5-amino-4-(3-iodobenzoyl)-1-(4-chloro-2-methylphenyl)pyrazole/3-bromopyridine,
5-amino-4-(3-iodobenzoyl)-1-(4-methylsulfonylphenyl)pyrazole/3-bromopyridine,
5-amino-4-(3-iodobenzoyl)-1-(2-ethylphenyl)pyrazole/3-bromopyridine, and
5-amino-4-(3-iodobenzoyl)-1-(2,4-difluorophenyl)pyrazole/2-bromoimidazole,
gave respectively the following compounds (as their hydrochloride salts as appropriate):
5-amino-1-(4-methylphenyl)-4-[3-(pyridin-3-yl)benzoyl]pyrazole (65),
5-amino-1-(3-methoxyphenyl)-4-[3-(pyridin-3-yl)benzoyl]pyrazole (66),
5-amino-1-(4-sulfamoylphenyl)-4-[3-(pyridin-3-yl)benzoyl]pyrazole (68),
5-amino-1-(2,4-dimethylphenyl)-4-[3-(pyridin-3-yl)benzoyl]pyrazole (69),
5-amino-1-(2-methylphenyl)-4-[3-(N-oxidopyridin-3-yl)benzoyl]pyrazole (70),
5-amino-1-(4-chloro-2-methylphenyl)-4-[3-(pyridin-3-yl)benzoyl]pyrazole (73),
5-amino-1-(4-methylsulfonylphenyl)-4-[3-(pyridin-3-yl)benzoyl]pyrazole (75),
5-amino-1-(2-ethylphenyl)-4-[3-(pyridin-3-yl)benzoyl]pyrazole (76), and
5-amino-1-(2,4-difluorophenyl)-4-[3-(imidazol-2-yl)benzoyl]pyrazole (77).

### Example 21

### 5-amino-1-(2,4-difluorophenyl)-4-[N-oxidopyridin-3-yl)benzoyl]pyrazole (60)

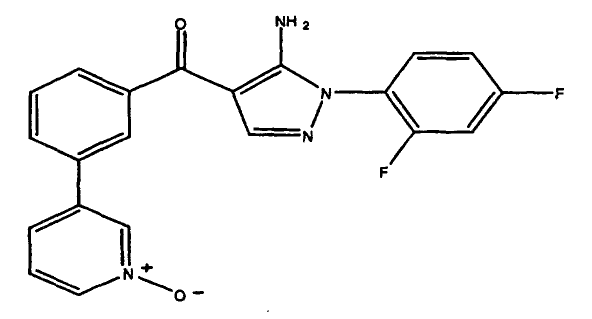

To a solution of 5-amino-1-(2,4-difluorophenyl)-4-[3-(pyridin-3-yl)benzoyl]pyrazole (4.6 g, 12.2 mmol) in dichloromethane (100ml) was added 3-chloroperoxybenzoic acid (5.6 g, 18.3 mmol) and the mixture was stirred at room temperature. After 4 h, a solution of 10 % aqueous sodium sulfite (50 ml) was added. After 0.5 h, the organic layer was separted, washed with brine, dried over sodium sulfate and filtered. The filtrate was concentrated to dryness and the residue purified by flash chromatography (gradient elution: ethyl acetate to 30 % methanol/ethyl acetate)to give, after recrystalliztion from methanol, 5-amino-1- (2,4-difluorphenyl)-4-[3-(N-oxidopyridin-3-yl) benzoyl]pyrazole (1.3g, Mpt. 251.1-251.7°C).

### Example 22

### 5-amino-1-(2,4-difluorophenyl)-4-[pyridin-4-yl)benzoyl]pyrazole (61)

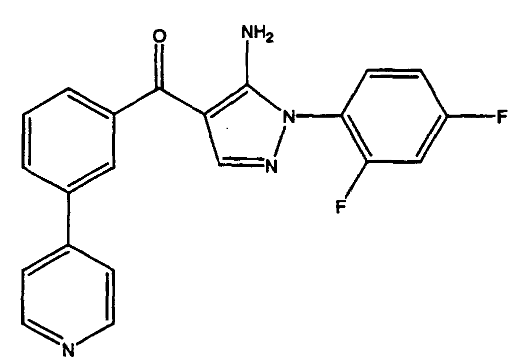

A mixture of 5-amino-4-(3-bromobenzoyl)-1-(2.4-difluorophenyl)pyrazole (.93 g, 2.5 mmol), 4-tributylstannylpyridine (1.0 g, 2.7 mmol) and bis(triphenylphosphine)palladium chloride (.17 g, 2.5 mmol) in DMF (15ml) was heated at 100 degrees under argon. After 16 h, the reaction mixture was cooled to room temperature and a solution of 10 % aqueous potassium flouride (30 ml) was added. After 1 h, the reaction mixture was poured into brine, extracted with ethyl acetate, dried over sodium sulfate, filtered and concentrated to dryness. The residue was purified by flash chromatography (gradient elution: 50-100 % ethyl acetate/hexane to 5 % methanol/ethyl acetate to give, after recrystallizaion from methanol/ethyl acetate, 5-amino-1-(2,4-difluorophenyl)-4-[3-(pyridin-4-yl)benzoyl]pyrazole (0.42 g, Mpt. 218-226 °C).

### Example 23

### 5-Amino-1-(2,4-dimethylphenyl)-4-[3-(pyridin-3-yl)benzoyl]pyrazole HCl salt (69)

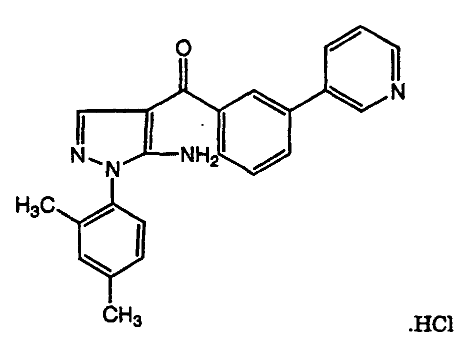

### Step 1

Into a solution of n-butyl lithium (165 ml, 264 mmol) in butyl ether (250 ml) at -78 degrees under nitrogen was added 3-bromopyridine (25.4 ml, 264 mmol). After 1 h, added diethylmethoxyborane (52ml, 396 mmol). The mixture was allowed to warm to room temperature. After 16 h, added water and brine, separated organic layer, dried over sodium sulfate, then concentrated. The resulting slurry was dissolved in isopropanol (500 ml), cooled and the product isolated by filtration give diethyl(3-pyridyl)borane (29.8g).

### Step 2

A mixture of diethyl(3-pyridyl)borane (176.4g, 1.2mole), methyl-3-iodobenzoate(262g, 1 mole), potassium phosphate (318.4 g, 1.5 mole) and tetrakistriphenlyphosphine palladium (0) (57.8g, .05 mole) in DMF (1000 ml) was heated at 80 degrees under argon. After 10 h, the mixture was diluted with water and extracted with ethyl acetate. The organic layer was filtered and washed with water. To the organic fraction was added concentrated HCL (65 ml). The organic layer was separated and extracted with aqueous HCl. The combined acid extractions were treated with ethyl acetate, followed by 50% aqueous sodium hydroxide (55 ml). The organic layer was separated, washed with water and saturated sodium bicarbonate solution, then dried over sodium sulfate. The solution was filtered and concentrated to give methyl-3-(pyridin-3-yl)benzoate (145.3g).

### Step 3

To a solution of methyl-3-(pyridin-3-yl)benzoate (126.2 g, 0.59 mole) in THF (600 ml) was added acetonitrile (37 ml, 0.71 mole) and the reaction was cooled to -40 degrees. A solution of lithium diisopropyl amide (590 ml, 1.18 mole) was added dropwise. After 30 minutes, added methanol (25 ml) and after another 30 minutes, added water (110 ml). Allowed the reaction mixture to warm to 10 degrees and added ethyl acetate. The layers were separated and the aqueous layer was acidified with 1 M HCl. The aqueous layer was extracted with ethyl acetate, diluted with hexane and washed with brine. The organic phase was concentrated, then combined with N,N'-diphenylformanidine (120 g, 0.61 mole) in 800 ml of ethyl acetate. The mixture was stirred at room temperature. After 3 days, the product was collected by filtration and recrystallized from isopropanol, hexane to give 2-(3-pyridin-3-yl)phenyl-3-phenylacrylonitrile (100 g).

### Step 4

A solution of 2-(3-pyridin-3-ylphenyl)-3-phenylacrylonitrile (1.0 g, 3 mmol) and 2,4-dimethyphenylhydrazine (0.4 g, 3 mmol) in ethanol (30 ml) was heated at reflux, under nitrogen. After 6 h, the reaction was cooled to room temperature, concentrated to dryness and the residue purified by flash column chromatography (elution gradient:40-100% ethyl acetate/hexane to 10% methanol/ethyl acetate). The purified residue was taken up in ethyl acetate and HCl/ether added to prepare the salt. After recrystallization from methanol/ethyl acetate was isolated 5-amino-1-(2,4-dimethylphenyl)-4-[3-(pyridin-3-yl)benzoyl]pyrazole hydrochloride salt (0.74 g, m.pt. 250.7-251.8).

Proceeding as above in Example 23, but replacing 2,4-dimethyphenylhydrazine in step 4 with:
phenylhydrazine,
2-methyl-4-chlorophenylhydrazine,
4-methoxyphenylhydrazine,
4-methylsulfonylphenylhydrazine,
2-ethylphenylhydrazine,
4-isopropylphenylhydrazine,
2-methoxyphenylhydrazine,
3-chloro-4-methylphenylhydrazine,
3-fluorophenylhydrazine, and
3-fluoro-6-methylphenylhydrazine respectively, the following compounds were obtained.

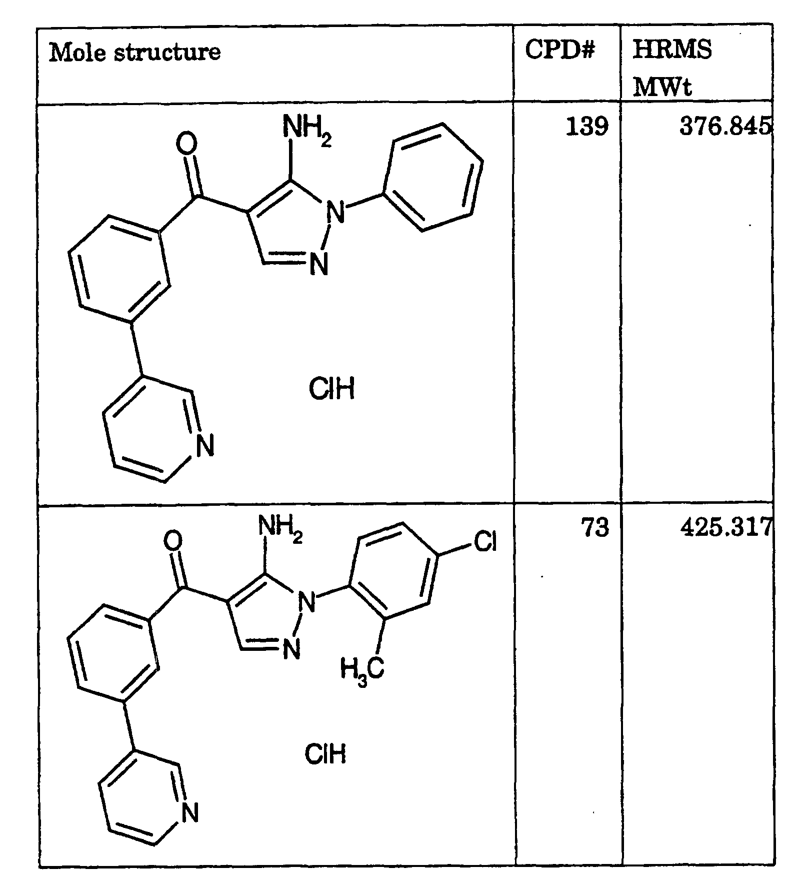

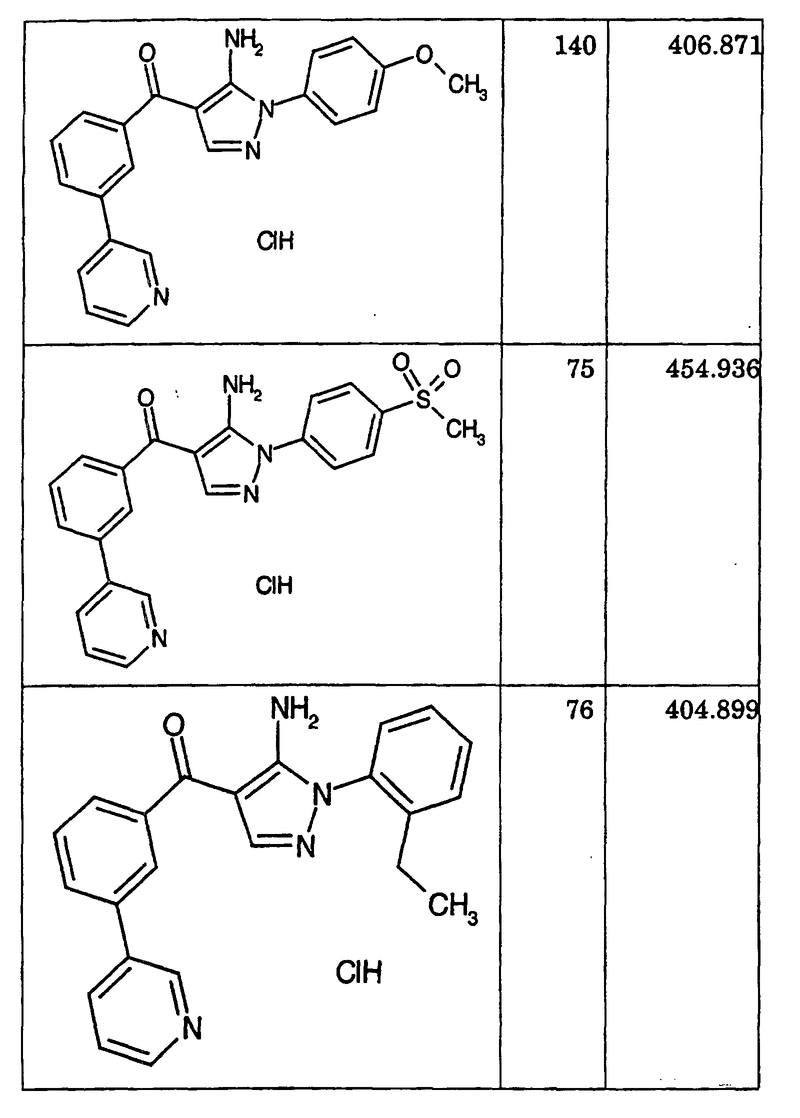

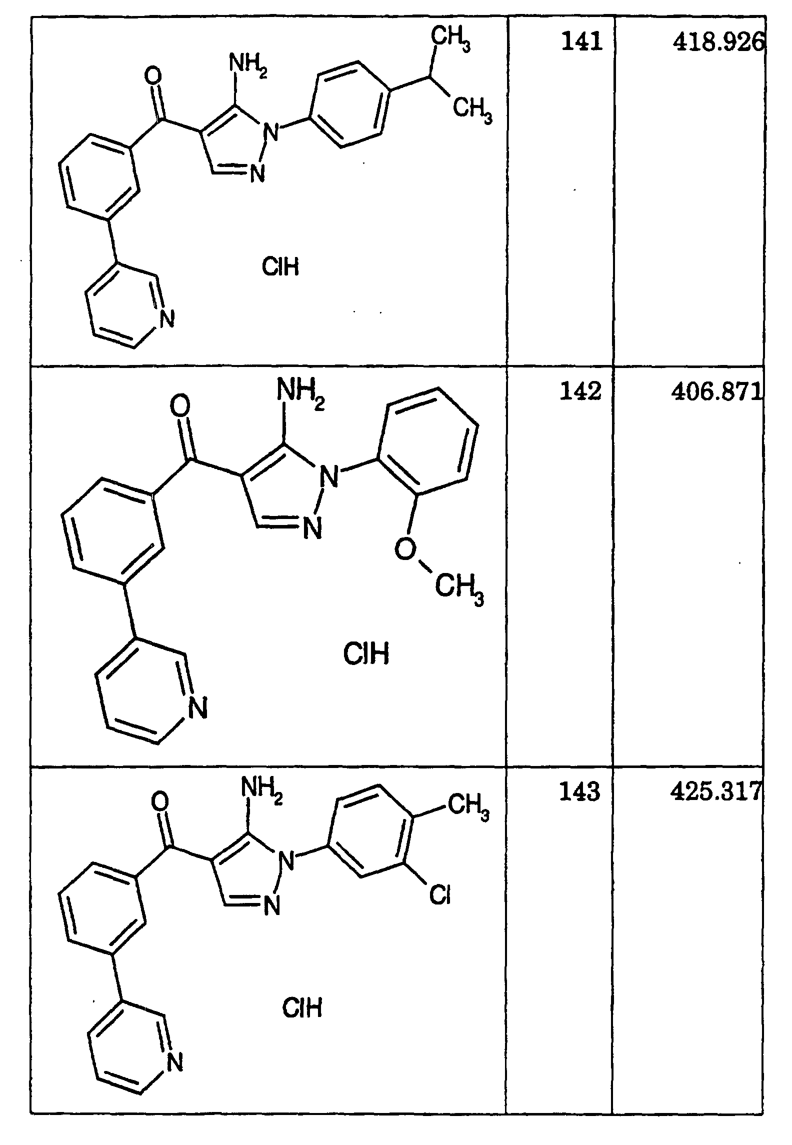

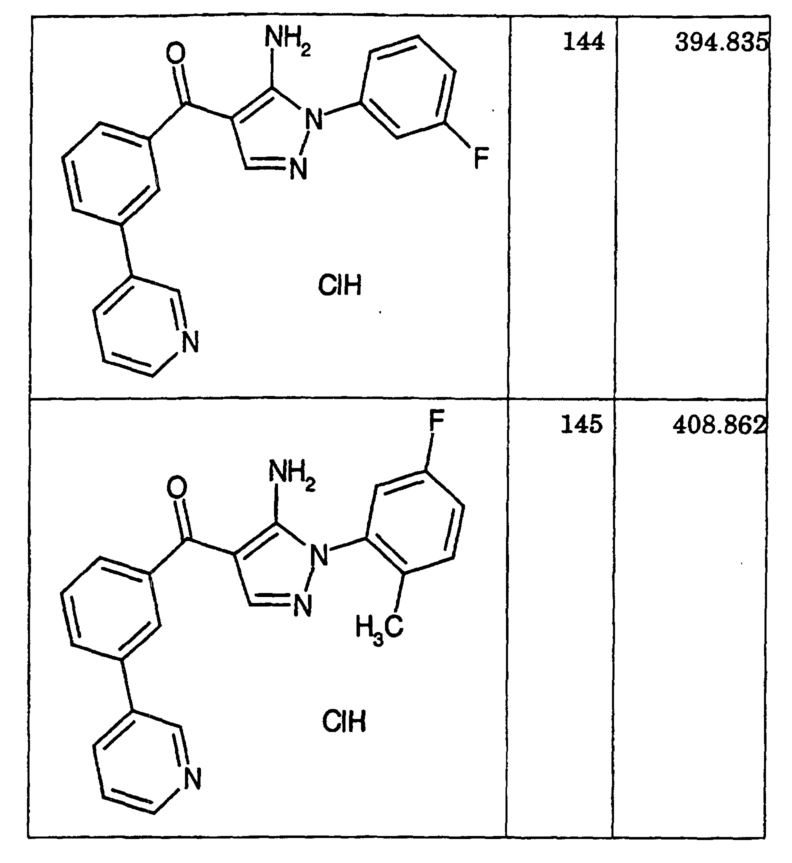

### Example 24

### 5-amino-1-(4-fluorophenyl)-4-[3-{2(R),3-dihydroxypropoxy}benzoyl] pyrazole (106)

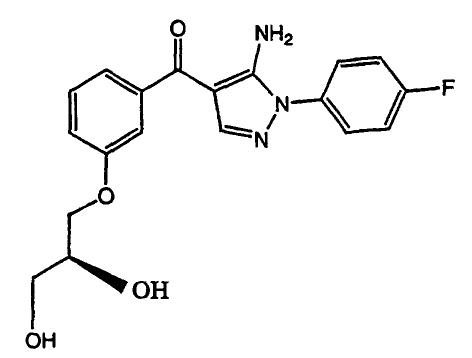

### Step 1

To a solution of 5-amino-4-(3-hydroxybenzoyl)-1-(4-fluorophenyl)pyrazole (0.5g, 1.68 mmol) in 5 ml dry dimethylformamide was added D-α, β-isopropylideneglycerol-γ-tosylate (0.72g, 2.52 mmol) followed by anhydrous potassium carbonate (0.695g, 5.04 mmol). The reation was warmed to 80 °C under argon. After 24 hours, the reaction was cooled to room temperature and an additional 500 mg of D-α,β-isopropylideneglycerol-γ-tosylate was added and the reaction was warmed back to 80 °C under argon. After 8 additional hours, the reaction was cooled to room temperature and diluted with distilled water (50 ml) and the product was extracted into ether. The combined organics were washed with brine (50 ml), dried over MgSO₄ and then concentrated *in vacuo* to a yellow oil. The oil was purified by flash column chromatography on silica gel using 2:1-1:1 hexanes/ethyl acetate as eluent. Concentration of the column fractions *in vacuo* gave 556 mg of the desired acetal.

### Step 2

To a solution of the acetal formed above (0.556 g, 1.35 mmol) in methanol (10 ml) was added distilled water (2 ml) and p-toluenesulfonic acid monohydrate (5 mg). The solution was warmed to 80 °C under an argon atmosphere. After 2 h, the reaction mixture was cooled to room temperature concentrated *in vacuo* to a yellow oil which was redissolved in ethyl acetate (50 ml) and saturated sodium bicarbonate (50 ml). The organic layer was separated, dried over MgSO₄ and then concentrated *in vacuo* to a white solid. Recrystallization from hexanes/ethyl acetate gave 196 mg of the desired diol (Mpt. 150.2-153.0°C).

### Example 25

### 5-amino-1-(4-fluorophenyl)-4-thenoyl-pyrazole (114)

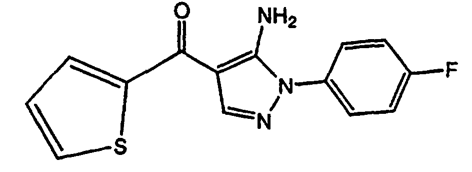

Proceeding as described in Example 1, step 2, but replacing 2-(3-bromobenzoyl)acetonitrile with 2-(2-thenoyl)acetonitrile and then following step 3 was obtained 5-amino-1-(4-fluorophenyl)-4-(2-thenoyl)-pyrazole.

Proceeding as described in Example 1, step 2, but replacing 2-(3-bromobenzoyl)acetonitrile with 2-(2-furanoyl)acetonitrile and then following step 3 was obtained 5-amino-1-(4-fluorophenyl)-4-(2-furanoyl)pyrazole (115).

Proceeding as described in Example 1, step 2, but replacing 2-(3-bromobenzoyl)acetonitrile with 2-(2-methyl-3-furanoyl)acetonitrile and substituting 4-fluorophenylhydrazine in step 3 with 2,4-difluorophenylhydrazine was obtained 5-amino-1-(2,4-difluorophenyl)-4-(2-methylfuran-3-oyl)-pyrazole (116).

Proceeding as described in Example 1, step 2, but replacing 2-(3-bromobenzoyl)acetonitrile with 2-(6-quinolinoyl)acetonitrile and substituting 4-fluorophenylhydrazine in step 3 with 2,4-difluorophenylhydrazine was obtained 5-amino-1-(2,4-difluorophenyl)-4-(6-quinolinoyl)-pyrazole.HCl (117) (mpt. 220-259.2).

### Example 26

The following are representative pharmaceutical formulations containing a compound of Formula (I).

| Tablet formulation | |
|---|---|
| The following ingredients are mixed intimately and pressed into single scored tablets. | |

| Ingredient | Quantity per tablet, mg |
|---|---|
| compound of this invention | 400 |
| cornstarch | 50 |
| croscarmellose sodium | 25 |
| lactose | 120 |
| magnesium stearate | 5 |

| Capsule formulation | |
|---|---|
| The following ingredients are mixed intimately and loaded into a hard-shell gelatin capsule. | |

| Ingredient | Quantity per capsule, mg |
|---|---|
| compound of this invention | 200 |
| lactose, spray-dried | 148 |
| magnesium stearate | 2 |

| Suspension formulation | |
|---|---|
| The following ingredients are mixed to form a suspension for oral administration. | |

| Ingredient | Amount |
|---|---|
| compound of this invention | 1.0 g |
| fumaric acid | 0.5 g |
| sodium chloride | 2.0 g |
| methyl paraben | 0.15 g |
| propyl paraben | 0.05 g |
| granulated sugar | 25.5 g |
| sorbitol (70% solution) | 12.85 g |
| Veegum K (Vanderbilt Co.) | 1.0 g |
| flavoring | 0.035 ml |
| colorings | 0.5 mg |
| distilled water | q.s. to 100 ml |

| Injectable formulation | |
|---|---|
| The following ingredients are mixed to form an injectable formulation. | |

| Ingredient | Amount |
|---|---|
| compound of this invention | 0.2 g |
| sodium acetate buffer solution, 0.4 M | 2.0 ml |
| HCl (1N) or NaOH (1N) | q.s. to suitable pH |
| water (distilled, sterile) | q.s. to 20 ml |

All of the above ingredients, except water, are combined and heated to 60-70 °C with stirring. A sufficient quantity of water at 60 °C is then added with vigorous stirring to emulsify the ingredients, and water then added q.s. to 100 g.

| Suppository formulation | |
|---|---|
| A suppository of total weight 2.5 g is prepared by mixing the compound of the invention with Witepsol® H-15 (triglycerides of saturated vegetable fatty acid; Riches-Nelson, Inc., New York), and has the following composition: | |
| compound of the invention | 500 mg |
| Witepsol® H-15 | balance |

### Example 27

### Inhibition Of p-38 (MAP) Kinase...In Vitro Assay

The p-38 MAP kinase inhibitory activity of compounds of this invention *in vitro* was determined by measuring the transfer of the γ-phosphate from γ-³³P-ATP by p-38 kinase to Myelin Basic Protein (MBP), using the a minor modification of the method described in Ahn, N. G.; et al. *J. Biol. Chem.* Vol. **266**(7), 4220-4227, (1991).

The phosphorylated form of the recombinant p38 MAP kinase was expressed with SEK-1 and MEKK in E. Coli and then purified by affinity chromatography using a Nickel column.

The phosphorylated p38 MAP kinase was diluted in kinase buffer (20 mM 3-(N-morpholino)propanesulfonic acid, pH 7.2, 25 mM β-glycerol phosphate, 5 mM ethylene glycol-bis(beta-aminoethyl ether)-N,N,N',N'-tetraacetic acid, 1mM sodium vanadate, 1mM dithiothreitol, 40mM magnesium chloride). Test compound dissolved in DMSO or only DMSO (control) was added and the samples were incubated for 10 min at 30 °C. The kinase reaction was initiated by the addition of a substrate cocktail containing MBP and γ-³³P-ATP. After incubating for an additional 20 min at 30 °C, the reaction was terminated by adding 0.75% phosphoric acid. The phosphorylated MBP was then separated from the residual γ-³³P-ATP using a phosphocellulose membrane (Millipore, Bedford, MA) and quantitated using a scintillation counter (Packard, Meriden, CT).

Compounds of the invention wer active in this assay. The p-38 inhibitory activities (expressed as IC₅₀ , the concentration causing 50 % inhibition of the p-38 enzyme being assayed) of some compounds of the invention are:

| CPD # | IC₅₀, µM | CPD # | IC₅₀, µM |
|---|---|---|---|
| 1 | 1.81 | 19 | 1.45 |
| 2 | 3.29 | 21 | 2.18 |
| 3 | 1.78 | 27 | 2.72 |
| 4 | 6.18 | 33 | 1.12 |
| 6 | 1.74 | 38 | 6.31 |
| 9 | 1.32 | 43 | 6.52 |
| 14 | 1.27 | 50 | 1.25 |

### Example 28

### Inhibition of LPS-Induced TNF-α Production In THP1 Cells....In Vitro Assay

The ability of the compounds of this invention to inhibit the TNF-α release was determined using a minor modification of the methods described in Blifeld, C. et al. *Transplantation*, Vol. **51**(2), 498-503, (1991).

### (a) Induction of TNF biosynthesis:

THP-1 cells were suspended in culture medium [RPMI (Gibco-BRL, Gailthersburg, MD) containing 15% fetal bovine serum, 0.02 mM 2-mercaptoethanol], at a concentration of 2.5 x 10⁶ cells/ml and then plated in 96 well plate (0.2 ml aliquots in each well). Test compounds were dissolved in DMSO and then diluted with the culture medium such that the final DMSO concentration was 5%. 20 µl aliquots of test solution or only medium with DMSO (control) were added to each well. The cells were incubated for 30 min., at 37 °C. LPS (Sigma, St. Louis, MO) was added to the wells at a final concentration of 0.5 µg/ml, and cells were incubated for an additional 2 h. At the end of the incubation period, culture supernatants were collected and the amount of TNF-α present was determined using an ELISA assay as described below.

### (b) ELISA Assay:

The amount of human TNF-α present was determined by a specific trapping ELISA assay using two anti-TNF-α antibodies (2TNF-H22 and 2TNF-H34) described in Reimund, J. M., et al. *GUT.* Vol. **39**(5), 684-689 (1996).

Polystyrene 96-well plates were coated with 50 µl per well of antibody 2TNF-H22 in PBS (10 µg/ml) and incubated in a humidified chamber at 4 °C overnight. The plates were washed with PBS and then blocked with 5% nonfat-dry milk in PBS for 1 hour at room temperature and washed with 0.1% BSA (bovine serum albumin) in PBS.

TNF standards were prepared from a stock solution of human recombinant TNF-α (R&D Systems, Minneapolis, MN). The concentration of the standards in the assay began at 10 ng/ml followed by 6 half log serial dilution's.

25 µl aliquots of the above culture supernatants or TNF standards or only medium (control) were mixed with 25 µl aliquots of biotinylated monoclonal antibody 2TNF-H34 (2 µg/ml in PBS containing 0.1% BSA) and then added to each well. The samples were incubated for 2 h at room temperature with gentle shaking and then washed 3 times with 0.1% BSA in PBS. 50 µl of peroxidase-streptavidin (Zymed, S. San Francisco, CA) solution containing 0.416 µg/ml of peroxidase-streptavidin and 0.1% BSA in PBS was added to each well. The samples were incubated for an additional 1 h at room temperature and then washed 4 times with 0.1% BSA in PBS. 50 µl of O-phenylenediamine solution (1µg/ml O-phenylene-diamine and 0.03 % hydrogen peroxide in 0.2M citrate buffer pH 4.5) was added to each well and the samples were incubated in the dark for 30 min., at room temperature. Optical density of the sample and the reference were read at 450 nm and 650 nm, respectively. TNF-α levels were determined from a graph relating the optical density at 450 nm to the concentration used.

The IC₅₀ value was defined as the concentration of the test compound corresponding to half-maximal reduction in 450 nm absorbance. Compounds of the invention were active in this assay. The activity of selected compounds is shown below.

| CPD # | IC₅₀, µM | CPD # | IC₅₀, µM |
|---|---|---|---|
| 1 | 1.77 | 21 | 0.61 |
| 2 | 6.30 | 27 | 0.83 |
| 4 | 1.26 | 33 | 0.14 |
| 6 | 1.04 | 38 | 0.69 |
| 10 | 1.62 | 43 | 0.17 |
| 13 | 0.77 | 50 | 0.51 |
| 19 | 0.17 | | |

### Example 29

### Inhibition of LPS-Induced TNF-α Production In Rats....In Vivo Assay

The ability of the compounds of this invention to inhibit the TNF-α release, *in vivo*, was determined using a minor modification of the methods described in described in Zanetti, G.; Heumann, D., *et. al.,* "Cytokine production after intravenous or peritoneal Gram-negative bacterial challenge in mice," *J. Immunol.,* **148**, 1890, (1992) and Sekut, L., Menius, J.A., *et. al.,* "Evaluation of the significance of elevated levels of systemic and localized tumor necrosis factor in different animal models of inflammation," *J. Lab. Clin. Med*., **124**, 813, (1994).

Female Sprague-Dawley rats weighing 110-140 grams (Charles River, Hollister, CA) were acclimated for one week. Groups containing 8 mice each were dosed orally either with the test compounds dissolved in an aqueous vehicle containing 0.9% sodium chloride, 0.5% sodium carboxymethyl-cellulose, 0.4% polysorbate 80, 0.9% benzyl alcohol (CMC vehicle) or only vehicle (control group). After 30 min., the mice were injected intraperitoneally with 50 µg/kg of LPS (Sigma, St. Louis, MO). After 1.5 h, the mice were sacrificed by CO₂ inhalation and blood was harvested by cardiocentesis. Blood was clarified by centrifugation at 15,600 X g for 5 min., and sera were transferred to clean tubes and frozen at -20°C until analyzed for TNF-α by ELISA assay (Biosource International, Camarillo, CA) following the manufacturer's protocol.

The TNF-α inhibitory activity of selected compounds of the invention, i.e., the measure of the TNF-α content in the test group relative to the vehicle treated group (control group) at 30 mg was:

| CPD # | % Inhibition | CPD # | % Inhibition |
|---|---|---|---|
| 3 | 96 | 19 | 76 |
| 8 | 86 | 34 | 75 |
| 16 | 86 | | |

### Example 30

### Adjuvant Arthritis Assay In Rats.....In Vivo assay

The Anti-inflammatory activity of the compounds of this invention was determined utilizing adjuvant induced arthritis in rats. Briefly, Female Sprague Dawley rats, weighing 120-155 g (Charles River, Hollister, CA) were acclimated in-house for approximately 1 week prior to use. On day 1, the animals were injected intradermally in the 1/4 proximal portion of the tail with 0.1 ml of a mineral oil (Sigma, St. Louis, MO) suspension of heat killed and dried *Mycobacterium Butyricum* (Difco, Bacto., Des., Lot 115979JA/EXP9/99) at a concentration of 1mg/0.1ml.

On day 7, the test compounds were administered in CMC vehicle through to day 18. On day 18, following the administration of the compound, animals were weighed. Clinical scores were obtained to evaluate the intensity of edema in the four paws and tail. A score of 0 to 4 was assigned to each paw and 0 to 3 to the tail such that the maximum score was 19. Polyarthritic animals were scored 0 when no inflammatory signs ( swelling and redness ) were observed in any of the small joints (intraphalangeal, metacarpophalangeal, metatarsophalangeal) or large joints (wrist/ carpus, ankle/tarsus). Animals were scored 1 when slight inflammation was observed, 2 moderate edema, 3 severe edema, and 4 when very severe edema was present. The tail was scored 0 when no signs of edema or necrotic tissue was observed, 1 when inocula injection sites and immediate surrounding tissue exhibit slight edema, 2 when approximately 1/4 of the tail was either inflamed or exhibiting necrotic tissue, and 3 when over 1/4 of the tail exhibited severe necroses or edema. Following clinical scores, the hind paws were transected at the distal tibia, just proximal to the tarsal joint. The left and right hind paws were weighed individually, and recorded.

The compounds of the present invention exhibit anti-inflammatory activity when tested in this assay.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive.

## Claims

1. A compound selected from the group of compounds represented by Formula (I): 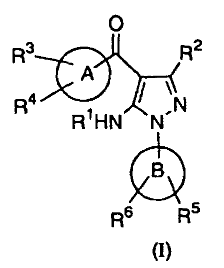 wherein:
R¹ is hydrogen, acyl or -P(O)(OH)₂;
R² is hydrogen;
A is an aryl, heteroaryl or a heterocyclyl ring optionally fused to a phenyl ring provided that the heterocyclyl ring is attached to the carbonyl group via a carbon ring atom;
B is an aryl or heteroaryl ring;
R³ is selected from the group consisting of:
(a) amino, alkylamino or dialkylamino;
(b) acylamino;
(c) optionally substituted heterocyclyl;
(d) optionally substituted aryl or heteroaryl;
(e) heteroalkyl;
(f) heteroalkenyl;
(g) heteroalkynyl;
(h) heteroalkoxy;
(i) heteroalkylamino;
(j) optionally substituted heterocyclylalkyl;
(k) optionally substituted heterocyclylalkenyl;
(l) optionally substituted heterocyclylalkynyl;
(m) optionally substituted heterocyclylalkoxy or heterocyclyloxy;
(n) optionally substituted heterocyclylalkylamino;
(o) optionally substituted heterocyclylalkylcarbonyl;
(p) heteroalkylcarbonyl;
(q) -NHSO₂R⁶ where R⁶ is alkyl, heteroalkyl or optionally substituted heterocyclylalkyl;
(r) -NHSO₂NR⁷R⁸ where R⁷ and R⁸ are, independently of each other, hydrogen, alkyl or heteroalkyl;
(s) -Y-(alkylene)-R⁹ where:
Y is a single bond, -O-, -NH- or -S(O)ₙ- (where n is an integer from 0 to 2); and
R⁹ is cyano, optionally substituted heteroaryl, -COOH, - COR¹⁰, -COOR¹¹, -CONR¹²R¹³, -SO₂R¹⁴, -SO₂NR¹⁵R¹⁶, -NHSO₂R¹⁷ or -NHSO₂NR¹⁸R¹⁹, where R¹⁰ is alkyl or optionally substituted heterocycle, R¹¹ is alkyl, and R¹², R¹³, R¹⁴, R¹⁵, R¹⁶, R¹⁷, R¹⁸ and R¹⁹ are, independently of each other, hydrogen, alkyl or heteroalkyl;
(t) -C(=NR²⁰)(NR²¹R²²) where R²⁰, R²¹ and R²² independently represent hydrogen, alkyl or hydroxy, or R²⁰ and R²¹ together are -(CH₂)ₙ- where n is 2 or 3 and R²² is hydrogen or alkyl;
(u) -NHC(X)NR²³R²⁴ where X is -O- or -S-, and R²³ and R²⁴ are, independently of each other, hydrogen, alkyl or heteroalkyl;
(v) -CONR²⁵R²⁶ where R²⁵ and R²⁶ independently represent hydrogen, alkyl, heteroalkyl or optionally substituted heterocyclylalkyl, or R²⁵ and R²⁶ together with the nitrogen to which they are attached form an optionally substituted heterocyclyl ring;
(w) -S(O)ₙR²⁷ where n is an integer from 0 to 2, and R²⁷ is alkyl, heteroalkyl, optionally substituted heterocyclylalkyl or -NR²⁸R²⁹ where R²⁸ and R²⁹ are, independently of each other, hydrogen, alkyl or heteroalkyl;
(x) cycloalkylalkyl, cycloalkylalkynyl and cycloalkylalkynyl, all optionally substituted with alkyl, halo, hydroxy or amino;
(y) arylaminoalkylene or heteroarylaminoalkylene;
(z) Z-alkylene-NR³⁰R³¹ or Z-alkylene-OR³² where Z is -NH-, -N(lower alkyl)- or -O-, and R³⁰, R³¹and R³² are independently of each other, hydrogen, alkyl or heteroalkyl;
(aa) -OC(O)-alkylene-CO₂H or -OC(O)-NR'R" (where R' and R" are independently hydrogen or alkyl); and
(bb) heteroarylalkenylene or heteroarylalkynylene;
R⁴ is selected from the group consisting of:
(a) hydrogen;
(b) halo;
(c) alkyl;
(d) alkoxy; and
(e) hydroxy;
R⁵ is selected from the group consisting of :
(a) hydrogen;
(b) halo;
(c) alkyl;
(d) haloalkyl;
(e) thioalkyl;
(f) hydroxy;
(g) amino;
(h) alkylamino;
(i) dialkylamino;
(j) heteroalkyl;
(k) optionally substituted heterocycle;
(l) optionally substituted heterocyclylalkyl;
(m) optionally substituted heterocyclylalkoxy;
(n) alkylsulfonyl;
(o) aminosulfonyl, mono-alkylaminosulfonyl or di-alkylaminosulfonyl;
(p) heteroalkoxy; and
(q) carboxy;
R⁶ is selected from the group consisting of:
(a) hydrogen;
(b) halo;
(c) alkyl; and
(d) alkoxy;
whereas:
alkyl means a linear saturated monovalent hydrocarbon radical of one to six carbon atoms or a branched saturated monovalent hydrocarbon radical of three to six carbon atoms;
alkylene means a linear saturated divalent hydrocarbon radical of one to six carbon atoms or a branched saturated divalent hydrocarbon radical of three to six carbon atoms;
alkenyl means a linear monovalent hydrocarbon radical of two to six carbon atoms or a branched monovalent hydrocarbon radical of three to six carbon atoms, containing at least one double bond;
alkenylene means a linear divalent hydrocarbon radical of two to six carbon atoms or a branched divalent hydrocarbon radical of three to six carbon atoms, containing at least one double bond;
alkynyl means a linear monovalent hydrocarbon radical of two to six carbon atoms or a branched divalent hydrocarbon radical of three to six carbon atoms, containing at least one triple bond;
alkynylene means a linear divalent hydrocarbon radical of two to six carbon atoms or a branched monovalent hydrocarbon radical of three to six carbon atoms, containing at least one triple bond;
alkoxy means a radical -OR where R is alkyl as defined above;
acyl means a radical -C(O)R where R is alkyl or haloalkyl;
acylamino means a radical -NRC(O)R' where R is hydrogen or alkyl, and R' is alkyl, heteroalkyl or optionally substituted heterocyclylalkyl;
haloalkyl means alkyl substituted with one or more same or different halo atoms;
aryl means a monovalent monocyclic or bicyclic aromatic hydrocarbon radical of 6 to 10 ring atoms whereby the aryl ring may optionally be fused to a 5-, 6- or 7-membered monocyclic saturated ring optionally containing 1 or 2 heteroatoms independently selected from oxygen, nitrogen or sulfur, the remaining ring atoms being C where one or two C atoms are optionally replaced by a carbonyl group;
heteroaryl means a monovalent monocyclic or bicyclic aromatic radical of 5 to 10 ring atoms containing one, two, or three ring heteroatoms selected from N, O, or S, the remaining ring atoms being C whereby the term heteroaryl also include those radicals where a heteroatom within the ring has been oxidized or quaternized;
heterocycle or heterocyclyl means a cyclic nonaromatic radical of 3 to 8 ring atoms in which one or two ring atoms are heteroatoms selected from N, O, or S(O)ₙ (where n is an integer from 0 to 2), the remaining ring atoms being C where one or two C atoms are optionally replaced by a carbonyl group whereby both terms also includes those radicals where a ring nitrogen atom has been oxidized or quaternized;
optionally substituted aryl, heteroaryl or heterocyclyl means an aryl, heteroaryl or heterocyclyl ring as defined above, which is optionally substituted independently with one or two substituents selected from alkyl, phenyl, benzyl, haloalkyl, heteroalkyl, halo, cyano, acyl, -OR (where R is hydrogen or alkyl), -NRR' (where R and R' are independently selected from hydrogen, alkyl or acyl), -NHCOR (where R is alkyl), -NRS(O)ₙR' (where R is hydrogen or alkyl, n is an integer from 0 to 2 and R' is hydrogen, alkyl or heteroalkyl), -NRS(O)ₙNR'R" (where R is hydrogen or alkyl, n is an integer from 0 to 2 and R' and R" are independently hydrogen, alkyl or heteroalkyl), -S(O)ₙR (where n is an integer from 0 to 2 and R is hydrogen, alkyl or heteroalkyl), -S(O)ₙNRR' (where n is an integer from 0 to 2 and R and R' are independently hydrogen, alkyl or heteroalkyl), -COOR, -(alkylene)COOR (where R is hydrogen or alkyl), -CONR'R" or -(alkylene)CONR'R" (where R' and R" are independently hydrogen or alkyl);
heteroalkyl means an alkyl radical as defined above, carrying one, two or three substituents selected from -NR^{a}R^{b}, -OR^{c} wherein R^{a}, R^{b} and R^{c} are independently of each other hydrogen, alkyl or acyl, or R^{a} and R^{b} together form heterocycloamino group;
heteroalkenyl means an alkenyl radical as defined above, carrying one or two substituents selected from -NR^{a}R^{b}, -OR^{c} or -S(O)ₙR^{d} wherein R^{a}, R^{b} and R^{c} are independently of each other hydrogen or alkyl, and R^{d} is alkyl or -NRR' (where R and R' are independently of each other hydrogen or alkyl;
heteroalkynyl means an alkynyl radical as defined above, carrying one or two substituents selected -NR^{a}R^{b}, -OR^{c}, -S(O)ₙR^{d} or -S(O)ₙNRR' (where R and R' are independently of each other hydrogen or alkyl) wherein R^{a}, R^{b} and R^{c} are independently of each other hydrogen or alkyl, and R^{d} is alkyl and n is an integer from zero to two;
heteroalkoxy means a radical -OR where R is heteroalkyl group as defined above;
heteroalkylamino means a radical -NR^{a}R^{b} where R^{a} is hydrogen or alkyl, and R^{b} is a heteroalkyl group as defined above;
optionally substituted heterocyclylalkyl means a radical -R^{a}R^{b} where R^{a} is an alkylene group, and R^{b} is an optionally substituted heterocyclyl group as defined above;
optionally substituted heterocyclylalkenyl means a radical -R^{a}R^{b} where R^{a} is an alkenylene group and R^{b} is an optionally substituted heterocyclyl group as defined above;
optionally substituted heterocyclylalkynyl means a radical -R^{a}R^{b} where R^{a} is an alkynyl group and R^{b} is an optionally substituted heterocyclyl group as defined above;
optionally substituted heterocyclylalkoxy means a radical -OR where R is an optionally substituted heterocyclylalkyl group as defined above;
optionally substituted heterocyclylalkylamino means a radical -NR^{a}R^{b} where R^{a} is hydrogen or alkyl and R^{b} is an optionally substituted heterocyclylalkyl group as defined above;
and individual isomers, mixtures of isomers and pharmaceutically acceptable salts thereof;.

2. A compound according to claim 1, wherein:
R¹ is hydrogen or acyl;
A is an aryl or heteroaryl ring;

3. A compound according to claim1, wherein:
R¹ is hydrogen, acyl or -P(O)(OH)₂;
A is an aryl, heteroaryl or a heterocyclyl ring optionally fused to a phenyl ring provided that the heterocyclyl ring is attached to the carbonyl group via a carbon ring atom;
B is an aryl or heteroaryl ring;
R³ is selected from the group consisting of:
(a) amino;
(b) acylamino;
(c) optionally substituted heterocycle;
(d) heteroaryl optionally substituted with a substituent selected from halo, alkyl or alkoxy;
(e) heteroalkyl;
(f) heteroalkenyl;
(g) heteroalkynyl;
(h) heteroalkoxy;
(i) heteroalkylamino;
(j) optionally substituted heterocyclylalkyl;
(k) optionally substituted heterocyclylalkenyl;
(l) optionally substituted heterocyclylalkynyl;
(m) optionally substituted heterocyclylalkoxy;
(n) optionally substituted heterocyclylalkylamino;
(o) optionally substituted heterocyclylalkylcarbonyl;
(p) heteroalkylcarbonyl;
(q) -NHSO₂R⁶ where R⁶ is alkyl, heteroalkyl or optionally substituted heterocyclylalkyl;
(r) -NHSO₂NR⁷R⁸ where R⁷ and R⁸ are, independently of each other, hydrogen, alkyl or heteroalkyl;
(s) -Y-(alkylene)-R⁹ where:
Y is a single bond, -O-, -NH- or -S(O)ₙ- (where n is an integer from 0 to 2); and
R⁹ is cyano, heteroaryl, -COOH, -COR¹⁰, -COOR¹¹ -CONR¹²R¹³, -SO₂R¹⁴, -SO₂NR¹⁵R¹⁶, -NHSO₂R¹⁷ or -NHSO₂NR¹⁸R¹⁹ where R¹⁰ is alkyl or optionally substituted heterocycle, R¹¹ is alkyl, and R¹², R¹³, R¹⁴, R¹⁵, R¹⁶, R¹⁷, R¹⁸ and R¹⁹ are, independently of each other, hydrogen, alkyl or heteroalkyl;
(t) -C(=NR²⁰)(NR²¹R²²) where R²⁰, R²¹ and R²² independently represent hydrogen, alkyl or hydroxy, or R²⁰ and R²¹ together are -(CH₂)ₙ- where n is 2 or 3 and R²² is hydrogen or alkyl;
(u) -NHC(X)NR²³R²⁴ where X is -O- or -S-, and R²³ and R²⁴ are, independently of each other, hydrogen, alkyl or heteroalkyl;
(v) -CONR²⁵R²⁶ where R²⁵ and R²⁶ independently represent hydrogen, alkyl, heteroalkyl or optionally substituted heterocyclylalkyl, or R²⁵ and R²⁶ together with the nitrogen to which they are attached form an optionally substituted heterocyclyl ring;
(w) -S(O)ₙR²⁷ where n is an integer from 0 to 2, and R²⁷ is alkyl, heteroalkyl, optionally substituted heterocyclylalkyl or
-NR²⁸R²⁹ where R²⁸ and R²⁹ are, independently of each other, hydrogen, alkyl or heteroalkyl;
R⁴ is selected from the group consisting of:
(a) hydrogen;
(b) halo;
(c) alkyl; and
(d) alkoxy;
R⁵ is selected from the group consisting of :
(a) hydrogen;
(b) halo;
(c) alkyl;
(d) haloalkyl;
(e) thioalkyl;
(f) hydroxy;
(g) amino;
(h) alkylamino;
(i) dialkylamino;
(j) heteroalkyl;
(k) optionally substituted heterocycle;
(l) optionally substituted heterocyclylalkyl; and
(m) optionally substituted heterocyclylalkoxy;
R⁶ is selected from a group consisting of:
(a) hydrogen;
(b) halo;
(c) alkyl; and
(d) alkoxy.

4. The compound of claim 1 or claim 2, wherein R³ is:
(a) optionally substituted heterocyclyl;
(b) aryl or heteroaryl both optionally substituted with a substituent selected from halo, alkyl, amino, alkoxy, carboxy, lower alkoxy carbonyl, SO₂R' (where R' is alkyl) or SO₂NHR'R" (where R' and R" are independently hydrogen or alkyl);
(c) heteroalkyl;
(d) heteroalkenyl;
(e) heteroalkylamino;
(f) heteroalkoxy;
(g) optionally substituted heterocyclylalkyl or heterocyclyloxy;
(h) optionally substituted heterocyclylalkenyl;
(i) optionally substituted heterocyclylalkynyl;
(j) optionally substituted heterocyclylalkoxy;
(k) optionally substituted heterocyclylalkylamino;
(l) optionally substituted heterocyclylalkylcarbonyl;
(s) -Y-(alkylene)-R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl;
(x) cycloalkylalkyl, cycloalkylalkynyl and cycloalkylalkynyl, all optionally substituted with alkyl, halo, hydroxy or amino;
(y) arylaminoalkylene or heteroarylaminoalkylene; or
(z) Z-alkylene-NR³⁰R³¹ where Z is -NH-, -N(alkyl)- or -O- , and R³⁰ and R³¹ are independently of each other, hydrogen, alkyl or heteroalkyl.

5. The compound of any one of claim 1-4, wherein R¹ is hydrogen and B is phenyl.

6. The compound of claim 1, wherein A is phenyl.

7. The compound of claim 1, wherein R⁴ is hydrogen and R⁵ is halo or alkyl.

8. The compound of claim 1 wherein R⁵ is chloro, fluoro or methyl and R⁶ is hydrogen, chloro, fluoro, methyl or methoxy.

9. The compound of claim 5, wherein R³ is optionally substituted heteroaryl.

10. The compound of claim 9, wherein R³ is pyridin-2-yl, pyridin-3-yl, pyridin-4-yl, N-oxidopyridin-2-yl, N-oxidopyridin-3-yl, N-oxidopyridin-4-yl or pyridon-2-yl, all optionally substituted.

11. The compound of claim 9 or claim 10, wherein R³ is at the 3-position.

12. The compound of claim 9, wherein R⁵ is 4-F or 2-Me and R⁶ is hydrogen.

13. The compound of claim 1, wherein R³ is optionally substituted phenyl.

14. The compound of claim 13, wherein R³ is 3-sulfamoylphenyl, 3-methylsulfonylphenyl, 3-carboxyphenyl or 3-ethoxycarbonylphenyl.

15. The compound of claim 13 , wherein R³ is at the 3-position.

16. The compound of any one of claim 13, wherein R⁵ is 4-F and R⁶ is hydrogen.

17. The compound of claim 1, wherein R³ is:
(a) heteroalkyl;
(b) heteroalkoxy;
(c) heteroalkylamino;
(d) optionally substituted heterocyclylalkyl;
(e) optionally substituted heterocyclylalkoxy;
(f) optionally substituted heterocyclylalkylamino;
(g) -Y-(alkylene)-R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl; or
(h) Z-alkylene-NR³⁰R³¹ where Z is -NH-, -N(alkyl)- or -O-, and R³⁰ and R³¹ are independently of each other, hydrogen, alkyl or heteroalkyl.

18. The compound of claim 17, wherein R³ is heteroalkyl.

19. The compound of claim 18, wherein R³ is at the 3-position and is selected from the group consisting of 2-dimethylaminoethyl, 3-dimethylaminopropyl, 4-dimethylaminobutyl, 2-dimethylaminoethylamino, 3-dimethylaminopropylamino, hydroxymethyl, 1,2-dihydroxyethyl, 3-hydroxy-3-methyl-1-butyl or 3-hydroxybutyl.

20. The compound of claim 18 or claim 19, wherein R⁵ is 2-F and R⁶ is 4-F.

21. The compound of claim 18 or claim 19, wherein R⁵ is 4-F and R⁶ is hydrogen.

22. The compound of claim 18 or claim 19, wherein R⁵ is 2-Me and R⁶ is hydrogen.

23. The compound of claim 17, wherein R³ is heteroalkoxy or heteroalkylamino.

24. The compound of claim 23, wherein R³ is at the 3-position and is selected from the group consisting of 3-dimethylaminopropoxy, 2-dimethylaminoethoxy, 2-hydroxyethoxy, 2,3-dihydroxypropoxy, 2-dimethylaminoethylamino and 3-dimethylaminopropylamino.

25. The compound of claim 23 or claim 24, wherein R⁵ is 4-F or 2-Me and R⁶ is hydrogen.

26. The compound of claim 17, wherein R³ is optionally substituted heterocyclylalkyl, optionally substituted heterocyclylalkoxy or optionally substituted heterocyclylalkylamino.

27. The compound of claim 26, wherein R³ is at the 3-position and is selected from the group consisting of 3-(morpholin-4-yl)propoxy, 2-(morpholin-4-yl)ethoxy, 2-(2-oxo-pyrrolidin-1-yl)ethoxy, 3-(morpholin-4-yl)propyl, 2-(morpholin-4-yl)ethyl, 4-(morpholin-4-yl)butyl, 3-(morpholin-4-yl)propylamino, 2-(morpholin-4-yl)ethylamino, 4-hydroxypiperidinylmethyl, 2-(S,S-dioxo-thiamorpholin-4-yl)ethyl, 3-(S,S-dioxo-thiamorpholin-4-yl)propyl and N-methylpiperazinylmethyl.

28. The compound of claim 26 or claim 27, wherein R⁵ is 4-F or 2-Me and R⁶ is hydrogen.

29. The compound of claim 17, wherein R³ is -Y-(alkylene)-R⁹ where Y is a single bond, -O- or -NH- and R⁹ is optionally substituted heteroaryl, -CONR¹²R¹³, SO₂R¹⁴, -SO₂NR¹⁵R¹⁶ -NHSO₂R¹⁷ or -NHSO₂NR¹⁸R¹⁹ where R¹², R¹³, R¹⁴, R¹⁵, R¹⁶ R¹⁷, R¹⁸ and R¹⁹ are independently of each other hydrogen, alkyl or heteroalkyl.

30. The compound of claim 29, wherein Y is a single bond and R⁹ is SO₂R¹⁴ or -SO₂NR¹⁵R¹⁶.

31. The compound of claim 29 or claim 30, wherein R³ is methylsulfonylethyl or sulfamoylethyl.

32. The compound of claim 29, wherein R⁵ is 4-F or 2-Me and R⁶ is hydrogen.

33. A compound as claimed in claim 1 and selected from the group consisting of:
5-amino-1-(4-fluorophenyl)-4-[3-(2-morpholin-4-ylethoxy)benzoyl]pyrazole,
5-amino-1-(2,4-difluorophenyl)-4-[3-(3-morpholin-4-ylpropyl)benzoyl]pyrazole,
5-amino-4-(3-aminobenzoyl)-1-(4-fluorophenyl)pyrazole,
5-amino-1-(4-fluorophenyl)-4-[3-(3-morpholin-4-ylpropyl)benzoyl]pyrazole,
5-amino-4-[3-(2-aminosulfonylethenyl)benzoyl]-1-(4-fluorophenyl)pyrazole,
5-amino-4-(3-acetylaminobenzoyl)-1-phenylpyrazole,
5-amino-4-[3-(2-aminoethyl)benzoyl]-1-(4-fluorophenyl)pyrazole,
5-amino-1-(4-fluorophenyl)-4-[3-(3-morpholin-4-ylpropylamino)benzoyl]pyrazole,
5-amino-4-[3-(2-aminosulfonylethyl)benzoyl]-1-(4-fluorophenyl)pyrazole and
5-amino-1-(4-fluorophenyl)-4-(3-pyridin-3-ylbenzoyl)pyrazole.

34. A compound as claimed in claim 1 and selected from the group consisting of:
5-amino-1-(2-methylphenyl)-4-[3-pyridin-3-yl)benzoyl]pyrazole,
5-amino-1-(2-methylphenyl)-4-[3-(N-oxidopyridin-3-yl)benzoyl]pyrazole,
5-amino-4-[3-(2,3-dihydroxypropoxy)benzoyl]-1-(4-fluorophenyl)pyrazole,
5-amino-4-[3-(1,2-dihydroxyethyl)benzoyl]-1-(4-fluorophenyl)pyrazole,
5-amino-1-(4-fluorophenyl)-4-[3-(sulfamoylbenzoyl]pyrazole,
5-amino-1-(4-fluorophenyl)-4-[3-(3-hydroxy-3-methylbutyl)benzoyl]pyrazole,
5-amino-1-(4-fluorophenyl)-4-[3-(2-(1-hydroxycyclopentyl)ethyl)benzoyl]pyrazole,
5-amino-4-[3-(2-methylsulfonylethyl)benzoyl]-1-(4-fluorophenyl)pyrazole and
5-amino-1-(2,4-difluorophenyl)-4-[3-(2-hydroxyethylsulfonyl)benzoyl]pyrazole.

35. A compound as claimed in claim 34 which is:
5-amino-4-[3-{2(S),3-dihydroxypropoxy}benzoyl]-1-(4-fluorophenyl)pyrazole.

36. A compound as claimed in claim 34 which is:
5-amino-4-[3-{2(R),3-dihydroxypropoxy}benzoyl]-1-(4-fluorophenyl)pyrazole.

37. A process for preparing a compound of Formula (I) selected from compounds of Claim 1, which process comprises:
(i) reacting a 2-keto-3-phenylaminoacrylonitrile of Formula 1: 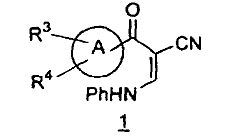 with a hydrazine of Formula 2: 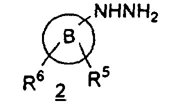 where R³, R⁴ R⁵ and R⁶ are as defined in Claim 1 to provide a compound of Formula (I) where R¹ is hydrogen; or
(ii) reacting a 2-keto-3-phenylaminoacrylonitrile of formula 3: 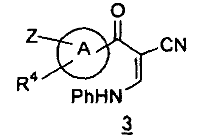 where Z is either hydroxy, nitro or halo group and R⁴ are as defined in Claim 1 with a hydrazine of formula 2 to provide a compound of formula 4: 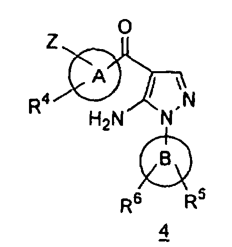 followed by conversion of the Z group to the desired R³ group to provide a compound of Formula (I) where R¹ is hydrogen;
(iii) optionally modifying any of the R¹, R³, R⁴, R⁵ or R⁶ groups;
(iv) optionally converting the compound of Formula (I) prepared in Steps (i), (ii) or (iii) above, to the corresponding acid addition salt by treatment with an acid;
(v) optionally converting the compound of Formula (I) prepared in Steps (i), (ii) or (iii) above, to the corresponding free base by treatment with a base; and
(vi) optionally separating a mixture of stereoisomers of a compound of Formula (I) prepared in Steps (i) - (v) above, to give a single stereoisomer.

38. A process for preparing a compound of Formula (I) selected from compounds of Claim 1, which process comprises reacting a compound of Formula 5: 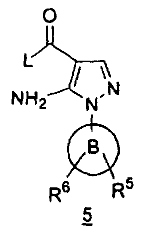 where R⁵ and R⁶ are as in claim 1 and L is a leaving group under organometallic displacement reaction conditions
with an organometallic reagent of formula 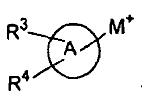 where R³ and R⁴ are as in claim 1 and M is a metallic moiety to provide a compound of Formula (I) where R¹ is hydrogen;
(ii) optionally modifying any of the R¹, R³, R⁴, R⁵ or R⁶ groups;
(iii) optionally converting the compound of Formula (I) prepared in Steps (i) or (ii) above, to the corresponding acid addition salt by treatment with an acid;
(iv) optionally converting the compound of Formula (I) prepared in Steps (i) or (ii) above, to the corresponding free base by treatment with a base; and
optionally separating a mixture of stereoisomers of a compound of Formula (I) prepared in Steps (i) or (iv) above, to give a single stereoisomer.

39. A compound of formula 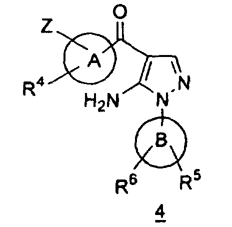 wherein Z is either hydroxy, nitro or halo group and A, B, R⁴, R⁵ and R⁶ are as defined in claim 1; compounds of this formula where A is phenyl, R⁴ is hydrogen, Z is p-chloro, o-chloro or p-nitro, B is phenyl, R⁵ and R⁶ are hydrogen or R⁵ is hydrogen and R⁶ is methyl or R⁵ is methyl and R⁶ is hydrogen being excluded.

40. A compound according to any one of claims 1-36 for use as a medicament.

41. A medicament containing a compound according to any one of claims 1-36 and a pharmaceutically acceptable excipient.

42. A medicament of claim 41 for the control or prevention of inflammatory diseases.

43. A process for the manufacture of medicaments which process comprises bringing a compound according to any one of claims 1-36 together with a pharmaceutically acceptable excipient and bringing the mixture into a galenical administration form.

44. The use of a compound of any one of claims 1-36, for the manufacture of a medicament for the treatment and prophylaxis of inflammatory diseases.

45. A compound according to any one of claims 1-36 whenever prepared according to a process of claim 37 or claim 38.

## Patentansprüche

1. Verbindung, ausgewählt aus Verbindungen der Formel (I) 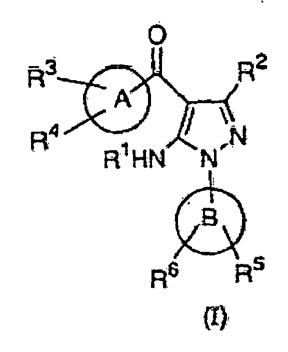 wobei:
R¹ Wasserstoff, Acyl oder -P(O)(OH)₂ ist;
R² Wasserstoff ist;
A ein Aryl-, Heteroaryl- oder Heterocyclylring ist, gegebenenfalls kondensiert an einen Phenylring, mit der Maßgabe, dass der Heterocyclylring an den Carbonylrest über ein Kohlenstoffringatom gebunden ist;
B ein Aryl- oder Heteroarylring ist;
R³ ausgewählt ist aus:
(a) Amino, Alkylamino oder Dialkylamino;
(b) Acylamino;
(c) gegebenenfalls substituiertem Heterocyclyl;
(d) gegebenenfalls substituiertem Aryl oder Heteroaryl;
(e) Heteroalkyl;
(f) Heteroalkenyl;
(g) Heteroalkinyl;
(h) Heteroalkoxy;
(i) Heteroalkylamino;
(j) gegebenenfalls substituiertem Heterocyclylalkyl;
(k) gegebenenfalls substituiertem Heterocyclylalkenyl;
(l) gegebenenfalls substituiertem Heterocyclylalkinyl;
(m) gegebenenfalls subsituiertem Heterocyclylalkoxy oder Heterocyclyloxy;
(n) gegebenenfalls substituiertem Heterocyclylalkylamino;
(o) gegebenenfalls substituiertem Heterocyclylalkylcarbonyl;
(p) Heteroalkylcarbonyl;
(q) -NHSO₂R⁶, wobei R⁶ Alkyl, Heteroalkyl oder gegebenenfalls substituiertes Heterocyclylalkyl ist;
(r) -NHSO₂NR⁷R⁸, wobei R⁷ und R⁸ unabhängig voneinander Wasserstoff, Alkyl oder Heteroalkyl sind;
(s) -Y-(Alkylen)-R⁹, wobei:
Y eine Einfachbindung, -O-, -NH- oder -S(O)ₙ- ist (wobei n ein ganze Zahl von 0 bis 2 ist); und
R⁹ Cyano, gegebenenfalls substituiertes Heteroaryl, -COOH, -COR¹⁰, -COOR¹¹, -CONR¹²R¹³, -SO₂R¹⁴, -SO₂NR¹⁵R¹⁶, -NHSO₂R¹⁷ oder -NHSO₂NR¹⁸R¹⁹ ist, wobei R¹⁰ Alkyl oder gegebenenfalls substituierter Heterocyclus ist, R¹¹ Alkyl ist und R¹², R¹³, R¹⁴, R¹⁵, R¹⁶, R¹⁷, R¹⁸ und R¹⁹ unabhängig voneinander Wasserstoff, Alkyl oder Heteroalkyl sind;
(t) -C(=NR²⁰)(NR²¹R²²), wobei R²⁰, R²¹ und R²² unabhängig Wasserstoff, Alkyl oder Hydroxy darstellen oder R²⁰ und R²¹ zusammen -(CH₂)ₙ- sind, wobei n 2 oder 3 und R²² Wasserstoff oder Alkyl ist;
(u) -NHC(X)NR²³R²⁴, wobei X -O- oder -S- ist und R²³ und R²⁴ unabhängig voneinander Wasserstoff, Alkyl oder Heteroalkyl sind;
(v) -CONR²⁵R²⁶, wobei R²⁵ und R²⁶ unabhängig Wasserstoff, Alkyl, Heteroalkyl oder gegebenenfalls substituiertes Heterocyclylalkyl darstellen oder R²⁵ und R²⁶ zusammen mit dem Stickstoffatom, an das sie gebunden sind, einen gegebenenfalls substituierten Heterocyclylring bilden;
(w) -S(O)ₙR²⁷, wobei n eine ganze Zahl von 0 bis 2 ist und R²⁷ Alkyl, Heteroalkyl, gegebenenfalls substituiertes Heterocyclylalkyl, oder -NR²⁸R²⁹, wobei R²⁸ und R²⁹ unabhängig voneinander Wasserstoff, Alkyl oder Heteroalkyl sind;
(x) Cycloalkylalkyl, Cycloalkylalkinyl und Cycloalkylalkinyl, alle gegebenenfalls substituiert mit Alkyl, Halogen, Hydroxy oder Amino;
(y) Arylaminoalkylen oder Heteroarylaminoalkylen;
(z) Z-Alkylen-NR³⁰R³¹ oder Z-Alkylen-OR³², wobei Z -NH-, -N(Niederalkyl)- oder -O- ist und R³⁰, R³¹ und R³² unabhängig voneinander Wasserstoff, Alkyl oder Heteroalkyl sind;
(aa) -OC(O)-Alkylen-CO₂H oder -OC(O)-NR'R" (wobei R' und R" unabhängig Wasserstoff oder Alkyl sind); und
(bb) Heteroarylalkenylen oder Heteroarylalkinylen;
R⁴ ausgewählt ist aus:
(a) Wasserstoff;
(b) Halogen;
(c) Alkyl;
(d) Alkoxy; und
(e) Hydroxy;
R⁵ ausgewählt ist aus:
(a) Wasserstoff;
(b) Halogen;
(c) Alkyl;
(d) Halogenalkyl;
(e) Thioalkyl;
(f) Hydroxy;
(g) Amino;
(h) Alkylamino;
(i) Dialkylamino;
(j) Heteroalkyl;
(k) gegebenenfalls substituiertem Heterocyclus;
(l) gegebenenfalls substituiertem Heterocyclylalkyl;
(m) gegebenenfalls substituiertem Heterocyclylalkoxy;
(n) Alkylsulfonyl;
(o) Aminosulfonyl, Monoalkylaminosulfonyl oder Dialkylaminosulfonyl;
(p) Heteroalkoxy; und
(q) Carboxy;
R⁶ ausgewählt ist aus:
(a) Wasserstoff;
(b) Halogen;
(c) Alkyl; und
(d) Alkoxy;
wobei:
Alkyl einen linearen gesättigten einwertigen Kohlenwasserstoffrest mit 1 bis 6 Kohlenstoffatomen oder einen verzweigten gesättigten einwertigen Kohlenwasserstoffrest mit 3 bis 6 Kohlenstoffatomen bedeutet;
Alkylen einen linearen gesättigten zweiwertigen Kohlenwasserstoffrest mit 1 bis 6 Kohlenstoffatomen oder einen verzweigten gesättigten zweiwertigen Kohlenwasserstoffrest mit 3 bis 6 Kohlenstoffatomen bedeutet;
Alkenyl einen linearen einwertigen Kohlenwasserstoffrest mit 2 bis 6 Kohlenstoffatomen oder einen verzweigten einwertigen Kohlenwasserstoffrest mit 3 bis 6 Kohlenstoffatomen mit mindestens einer Doppelbindung bedeutet;
Alkenylen einen linearen zweiwertigen Kohlenwasserstoffrest mit 2 bis 6 Kohlenstoffatomen oder einen verzweigten zweiwertigen Kohlenwasserstoffrest mit 3 bis 6 Kohlenstoffatomen mit mindestens einer Doppelbindung bedeutet;
Alkinyl einen linearen einwertigen Kohlenwasserstoffrest mit 2 bis 6 Kohlenstoffatomen oder einen verzweigten zweiwertigen Kohlenwasserstoffrest mit 3 bis 6 Kohlenstoffatomen mit mindestens einer Dreifachbindung bedeutet;
Alkinylen einen linearen zweiwertigen Kohlenwasserstoffrest mit 2 bis 6 Kohlenstoffatomen oder einen verzweigten einwertigen Kohlenwasserstoffrest mit 3 bis 6 Kohlenstoffatomen mit mindestens einer Dreifachbindung bedeutet;
Alkoxy einen Rest -OR bedeutet, wobei R Alkyl wie vorstehend definiert ist;
Acyl einen Rest -C(O)R bedeutet, wobei R Alkyl oder Halogenalkyl ist;
Acylamino einen Rest -NRC(O)R' bedeutet, wobei R Wasserstoff oder Alkyl ist und R' Alkyl, Heteroalkyl oder gegebenenfalls substituiertes Heterocyclylalkyl ist;
Halogenalkyl Alkyl, substituiert mit einem oder mehreren gleichen oder verschiedenen
Halogenatomen bedeutet;
Aryl einen einwertigen monocyclischen oder bicyclischen aromatischen Kohlenwasserstoffrest mit 6 bis 10 Ringatomen bedeutet, wobei der Arylring gegebenenfalls an einen 5-, 6-, oder 7-gliedrigen monocyclischen gesättigten Ring, gegebenenfalls enthaltend 1 oder 2 Heteroatome, unabhängig ausgewählt aus Sauerstoff, Stickstoff oder Schwefel, kondensiert sein kann, wobei die übrigen Ringatome C sind, wobei ein oder zwei C-Atome gegebenenfalls durch eine Carbonylgruppe ersetzt sind;
Heteroaryl einen einwertigen monocyclischen oder bicyclischen aromatischen Rest mit 5 bis 10 Ringatomen, enthaltend 1, 2 oder 3 Ringheteroatome, ausgewählt aus N, O oder S, bedeutet, wobei die übrigen Ringatome C sind, wobei der Begriff Heteroaryl auch jene Reste einschließt, bei denen ein Heteroatom innerhalb des Rings oxidiert oder quartärnisiert worden ist;
Heterocyclus oder Heterocyclyl einen cyclischen nichtaromatischen Rest mit 3 bis 8 Ringatomen bedeutet, wobei 1 oder 2 Ringatome Heteroatome sind, ausgewählt aus N, O oder S(O)ₙ (wobei n eine ganze Zahl von 0 bis 2 ist), wobei die übrigen Ringatome C sind, wobei 1 oder 2 C-Atome gegebenenfalls durch eine Carbonylgruppe ersetzt sind, wobei beide Begriffe auch jene Reste einschließen, bei denen ein Stickstoffatom oxidiert oder quartärnisiert worden ist;
gegebenenfalls substituiertes Aryl, Heteroaryl oder Heterocyclyl einen Aryl-, Heteroaryl-, oder Heterocyclylring wie vorstehend definiert bedeuten, gegebenenfalls unabhängig substituiert mit einem oder zwei Substituenten, ausgewählt aus Alkyl, Phenyl, Benzyl, Halogenalkyl, Heteroalkyl, Halogen, Cyano, Acyl, -OR (wobei R Wasserstoff oder Alkyl ist), -NRR' (wobei R und R' unabhängig ausgewählt sind aus Wasserstoff, Alkyl oder Acyl), -NHCOR (wobei R Alkyl ist), -NRS(O)ₙR' (wobei R Wasserstoff oder Alkyl ist, n eine ganze Zahl von 0 bis 2 ist und R' Wasserstoff, Alkyl oder Heteroalkyl ist), -NRS(O)ₙNR'R" (wobei R Wasserstoff oder Alkyl ist, n eine ganze Zahl von 0 bis 2 ist und R' und R" unabhängig Wasserstoff, Alkyl oder Heteroalkyl sind), -S(O)ₙR (wobei n eine ganze Zahl von 0 bis 2 ist und R Wasserstoff, Alkyl oder Heteroalkyl ist), -S(O)NRR' (wobei n eine ganze Zahl von 0 bis 2 ist und R und R' unabhängig Wasserstoff, Alkyl oder Heteroalkyl sind), -COOR, -(Alkylen)COOR (wobei R Wasserstoff oder Alkyl ist), -CONR'R" oder -(Alkylen)CONR'R" (wobei R' und R" unabhängig Wasserstoff oder Alkyl sind);
Heteroalkyl einen Alkylrest wie vorstehend definiert mit einem, zwei oder drei Substituenten, ausgewählt aus -NR^{a}R^{b}, -OR^{c}, wobei R^{a}, R^{b} und R^{c} unabhängig voneinander Wasserstoff, Alkyl oder Acyl sind oder R^{a} und R^{b} zusammen einen Heterocycloaminorest bilden, bedeutet;
Heteroalkenyl einen Alkenylrest wie vorstehend definiert mit einem oder zwei Substituenten, ausgewählt aus -NR^{a}R^{b}, -OR^{c} oder -S(O)ₙR^{d}, wobei R^{a}, R^{b} und R^{c} unabhängig voneinander Wasserstoff oder Alkyl sind und R^{d} Alkyl oder -NRR' (wobei R und R' unabhängig voneinander Wasserstoff oder Alkyl sind) ist, bedeutet;
Heteroalkinyl einen Alkinylrest wie vorstehend definiert mit einem oder zwei Substituenten, ausgewählt aus -NR^{a}R^{b}, -OR^{c}, -S(O)ₙR^{d} oder -S(O)ₙNRR' (wobei R und R' unabhängig voneinander Wasserstoff oder Alkyl sind) bedeutet, wobei R^{a}, R^{b} und R^{c} unabhängig voneinander Wasserstoff oder Alkyl sind und R^{d} Alkyl ist und n eine ganze Zahl von 0 bis 2 ist;
Heteroalkoxy einen Rest -OR bedeutet, wobei R ein Heteroalkylrest wie vorstehend definiert ist;
Heteroalkylamino einen Rest -NR^{a}R^{b} bedeutet, wobei R^{a} Wasserstoff oder Alkyl ist und R^{b} ein Heteroalkylrest wie vorstehend definiert ist;
gegebenenfalls substituiertes Heterocyclylalkyl einen Rest -R^{a}R^{b} bedeutet, wobei R^{a} ein Alkylenrest ist und R^{b} ein gegebenenfalls substituierter Heterocyclylrest wie vorstehend definiert ist;
gegebenenfalls substituiertes Heterocyclylalkenyl einen Rest -R^{a}R^{b} bedeutet, wobei R^{a} ein Alkenylenrest ist und R^{b} ein gegebenenfalls substituierter Heterocyclylrest wie vorstehend definiert ist;
gegebenenfalls substituiertes Heterocyclylalkinyl einen Rest -R^{a}R^{b} bedeutet, wobei R^{a} ein Alkinylrest ist und R^{b} ein gegebenenfalls substituierter Heterocyclylrest wie vorstehend definiert ist;
gegebenenfalls substituiertes Heterocyclylalkoxy einen Rest -OR bedeutet, wobei R ein gegebenenfalls substituierter Heterocyclylalkylrest wie vorstehend definiert ist;
gegebenenfalls substituiertes Heterocyclylalkylamino einen Rest -NR^{a}R^{b} bedeutet, wobei R^{a} Wasserstoff oder Alkyl ist und R^{b} ein gegebenenfalls substituierter Heterocyclylalkylrest wie vorstehend definiert ist;
und individuelle Isomere, Gemische von Isomeren und pharmazeutisch verträgliche Salze davon.

2. Verbindung gemäß Anspruch 1, wobei:
R¹ ein Wasserstoff oder Acyl ist;
A ein Aryl- oder Heteroarylring ist.

3. Verbindung gemäß Anspruch 1, wobei:
R¹ Wasserstoff, Acyl oder -P(O)(OH)₂ ist;
A ein Aryl-, Heteroaryl- oder ein Heterocyclylring ist, gegebenenfalls kondensiert an einen Phenylring, mit der Maßgabe, dass der Heterocyclylring an die Carbonylgruppe über ein Kohlenstoffringatom gebunden ist;
B ein Aryl- oder Heteroarylring ist;
R³ ausgewählt ist aus:
(a) Amino;
(b) Acylamino;
(c) gegebenenfalls substituiertem Heterocyclus;
(d) Heteroaryl, gegebenenfalls substituiert mit einem Substituenten, ausgewählt aus Halogen, Alkyl oder Alkoxy;
(e) Heteroalkyl;
(f) Heteroalkenyl;
(g) Heteroalkinyl;
(h) Heteroalkoxy;
(i) Heteroalkylamino;
(j) gegebenenfalls substituiertem Heterocyclylalkyl;
(k) gegebenenfalls substituiertem Heterocyclylalkenyl;
(l) gegebenenfalls substituiertem Heterocyclylalkinyl;
(m) gegebenenfalls substituiertem Heterocyclylalkoxy;
(n) gegebenenfalls substituiertem Heterocyclylalkylamino;
(o) gegebenenfalls substituiertem Heterocyclylalkylcarbonyl;
(p) Heteroalkylcarbonyl;
(q) -NHSO₂R⁶, wobei R⁶ Alkyl, Heteroalkyl oder gegebenenfalls substituiertes Heterocyclylalkyl ist;
(r) -NHSO₂NR⁷R⁸, wobei R⁷ und R⁸ unabhängig voneinander Wasserstoff, Alkyl oder Heteroalkyl sind;
(s) -Y-(Alkylen)-R⁹, wobei:
Y eine Einfachbindung, -O-, -NH- oder -S(O)ₙ- ist (wobei n ein ganze Zahl von 0 bis 2 ist); und
R⁹ Cyano, Heteroaryl, -COOH, -COR¹⁰, -COOR¹¹, -CONR¹²R¹³, -SO₂R¹⁴, -SO₂NR¹⁵R¹⁶, -NHSO₂R¹⁷ oder -NHSO₂NR¹⁸R¹⁹ ist, wobei R¹⁰ Alkyl oder gegebenenfalls substituierter Heterocyclus ist, R¹¹ Alkyl ist und R¹², R¹³, R¹⁴, R¹⁵, R¹⁶, R¹⁷, R¹⁸ und R¹⁹ unabhängig voneinander Wasserstoff, Alkyl oder Heteroalkyl sind;
(t) -C(=NR²⁰)(NR²¹R²¹), wobei R²⁰, R²¹ und R²² unabhängig Wasserstoff, Alkyl oder Hydroxy darstellen oder R²⁰ und R²¹ zusammen -(CH₂)ₙ- sind, wobei n 2 oder 3 und R²² Wasserstoff oder Alkyl ist;
(u) -NHC(X)NR²³R²⁴, wobei X -O- oder -S- ist und R²³ und R²⁴ unabhängig voneinander Wasserstoff, Alkyl oder Heteroalkyl sind;
(v) -CONR²⁵R²⁶, wobei R²⁵ und R²⁶ unabhängig Wasserstoff, Alkyl, Heteroalkyl oder gegebenenfalls substituiertes Heterocyclylalkyl darstellen, oder R²⁵ und R²⁶ zusammen mit dem Stickstoffatom, an das sie gebunden sind, einen gegebenenfalls substituierten Heterocyclylring bilden;
(w) -S(O)ₙR²⁷, wobei n eine ganze Zahl von 0 bis 2 ist und R²⁷ Alkyl, Heteroalkyl, gegebenenfalls substituiertes Heterocyclylalkyl oder
-NR²⁸R²⁹ ist, wobei R²⁸ und R²⁹ unabhängig voneinander Wasserstoff, Alkyl oder Heteroalkyl sind;
R⁴ ausgewählt ist aus:
(a) Wasserstoff;
(b) Halogen;
(c) Alkyl; und
(d) Alkoxy;
R⁵ ausgewählt ist aus:
(a) Wasserstoff;
(b) Halogen;
(c) Alkyl;
(d) Halogenalkyl;
(e) Thioalkyl;
(f) Hydroxy;
(g) Amino;
(h) Alkylamino;
(i) Dialkylamino;
(j) Heteroalkyl;
(k) gegebenenfalls substituiertem Heterocyclus;
(l) gegebenenfalls substituiertem Heterocyclylalkyl; und
(m) gegebenenfalls substituiertem Heterocyclylalkoxy;
R⁶ ausgewählt ist aus:
(a) Wasserstoff;
(b) Halogen;
(c) Alkyl; und
(d) Alkoxy.

4. Verbindung gemäß Anspruch 1 oder Anspruch 2, wobei R³ ist:
(a) gegebenenfalls substituiertes Heterocyclyl;
(b) Aryl oder Heteroaryl, jeweils gegebenenfalls substituiert mit einem Substituenten, ausgewählt aus Halogen, Alkyl, Amino, Alkoxy, Carboxy, Niederalkoxycarbonyl, SO₂R' (wobei R' Alkyl ist) oder SO₂NHR'R" (wobei R' und R" unabhängig Wasserstoff oder Alkyl sind);
(c) Heteroalkyl;
(d) Heteroalkenyl;
(e) Heteroalkylamino;
(f) Heteroalkoxy;
(g) gegebenenfalls substituiertes Heterocyclylalkyl oder Heterocyclyloxy;
(h) gegebenenfalls substituiertes Heterocyclylalkenyl;
(i) gegebenenfalls substituiertes Heterocyclylalkinyl;
(j) gegebenenfalls substituiertes Heterocyclylalkoxy;
(k) gegebenenfalls substituiertes Heterocyclylalkylamino;
(l) gegebenenfalls substituiertes Heterocyclylalkylcarbonyl;
(s) -Y-(Alkylen)-R⁹, wobei Y eine Einfachbindung, -O- oder -NH- ist und R⁹ gegebenenfalls substituiertes Heteroaryl, -CONR¹²R¹³, -SO₂R¹⁴, -SO₂NR¹⁵R¹⁶, -NHSO₂R¹⁷ oder -NHSO₂NR¹⁸R¹⁹ ist, wobei R¹², R¹³, R¹⁴, R¹⁵, R¹⁶, R¹⁷, R¹⁸ und R¹⁹ unabhängig voneinander Wasserstoff, Alkyl oder Heteroalkyl sind;
(x) Cycloalkylalkyl, Cycloalkylalkinyl und Cycloalkylalkinyl, alle gegebenenfalls substituiert mit Alkyl, Halogen, Hydroxy oder Amino;
(y) Arylaminoalkylen oder Heteroarylaminoalkylen; oder
(z) Z-Alkylen-NR³⁰R³¹, wobei Z -NH-, -N(Alkyl)- oder -O- ist und R³⁰ und R³¹ unabhängig voneinander Wasserstoff, Alkyl oder Heteroalkyl sind.

5. Verbindung gemäß einem der Ansprüche 1 bis 4, wobei R¹ Wasserstoff ist und B Phenyl ist.

6. Verbindung gemäß Anspruch 1, wobei A Phenyl ist.

7. Verbindung gemäß Anspruch 1, wobei R⁴ Wasserstoff ist und R⁵ Halogen oder Alkyl ist.

8. Verbindung gemäß Anspruch 1, wobei R⁵ Chlor, Fluor oder Methyl ist und R⁶ Wasserstoff, Chlor, Fluor, Methyl oder Methoxy ist.

9. Verbindung gemäß Anspruch 5, wobei R³ gegebenenfalls substituiertes Heteroaryl ist.

10. Verbindung gemäß Anspruch 9, wobei R³ Pyridin-2-yl, Pyridin-3-yl, Pyridin-4-yl, N-Oxidopyridin-2-yl, N-Oxidopyridin-3-yl, N-Oxidopyridin-4-yl oder Pyridon-2-yl, alle gegebenenfalls substituiert, ist.

11. Verbindung gemäß Anspruch 9 oder Anspruch 10, wobei R³ sich an der 3-Position befindet.

12. Verbindung gemäß Anspruch 9, wobei R⁵ 4-F oder 2-Me ist und R⁶ Wasserstoff ist.

13. Verbindung gemäß Anspruch 1, wobei R³ gegebenenfalls substituiertes Phenyl ist.

14. Verbindung gemäß Anspruch 13, wobei R³ 3-Sulfamoylphenyl, 3-Methylsulfonylphenyl, 3-Carboxyphenyl oder 3-Ethoxycarbonylphenyl ist.

15. Verbindung gemäß Anspruch 13, wobei R³ sich an der 3-Position befindet.

16. Verbindung nach Anspruch 13, wobei R⁵ 4-F ist und R⁶ Wasserstoff ist,

17. Verbindung gemäß Anspruch 1, wobei R³ ist:
(a) Heteroalkyl;
(b) Heteroalkoxy;
(c) Heteroalkylamino;
(d) gegebenenfalls substituiertes Heterocyclylalkyl;
(e) gegebenenfalls substituiertes Heterocyclylalkoxy;
(f) gegebenenfalls substituiertes Heterocyclylalkylamino;
(g) -Y-(Alkylen)-R⁹, wobei Y eine Einfachbindung, -O- oder -NH- ist und R⁹ gegebenenfalls substituiertes Heteroaryl, -CONR¹²R¹³, -SO₂R¹⁴, -SO₂NR¹⁵R¹⁶, -NHSO₂R¹⁷ oder -NHSO₂NR¹⁸R¹⁹ ist, wobei R¹², R¹³, R¹⁴, R¹⁵, R¹⁶, R¹⁷, R¹⁸ und R¹⁹ unabhängig voneinander Wasserstoff, Alkyl oder Heteroalkyl sind; oder
(h) Z-Alkylen-NR³⁰R³¹, wobei Z -NH-, -N(Alkyl)- oder -O- ist und R³⁰ und R³¹ unabhängig voneinander Wasserstoff, Alkyl oder Heteroalkyl sind.

18. Verbindung gemäß Anspruch 17, wobei R³ Heteroalkyl ist.

19. Verbindung gemäß Anspruch 18, wobei R³ sich an der 3-Position befindet und ausgewählt ist aus 2-Dimethylaminoethyl, 3-Dimethylaminopropyl, 4-Dimethylaminobutyl, 2-Dimethylaminoethylamino, 3-Dimethylaminopropylamino, Hydroxymethyl, 1,2-Dihydroxyethyl, 3-Hydroxy-3-methyl-1-butyl oder 3-Hydroxybutyl.

20. Verbindung gemäß Anspruch 18 oder Anspruch 19, wobei R⁵ 2-F ist und R⁶ 4-F ist.

21. Verbindung gemäß Anspruch 18 oder Anspruch 19, wobei R⁵ 4-F ist und R⁶ Wasserstoff ist.

22. Verbindung gemäß Anspruch 18 oder Anspruch 19, wobei R⁵ 2-Me ist und R⁶ Wasserstoff ist.

23. Verbindung gemäß Anspruch 17, wobei R³ Heteroalkoxy oder Heteroalkylamino ist.

24. Verbindung gemäß Anspruch 23, wobei R³ sich an der 3-Position befindet und ausgewählt ist aus 3-Dimethylaminopropoxy, 2-Dimethylaminoethoxy, 2-Hydroxyethoxy, 2,3-Dihydroxypropoxy, 2-Dimethylaminoethylamino und 3-Dimethylaminopropylamino.

25. Verbindung gemäß Anspruch 23 oder Anspruch 24, wobei R⁵ 4-F oder 2-Me ist und R⁶ Wasserstoff ist.

26. Verbindung gemäß Anspruch 17, wobei R³ gegebenenfalls substituiertes Heterocyclylalkyl, gegebenenfalls substituiertes Heterocyclylalkoxy oder gegebenenfalls substituiertes Heterocyclylalkylamino ist.

27. Verbindung gemäß Anspruch 26, wobei R³ sich an der 3-Position befindet und ausgewählt ist aus 3-(Morpholin-4-yl)propoxy, 2-(Morpholin-4-yl)ethoxy, 2-(2-Oxopyrrolidin-1-yl)ethoxy, 3-(Morpholin-4-yl)propyl, 2-(Morpholin-4-yl)ethyl, 4-(Morpholin-4-yl)butyl, 3-(Morpholin-4-yl)propylamino, 2-(Morpholin-4-yl)ethylamino, 4-Hydroxypiperidinylmethyl, 2-(S,S-Dioxothiamorpholin-4-yl)ethyl, 3-(S,S-Dioxothiamorpholin-4-yl)propyl und N-Methylpiperazinylmethyl.

28. Verbindung gemäß Anspruch 26 oder Anspruch 27, wobei R⁵ 4-F oder 2-Me ist und R⁶ Wasserstoff ist.

29. Verbindung gemäß Anspruch 17, wobei R³ -Y-(Alkylen)-R⁹ ist, wobei Y eine Einfachbindung, -O- oder -NH- ist und R⁹ gegebenenfalls substituiertes Heteroaryl, -CONR¹²R¹³, -SO₂R¹⁴, -SO₂NR¹⁵R¹⁶, -NHSO₂R¹⁷ oder NHSO₂NR¹⁸R¹⁹ ist, wobei R¹², R¹³, R¹⁴, R¹⁵, R¹⁶, R¹⁷, R¹⁸ und R¹⁹ unabhängig voneinander Wasserstoff, Alkyl oder Heteroalkyl sind.

30. Verbindung gemäß Anspruch 29, wobei Y eine Einfachbindung ist und R⁹ SO₂R¹⁴ oder -SO₂NR¹⁵R¹⁶ ist.

31. Verbindung gemäß Anspruch 29 oder Anspruch 30, wobei R³ Methylsulfonylethyl oder Sulfamoylethyl ist.

32. Verbindung gemäß Anspruch 29, wobei R⁵ 4-F oder 2-Me ist und R⁶ Wasserstoff ist.

33. Verbindung gemäß Anspruch 1 und ausgewählt aus:
5-Amino-1-(4-fluorphenyl)-4-[3-(2-morpholin-4-ylethoxy)benzoyl]pyrazol,
5-Amino-1-(2,4-difluorphenyl)-4-[3-(3-morpholin-4-ylpropyl)benzoyl]pyrazol,
5-Amino-4-(3-aminobenzoyl)-1-(4-fluorphenyl)pyrazol,
5-Amino-1-(4-fluorphenyl)-4-[3-(3-morpholin-4-ylpropyl)benzoyl]pyrazol,
5-Amino-4-[3-(2-aminosulfonylethenyl)benzoyl]-1-(4-fluorphenyl)pyrazol,
5-Amino-4-(3-acetylaminobenzoyl)-1-phenylpyrazol,
5-Amino-4-[3-(2-aminoethyl)benzoyl]-1-(4-fluorphenyl)pyrazol,
5-Amino-1-(4-fluorphenyl)-4-[3-(3-morpholin-4-ylpropylamino)benzoylpyrazol,
5-Amino-4-[3-(2-aminosulfonylethyl)benzoyl]-1-(4-fluorphenyl)pyrazol und
5-Amino-1-(4-fluorphenyl)-4-(3-pyridin-3-ylbenzoyl)pyrazol.

34. Verbindung gemäß Anspruch 1 und ausgewählt aus:
5-Amino-1-(2-methylphenyl)-4-[3-(pyridin-3-yl)benzoyl]pyrazol,
5-Amino-1-(2-methylphenyl)-4-[3-(N-oxidopyridin-3-yl)benzoyl]pyrazol,
5-Amino-4-[3-(2,3-dihydroxypropoxy)benzoyl]-1-(4-fluorphenyl)pyrazol,
5-Amino-4-[3-(1,2-dihydroxyethyl)benzoyl]-1-(4-fluorphenyl)pyrazol,
5-Amino-1-(4-fluorphenyl)-4-[3-(sulfamoylbenzoyl)]pyrazol,
5-Amino-1-(4-fluorphenyl)-4-[3-(3-hydroxy-3-methylbutyl)benzoyl]pyrazol,
5-Amino-1-(4-fluorphenyl)-4-[3-(2-(1-hydroxycyclopentyl)ethyl)benzoyl]pyrazol,
5-Amino-4-[3-(2-methylsulfonylethyl)benzoyl]-1-(4-fluorphenyl)pyrazol und
5-Amino-1-(2,4-difluorphenyl)-4-[3-(2-hydroxyethylsulfonyl)benzoyl]pyrazol.

35. Verbindung gemäß Anspruch 34, nämlich:
5-Amino-4-[3-{2(S),3-dihydroxypropoxy}benzoyl]-1-(4-fluorphenyl)pyrazol.

36. Verbindung gemäß Anspruch 34, nämlich:
5-Amino-4-[3-{2(R),3-dihydroxypropoxy}benzoyl]-1-(4-fluorphenyl)pyrazol.

37. Verfahren zur Herstellung einer Verbindung der Formel (I), ausgewählt aus Verbindungen gemäß Anspruch 1, wobei das Verfahren umfasst:
(i) Umsetzen eines 2-Keto-3-phenylaminoacrylnitrils der Formel 1: 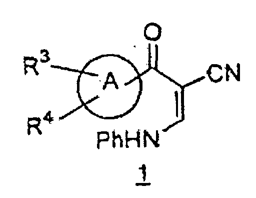 mit einem Hydrazin der Formel 2: 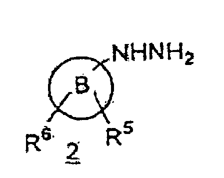 wobei R³, R⁴, R⁵ und R⁶ wie in Anspruch 1 definiert sind, um eine Verbindung der Formel (I) bereitzustellen, wobei R¹ Wasserstoff ist; oder
(ii) Umsetzen eines 2-Keto-3-phenylaminoacrylnitrils der Formel 3: 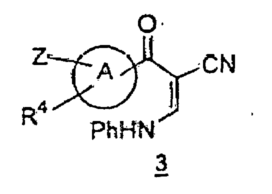 wobei Z entweder eine Hydroxy-, Nitro- oder Halogengruppe ist und R⁴ wie in Anspruch 1 definiert ist, mit einem Hydrazin der Formel 2, um eine Verbindung der Formel 4 bereitzustellen: 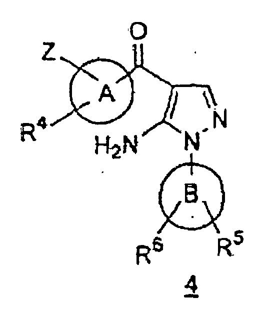 gefolgt von der Umwandlung des Z-Rests in den gewünschten R³-Rest, um eine Verbindung der Formel (I) bereitzustellen, wobei R¹ Wasserstoff ist;
(iii) gegebenenfalls Modifizieren einer der Reste R¹, R³, R⁴, R⁵ oder R⁶;
(iv) gegebenenfalls Umwandeln der Verbindung der Formel (I), hergestellt in den vorstehenden Schritten (i), (ii) oder (iii), in das entsprechende Säureadditionssalz durch Behandlung mit einer Säure;
(v) gegebenenfalls Umwandeln der Verbindung der Formel (I), hergestellt in den vorstehenden Schritten (i), (ii) oder (iii), in die entsprechende freie Base durch Behandlung mit einer Base; und
(vi) gegebenenfalls Trennen eines Gemischs von Stereoisomeren von einer Verbindung der Formel (I), hergestellt in den vorstehenden Schritten (i)-(v), um ein einzelnes Stereoisomer zu ergeben.

38. Verfahren zur Herstellung einer Verbindung der Formel (I), ausgewählt aus Verbindungen gemäß Anspruch 1, wobei das Verfahren (i) das Umsetzen einer Verbindung der Formel 5 umfasst: 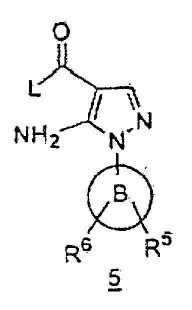 wobei R⁵ und R⁶ die Bedeutung wie in Anspruch 1 haben und L unter metallorganischen Verdrängungsreaktionsbedingungen eine Abgangsgruppe ist, mit einem metallorganischen Reagens der Formel 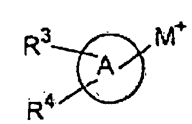 wobei R³ und R⁴ die Bedeutung wie in Anspruch 1 haben und M eine metallische Einheit ist, um eine Verbindung der Formel (I) bereitzustellen, wobei R¹ Wasserstoff ist;
(ii) gegebenenfalls Modifizieren einer der Reste R¹, R³, R⁴, R⁵ oder R⁶;
(iii) gegebenenfalls Umwandeln der Verbindung der Formel (I), hergestellt in den vorstehenden Schritten (i) oder (ii), in das entsprechende Säureadditionssalz durch Behandlung mit einer Säure;
(iv) gegebenenfalls Umwandeln der Verbindung der Formel (I), hergestellt in den vorstehenden Schritten (i) oder (ii), in die entsprechende freie Base durch Behandlung mit einer Base; und
gegebenenfalls Trennen eines Gemischs von Stereoisomeren von einer Verbindung der Formel (I), hergestellt in den vorstehenden Schritten (i) oder (iv), um ein einzelnes Stereoisomer zu ergeben.

39. Verbindung der Formel 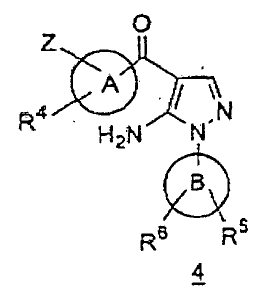 wobei Z entweder eine Hydroxy-, Nitro- oder Halogengruppe ist und A, B, R⁴, R⁵ und R⁶ wie in Anspruch 1 definiert sind; wobei Verbindungen dieser Formel, wobei A Phenyl ist, R⁴ Wasserstoff ist, Z p-Chlor, o-Chlor oder p-Nitro ist, B Phenyl ist, R⁵ und R⁶ Wasserstoff sind oder R⁵ Wasserstoff ist und R⁶ Methyl ist oder R⁵ Methyl ist und R⁶ Wasserstoff ist, ausgeschlossen sind.

40. Verbindung gemäß einem der Ansprüche 1 bis 36 zur Verwendung als ein Medikament.

41. Medikament enthaltend eine Verbindung gemäß einem der Ansprüche 1 bis 36 und einen pharmazeutisch verträglichen Exzipienten.

42. Medikament gemäß Anspruch 41 zur Kontrolle oder Verhinderung von Entzündungskrankheiten.

43. Verfahren zur Herstellung von Medikamenten, wobei das Verfahren das Zusammenbringen einer Verbindung gemäß einem der Ansprüche 1 bis 36 mit einem pharmazeutisch verträglichen Exzipienten und das Überführen des Gemischs in eine galenische Darreichungsform umfasst.

44. Verwendung einer Verbindung gemäß einem der Ansprüche 1 bis 36 zur Herstellung eines Medikaments zur Behandlung und Prophylaxe von Entzündungskrankheiten.

45. Verbindung gemäß einem der Ansprüche 1 bis 36, immer wenn diese nach einem Verfahren gemäß Anspruch 37 oder Anspruch 38 hergestellt worden ist.

## Revendications

1. Composé choisi dans le groupe des composés représentés par la Formule (I) : 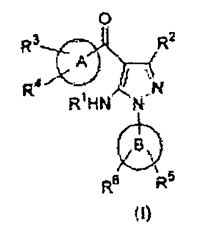 dans laquelle
R¹ représente un atome d'hydrogène, un groupe acyle ou -P(O)(OH)₂ ;
R² représente un atome d'hydrogène ;
A représente un cycle aryle, hétéroaryle ou hétérocyclyle éventuellement fusionné à un cycle phényle à condition que le cycle hétérocyclyle soit fixé au groupe carbonyle par l'intermédiaire d'un atome de carbone du cycle ;
B représente un cycle aryle ou hétéroaryle ;
R³ est choisi dans le groupe constitué par :
(a) un groupe amino, alkylamino ou dialkylamino ;
(b) un groupe acylamino ;
(c) un cycle hétérocyclyle éventuellement substitué ;
(d) un cycle aryle ou hétéroaryle éventuellement substitué ;
(e) un groupe hétéroalkyle ;
(f) un groupe hétéroalcényle ;
(g) un groupe hétéroalcynyle ;
(h) un groupe hétéroalcoxy ;
(i) un groupe hétéroalkylamino ;
(j) un groupe hétérocyclylalkyle éventuellement substitué ;
(k) un groupe hétérocyclylalcényle éventuellement substitué ;
(l) un groupe hétérocyclylalcynyle éventuellement substitué ;
(m) un groupe hétérocyclylalcoxy ou hétérocyclyloxy éventuellement substitué ;
(n) un groupe hétérocyclylalkylamino éventuellement substitué ;
(o) un groupe hétérocyclylalkylcarbonyle éventuellement substitué ;
(p) un groupe hétéroalkylcarbonyle ;
(q) -NHSO₂R⁶ où R⁶ représente un groupe alkyle, hétéroalkyle ou un groupe hétérocyclylalkyle éventuellement substitué ;
(r) -NHSO₂NR⁷R⁸ où R⁷ et R⁸ représentent, indépendamment l'un de l'autre, un atome d'hydrogène, un groupe alkyle ou hétéroalkyle ;
(s) -Y-(alkylène)-R⁹ où :
Y représente une liaison simple, -O-, -NH- ou -S(O)ₙ- (où n est un nombre entier de 0 à 2) ; et
R⁹ représente un groupe cyano, un cycle hétéroaryle éventuellement substitué, -COOH, -COR¹⁰, -COOR¹¹, -CONR¹²R¹³, -SO₂R¹⁴, -SO₂NR¹⁵R¹⁶, -NHSO₂R¹⁷ ou -NHSO₂NR¹⁸R¹⁹, où R¹⁰ représente un groupe alkyle ou un hétérocycle éventuellement substitué, R¹¹ représente un groupe alkyle et R¹², R¹³, R¹⁴, R¹⁵, R¹⁶, R¹⁷, R¹⁸ et R¹⁹ représentent, indépendamment les uns des autres, un atome d'hydrogène, un groupe alkyle ou hétéroalkyle ;
(t) -C(=NR²⁰)(NR²¹R²²) où R²⁰, R²¹ et R²² représentent indépendamment un atome d'hydrogène, un groupe alkyle ou hydroxy ou R²⁰ et R²¹ ensemble représentent -(CH₂)ₙ- où n vaut 2 ou 3 et R²² représente un atome d'hydrogène ou un groupe alkyle ;
(u) -NHC(X)NR²³R²⁴ où X représente -O- ou -S- et R²³ et R²⁴ représentent, indépendamment l'un de l'autre, un atome d'hydrogène, un groupe alkyle ou hétéroalkyle ;
(v) -CONR²⁵R²⁶ où R²⁵ et R²⁶ représentent indépendamment un atome d'hydrogène, un groupe alkyle, hétéroalkyle ou un groupe hétérocyclylalkyle éventuellement substitué, ou R²⁵ et R²⁶ conjointement avec l'atome d'azote auquel ils sont fixés, forment un cycle hétérocyclyle éventuellement substitué ;
(w) -S(O)ₙR²⁷ où n est un nombre entier de 0 à 2 et R²⁷ représente un groupe alkyle, hétéroalkyle, un groupe hétérocyclylalkyle éventuellement substitué ou
-NR²⁸R²⁹ où R²⁸ et R²⁹ représentent, indépendamment l'un de l'autre, un atome d'hydrogène, un groupe alkyle ou hétéroalkyle ;
(x) un groupe cycloalkylalkyle, cycloalkylalcynyle et cycloalkylalcynyle, tous éventuellement substitués par un groupe alkyle, halogéno, hydroxy ou amino ;
(y) un groupe arylaminoalkylène ou hétéroarylaminoalkylène ;
(z) Z-alkylène-NR³⁰R³¹ ou Z-alkylène-OR³² où Z représente -NH-, -N(alkyle inférieur)- ou -O- et R³⁰, R³¹ et R³² représentent, indépendamment les uns des autres, un atome d'hydrogène, un groupe alkyle ou hétéroalkyle ;
(aa) -OC(O)-alkylène-CO₂H ou -OC(O)-NR'R" (où R' et R" représentent indépendamment un atome d'hydrogène ou un groupe alkyle) ; et
(bb) un groupe hétéroarylalcénylène ou hétéroarylalcynylène ;
R⁴ est choisi dans le groupe constitué par :
(a) un atome d'hydrogène ;
(b) un groupe halogéno ;
(c) un groupe alkyle ;
(d) un groupe alcoxy ; et
(e) un groupe hydroxy ;
R⁵ est choisi dans le groupe constitué par :
(a) un atome d'hydrogène ;
(b) un groupe halogéno ;
(c) un groupe alkyle ;
(d) un groupe halogénoalkyle ;
(e) un groupe thioalkyle ;
(f) un groupe hydroxy ;
(g) un groupe amino ;
(h) un groupe alkylamino ;
(i) un groupe dialkylamino ;
(j) un groupe hétéroalkyle ;
(k) un hétérocycle éventuellement substitué ;
(l) un groupe hétérocyclylalkyle éventuellement substitué ;
(m) un groupe hétérocyclylalcoxy éventuellement substitué ;
(n) un groupe alkylsulfonyle ;
(o) un groupe aminosulfonyle, monoalkylamino-sulfonyle ou dialkylaminosulfonyle ;
(p) un groupe hétéroalcoxy ; et
(q) un groupe carboxy ;
R⁶ est choisi dans le groupe constitué par :
(a) un atome d'hydrogène ;
(b) un groupe halogéno ;
(c) un groupe alkyle ; et
(d) un groupe alcoxy ;
tandis que :
alkyle signifie un radical hydrocarbure monovalent saturé linéaire d'un à six atomes de carbone ou un radical hydrocarbure monovalent saturé ramifié de trois à six atomes de carbone ;
alkylène signifie un radical hydrocarbure bivalent saturé linéaire d'un à six atomes de carbone ou un radical hydrocarbure bivalent saturé ramifié de trois à six atomes de carbone ;
alcényle signifie un radical hydrocarbure monovalent linéaire de deux à six atomes de carbone ou un radical hydrocarbure monovalent ramifié de trois à six atomes de carbone, contenant au moins une double liaison ;
alcénylène signifie un radical hydrocarbure bivalent linéaire de deux à six atomes de carbone ou un radical hydrocarbure bivalent ramifié de trois à six atomes de carbone, contenant au moins une double liaison ;
alcynyle signifie un radical hydrocarbure monovalent linéaire de deux à six atomes de carbone ou un radical hydrocarbure bivalent ramifié de trois à six atomes de carbone, contenant au moins une triple liaison ;
alcynylène signifie un radical hydrocarbure bivalent linéaire de deux à six atomes de carbone ou un radical hydrocarbure monovalent ramifié de trois à six atomes de carbone, contenant au moins une triple liaison ;
alcoxy signifie un radical -OR où R représente un groupe alkyle tel que défini ci-dessus ;
acyle signifie un radical -C(O)R où R représente un groupe alkyle ou halogénoalkyle ;
acylamino signifie un radical -NRC(O)R' où R représente un atome d'hydrogène ou un groupe alkyle et R' représente un groupe alkyle, hétéroalkyle ou un groupe hétérocyclylalkyle éventuellement substitué ;
halogénoalkyle signifie un groupe alkyle substitué par un ou plusieurs atomes d'halogène identiques ou différents ;
aryle signifie un radical hydrocarbure aromatique monocyclique ou bicyclique monovalent de 6 à 10 atomes de cycle, moyennant quoi le cycle aryle peut être éventuellement fusionné à un cycle saturé monocyclique à 5, 6 ou 7 chaînons contenant éventuellement 1 ou 2 hétéroatomes choisis indépendamment parmi l'oxygène, l'azote ou le soufre, les atomes restants du cycle étant C où un ou deux atomes C sont éventuellement remplacés par un groupe carbonyle ;
hétéroaryle signifie un radical aromatique monocyclique ou bicyclique monovalent de 5 à 10 atomes de cycle contenant un, deux ou trois hétéroatomes de cycle choisis parmi N, O ou S, les atomes restants du cycle étant C, moyennant quoi le terme hétéroaryle comprend également ces radicaux où un hétéroatome au sein du cycle a été oxydé ou quaternisé ;
hétérocycle ou hétérocyclyle signifie un radical non-aromatique cyclique de 3 à 8 atomes de cycle dans lequel un ou deux atomes de cycle sont des hétéroatomes choisis parmi N, O ou S(O)ₙ (où n est un nombre entier de 0 à 2), les atomes restants du cycle étant C où un ou deux atomes C sont éventuellement remplacés par un groupe carbonyle, moyennant quoi les deux termes comprennent également ces radicaux où un atome d'azote du cycle a été oxydé ou quaternisé ;
aryle, hétéroaryle ou hétérocyclyle éventuellement substitué signifie un cycle aryle, hétéroaryle ou hétérocyclyle tel que défini ci-dessus, qui est éventuellement substitué indépendamment par un ou deux substituants choisis parmi les groupes alkyle, phényle, benzyle, halogénoalkyle, hétéroalkyle, halogéno, cyano, acyle, -OR (où R représente un atome d'hydrogène ou un groupe alkyle), -NRR' (où R et R' sont choisis indépendamment parmi un atome d'hydrogène, un groupe alkyle ou acyle), -NHCOR (où R représente un groupe alkyle), -NRS(O)ₙR' (où R représente un atome d'hydrogène ou un groupe alkyle, n est un nombre entier de 0 à 2 et R' représente un atome d'hydrogène, un groupe alkyle ou hétéroalkyle), -NRS(O)ₙNR'R" (où R représente un atome d'hydrogène ou un groupe alkyle, n est un nombre entier de 0 à 2 et R' et R" représentent indépendamment un atome d'hydrogène, un groupe alkyle ou hétéroalkyle), -S(O)ₙR (où n est un nombre entier de 0 à 2 et R représente un atome d'hydrogène, un groupe alkyle ou hétéroalkyle), -S(O)ₙNRR' (où n est un nombre entier de 0 à 2 et R et R' représentent indépendamment un atome d'hydrogène, un groupe alkyle ou hétéroalkyle), -COOR, -(alkylène)COOR (où R représente un atome d'hydrogène ou un groupe alkyle), -CONR'R" ou -(alkylène)CONR'R" (où R' et R" représentent indépendamment un atome d'hydrogène ou un groupe alkyle) ;
hétéroalkyle signifie un radical alkyle tel que défini ci-dessus, portant un, deux ou trois substituants choisis parmi -NR^{a}R^{b}, -OR^{c} où R^{a}, R^{b} et R^{c} représentent, indépendamment les uns des autres, un atome d'hydrogène, un groupe alkyle ou acyle, ou R^{a} et R^{b} ensemble forment un groupe hétérocycloamino ;
hétéroalcényle signifie un radical alcényle tel que défini ci-dessus, portant un ou deux substituants choisis parmi -NR^{a}R^{b}, -OR^{c} ou -S(O)ₙR^{d} où R^{a}, R^{b} et R^{c} représentent, indépendamment les uns des autres, un atome d'hydrogène ou un groupe alkyle, et R^{d} représente un groupe alkyle ou -NRR' (où R et R' représentent, indépendamment l'un de l'autre, un atome d'hydrogène ou un groupe alkyle) ;
hétéroalcynyle signifie un radical alcynyle tel que défini ci-dessus, portant un ou deux substituants choisis parmi -NR^{a}R^{b}, -OR^{c}, -S(O)ₙR^{d} ou -S(O)ₙNRR' (où R et R' représentent, indépendamment l'un de l'autre, un atome d'hydrogène ou un groupe alkyle) où R^{a}, R^{b} et R^{c} représentent, indépendamment les uns des autres, un atome d'hydrogène ou un groupe alkyle, et R^{d} représente un groupe alkyle et n est un nombre entier de zéro à deux ;
hétéroalcoxy signifie un radical -OR où R représente un groupe hétéroalkyle tel que défini ci-dessus ;
hétéroalkylamino signifie un radical -NR^{a}R^{b} où R^{a} représente un atome d'hydrogène ou un groupe alkyle et R^{b} représente un groupe hétéroalkyle tel que défini ci-dessus ;
hétérocyclylalkyle éventuellement substitué signifie un radical -R^{a}R^{b} où R^{a} représente un groupe alkylène et R^{b} représente un groupe hétérocyclyle éventuellement substitué tel que défini ci-dessus ;
hétérocyclylalcényle éventuellement substitué signifie un radical -R^{a}R^{b} où R^{a} représente un groupe alcénylène et R^{b} représente un groupe hétérocyclyle éventuellement substitué tel que défini ci-dessus ;
hétérocyclylalcynyle éventuellement substitué signifie un radical -R^{a}R^{b} où R^{a} représente un groupe alcynyle et R^{b} représente un groupe hétérocyclyle éventuellement substitué tel que défini ci-dessus ;
hétérocyclylalcoxy éventuellement substitué signifie un radical -OR où R représente un groupe hétérocyclylalkyle éventuellement substitué tel que défini ci-dessus ;
hétérocyclylalkylamino éventuellement substitué signifie un radical -NR^{a}R^{b} où R^{a} représente un atome d'hydrogène ou un groupe alkyle et R^{b} représente un groupe hétérocyclylalkyle éventuellement substitué tel que défini ci-dessus ;
et des isomères individuels, des mélanges d'isomères et des sels pharmaceutiquement acceptables de celui-ci.

2. Composé selon la revendication 1, dans lequel :
R¹ représente un atome d'hydrogène ou un groupe acyle ;
A représente un cycle aryle ou hétéroaryle.

3. Composé selon la revendication 1, dans lequel :
R¹ représente un atome d'hydrogène, un groupe acyle ou -P(O)(OH)₂ ;
A représente un cycle aryle, hétéroaryle ou hétérocyclyle éventuellement fusionné à un cycle phényle à condition que le cycle hétérocyclyle soit fixé au groupe carbonyle par l'intermédiaire d'un atome de carbone du cycle ;
B représente un cycle aryle ou hétéroaryle ;
R³ est choisi dans le groupe constitué par :
(a) un groupe amino ;
(b) un groupe acylamino ;
(c) un hétérocycle éventuellement substitué ;
(d) un cycle hétéroaryle éventuellement substitué par un substituant choisi parmi un groupe halogéno, alkyle ou alcoxy ;
(e) un groupe hétéroalkyle ;
(f) un groupe hétéroalcényle ;
(g) un groupe hétéroalcynyle ;
(h) un groupe hétéroalcoxy ;
(i) un groupe hétéroalkylamino ;
(j) un groupe hétérocyclylalkyle éventuellement substitué ;
(k) un groupe hétérocyclylalcényle éventuellement substitué ;
(l) un groupe hétérocyclylalcynyle éventuellement substitué ;
(m) un groupe hétérocyclylalcoxy éventuellement substitué ;
(n) un groupe hétérocyclylalkylamino éventuellement substitué ;
(o) un groupe hétérocyclylalkylcarbonyle éventuellement substitué ;
(p) un groupe hétéroalkylcarbonyle ;
(q) -NHSO₂R⁶ où R⁶ représente un groupe alkyle, hétéroalkyle ou un groupe hétérocyclylalkyle éventuellement substitué ;
(r) -NHSO₂NR⁷R⁸ où R⁷ et R⁸ représentent, indépendamment l'un de l'autre, un atome d'hydrogène, un groupe alkyle ou hétéroalkyle ;
(s) -Y-(alkylène)-R⁹ où :
Y représente une liaison simple, -O-, -NH- ou -S(O)ₙ- (où n est un nombre entier de 0 à 2) ; et
R⁹ représente un groupe cyano, un cycle hétéroaryle, -COOH, -COR¹⁰, -COOR¹¹, -CONR¹²R¹³, -SO₂R¹⁴, -SO₂NR¹⁵R¹⁶, -NHSO₂R¹⁷ ou -NHSO₂NR¹⁸R¹⁹, où R¹⁰ représente un groupe alkyle ou un hétérocycle éventuellement substitué, R¹¹ représente un groupe alkyle et R¹², R¹³, R¹⁴, R¹⁵, R¹⁶, R¹⁷, R¹⁸ et R¹⁹ représentent, indépendamment les uns des autres, un atome d'hydrogène, un groupe alkyle ou hétéroalkyle ;
(t) -C(=NR²⁰)(NR²¹R²²) où R²⁰, R²¹ et R²² représentent indépendamment un atome d'hydrogène, un groupe alkyle ou hydroxy ou R²⁰ et R²¹ ensemble représentent -(CH₂)ₙ- où n vaut 2 ou 3 et R²² représente un atome d'hydrogène ou un groupe alkyle ;
(u) -NHC(X)NR²³R²⁴ où X représente -O- ou -S- et R²³ et R²⁴ représentent, indépendamment l'un de l'autre, un atome d'hydrogène, un groupe alkyle ou hétéroalkyle ;
(v) -CONR²⁵R²⁶ où R²⁵ et R²⁶ représentent indépendamment un atome d'hydrogène, un groupe alkyle, hétéroalkyle ou un groupe hétérocyclylalkyle éventuellement substitué, ou R²⁵ et R²⁶ conjointement avec l'atome d'azote auquel ils sont fixés, forment un cycle hétérocyclyle éventuellement substitué ;
(w) -S(O)ₙR²⁷ où n est un nombre entier de 0 à 2 et R²⁷ représente un groupe alkyle, hétéroalkyle, un groupe hétérocyclylalkyle éventuellement substitué ou
-NR²⁸R²⁹ où R²⁸ et R²⁹ représentent, indépendamment l'un de l'autre, un atome d'hydrogène, un groupe alkyle ou hétéroalkyle ;
R⁴ est choisi dans le groupe constitué par :
(a) un atome d'hydrogène ;
(b) un groupe halogéno ;
(c) un groupe alkyle ; et
(d) un groupe alcoxy ;
R⁵ est choisi dans le groupe constitué par :
(a) un atome d'hydrogène ;
(b) un groupe halogéno ;
(c) un groupe alkyle ;
(d) un groupe halogénoalkyle ;
(e) un groupe thioalkyle ;
(f) un groupe hydroxy ;
(g) un groupe amino ;
(h) un groupe alkylamino ;
(i) un groupe dialkylamino ;
(j) un groupe hétéroalkyle ;
(k) un hétérocycle éventuellement substitué ;
(l) un groupe hétérocyclylalkyle éventuellement substitué ; et
(m) un groupe hétérocyclylalcoxy éventuellement substitué ;
R⁶ est choisi dans le groupe constitué par :
(a) un atome d'hydrogène ;
(b) un groupe halogéno ;
(c) un groupe alkyle ; et
(d) un groupe alcoxy.

4. Composé selon la revendication 1 ou la revendication 2, dans lequel R³ représente :
(a) un cycle hétérocyclyle éventuellement substitué ;
(b) un cycle aryle ou hétéroaryle, les deux éventuellement substitués par un substituant choisi parmi les groupes halogéno, alkyle, amino, alcoxy, carboxy, alcoxy inférieur-carbonyle, -SO₂R' (où R' représente un groupe alkyle) ou SO₂NHR'R" (où R' et R" représentent indépendamment un atome d'hydrogène ou un groupe alkyle) ;
(c) un groupe hétéroalkyle ;
(d) un groupe hétéroalcényle ;
(e) un groupe hétéroalkylamino ;
(f) un groupe hétéroalcoxy ;
(g) un groupe hétérocyclylalkyle ou hétérocyclyloxy éventuellement substitué ;
(h) un groupe hétérocyclylalcényle éventuellement substitué ;
(i) un groupe hétérocyclylalcynyle éventuellement substitué ;
(j) un groupe hétérocyclylalcoxy éventuellement substitué ;
(k) un groupe hétérocyclylalkylamino éventuellement substitué ;
(l) un groupe hétérocyclylalkylcarbonyle éventuellement substitué ;
(s) -Y-(alkylène)-R⁹ où Y représente une liaison simple, -O- ou -NH- et R⁹ représente un groupe hétéroaryle éventuellement substitué, -CONR¹²R¹³, -SO₂R¹⁴, -SO₂NR¹⁵R¹⁶, -NHSO₂R¹⁷ ou -NHSO₂NR¹⁸R¹⁹, où R¹², R¹³, R¹⁴, R¹⁵, R¹⁶, R¹⁷, R¹⁸ et R¹⁹ représentent, indépendamment les uns des autres, un atome d'hydrogène, un groupe alkyle ou hétéroalkyle ;
(x) un groupe cycloalkylalkyle, cycloalkylalcynyle et cycloalkylalcynyle, tous éventuellement substitués par un groupe alkyle, halogéno, hydroxy ou amino ;
(y) un groupe arylaminoalkylène ou hétéroarylaminoalkylène ; ou
(z) Z-alkylène-NR³⁰R³¹ où Z représente -NH-, -N(alkyl)- ou -O- et R³⁰ et R³¹ représentent, indépendamment l'un de l'autre, un atome d'hydrogène, un groupe alkyle ou hétéroalkyle ;

5. Composé selon l'une quelconque des revendications 1 à 4, dans lequel R¹ représente un atome d'hydrogène et B représente un cycle phényle.

6. Composé selon la revendication 1, dans lequel A représente un cycle phényle.

7. Composé selon la revendication 1, dans lequel R⁴ représente un atome d'hydrogène et R⁵ représente un groupe halogéno ou alkyle.

8. Composé selon la revendication 1, dans lequel R⁵ représente un groupe chloro, fluoro ou méthyle et R⁶ représente un atome d'hydrogène, un groupe chloro, fluoro, méthyle ou méthoxy.

9. Composé selon la revendication 5, dans lequel R³ représente un cycle hétéroaryle éventuellement substitué.

10. Composé selon la revendication 9, dans lequel R³ représente un groupe pyridin-2-yle, pyridin-3-yle, pyridin-4-yle, N-oxydopyridin-2-yle, N-oxydopyridin-3-yle, N-oxydopyridin-4-yle ou pyridon-2-yle, tous éventuellement substitués.

11. Composé selon la revendication 9 ou la revendication 10, dans lequel R³ est en position 3.

12. Composé selon la revendication 9, dans lequel R⁵ représente 4-F ou 2-Me et R⁶ représente un atome d'hydrogène.

13. Composé selon la revendication 1, dans lequel R³ représente un cycle phényle éventuellement substitué.

14. Composé selon la revendication 13, dans lequel R³ représente un cycle 3-sulfamoylphényle, 3-méthylsulfonylphényle, 3-carboxyphényle ou 3-éthoxycarbonylphényle.

15. Composé selon la revendication 13, dans lequel R³ est en position 3.

16. Composé selon la revendication 13, dans lequel R⁵ représente 4-F et R⁶ représente un atome d'hydrogène.

17. Composé selon la revendication 1, dans lequel R³ représente :
(a) un groupe hétéroalkyle ;
(b) un groupe hétéroalcoxy ;
(c) un groupe hétéroalkylamino ;
(d) un groupe hétérocyclylalkyle éventuellement substitué ;
(e) un groupe hétérocyclylalcoxy éventuellement substitué ;
(f) un groupe hétérocyclylalkylamino éventuellement substitué ;
(g) -Y-(alkylène)-R⁹ où Y représente une liaison simple, -O- ou -NH- et R⁹ représente un cycle hétéroaryle éventuellement substitué, -CONR¹²R¹³, -SO₂R¹⁴, -SO₂NR¹⁵R¹⁶, -NHSO₂R¹⁷ ou -NHSO₂NR¹⁸R¹⁹, où R¹², R¹³, R¹⁴, R¹⁵, R¹⁶, R¹⁷, R¹⁸ et R¹⁹ représentent, indépendamment les uns des autres, un atome d'hydrogène, un groupe alkyle ou hétéroalkyle ; ou
(h) Z-alkylène-NR³⁰R³¹ où Z représente -NH-, -N(alkyl)- ou -O- et R³⁰ et R³¹ représentent, indépendamment l'un de l'autre, un atome d'hydrogène, un groupe alkyle ou hétéroalkyle.

18. Composé selon la revendication 17, dans lequel R³ représente un groupe hétéroalkyle.

19. Composé selon la revendication 18, dans lequel R³ est en position 3 et est choisi dans le groupe constitué par les groupes 2-diméthylaminoéthyle, 3-diméthylaminopropyle, 4-diméthylaminobutyle, 2-diméthylaminoéthylamino, 3-diméthylaminopropylamino, hydroxyméthyle, 1,2-dihydroxyéthyle, 3-hydroxy-3-méthyl-1-butyle ou 3-hydroxybutyle.

20. Composé selon la revendication 18 ou la revendication 19, dans lequel R⁵ représente 2-F et R⁶ représente 4-F.

21. Composé selon la revendication 18 ou la revendication 19, dans lequel R⁵ représente 4-F et R⁶ représente un atome d'hydrogène.

22. Composé selon la revendication 18 ou la revendication 19, dans lequel R⁵ représente 2-Me et R⁶ représente un atome d'hydrogène.

23. Composé selon la revendication 17, dans lequel R³ représente un groupe hétéroalcoxy ou hétéroalkylamino.

24. Composé selon la revendication 23, dans lequel R³ est en position 3 et est choisi dans le groupe constitué par les groupes 3-diméthylaminopropoxy, 2-diméthylaminoéthoxy, 2-hydroxyéthoxy, 2,3-dihydroxypropoxy, 2-diméthylaminoéthylamino et 3-diméthylaminopropylamino.

25. Composé selon la revendication 23 ou la revendication 24, dans lequel R³ représente 4-F ou 2-Me et R⁶ représente un atome d'hydrogène.

26. Composé selon la revendication 17, dans lequel R³ représente un groupe hétérocyclylalkyle éventuellement substitué, hétérocyclylalcoxy éventuellement substitué ou hétérocyclylalkylamino éventuellement substitué.

27. Composé selon la revendication 26, dans lequel R⁵ est en position 3 et est choisi dans le groupe constitué par les groupes 3-(morpholin-4-yl)propoxy, 2-(morpholin-4-yl)éthoxy, 2-(2-oxo-pyrrolidin-1-yl)éthoxy, 3-(morpholin-4-yl)propyle, 2-(morpholin-4-yl)éthyle, 4-(morpholin-4-yl)butyle, 3-(morpholin-4-yl)propylamino, 2-(morpholin-4-yl)éthylamino, 4-hydroxypipéridinylméthyle, 2-(S,S-dioxo-thiamorpholin-4-yl)éthyle, 3-(S,S-dioxo-thiamorpholin-4-yl)propyle et N-méthylpipérazinylméthyle.

28. Composé selon la revendication 26 ou la revendication 27, dans lequel R⁵ représente 4-F ou 2-Me et R⁶ représente un atome d'hydrogène.

29. Composé selon la revendication 17, dans lequel R³ représente -Y-(alkylène)-R⁹ où Y représente une liaison simple, -O- ou -NH- et R⁹ représente un cycle hétéroaryle éventuellement substitué, -CONR¹²R¹³, -SO₂R¹⁴, -SO₂NR¹⁵R¹⁶, -NHSO₂R¹⁷ ou -NHSO₂NR¹⁸R¹⁹, où R¹², R¹³, R¹⁴, R¹⁵, R¹⁶, R¹⁷, R¹⁸ et R¹⁹ représentent, indépendamment les uns des autres, un atome d'hydrogène, un groupe alkyle ou hétéroalkyle.

30. Composé selon la revendication 29, dans lequel Y représente une liaison simple et R⁹ représente SO₂R¹⁴ ou -SO₂NR¹⁵R¹⁶.

31. Composé selon la revendication 29 ou la revendication 30, dans lequel R³ représente un groupe méthylsulfonyléthyle ou sulfamoyléthyle.

32. Composé selon la revendication 29, dans lequel R⁵ représente 4-F ou 2-Me et R⁶ représente un atome d'hydrogène.

33. Composé selon la revendication 1 et choisi dans le groupe constitué par :
le 5-amino-1-(4-fluorophényl)-4-[3-(2-morpholin-4-yléthoxy)benzoyl]pyrazole,
le 5-amino-1-(2,4-difluorophényl)-4-[3-(3morpholin-4-ylpropyl)benzoyl]pyrazole,
le 5-amino-4-(3-aminobenzoyl)-1-(4-fluorophényl)pyrazole,
le 5-amino-1-(4-fluorophényl)-4-[3-(3-morpholin-4-ylpropyl)benzoyl]pyrazole,
le 5-amino-4-[3-(2-aminosulfonyléthényl)benzoyl]1-(4-fluorophényl)pyrazole,
le 5-amino-4-(3-acétylaminobenzoyl)-1-phényl-pyrazole,
le 5-amino-4-[3-(2-aminoéthyl)benzoyl]-1-(4-fluorophényl)pyrazole,
le 5-amino-1-(4-fluorophényl)-4-[3-(3-morpholin-4-ylpropylamino)benzoyl]pyrazole,
le 5-amino-4-[3-(2-aminosulfonyléthyl)benzoyl]-1-(4-fluorophényl)pyrazole et
le 5-amino-1-(4-fluorophényl)-4-(3-pyridin-3-yl-benzoyl)pyrazole.

34. Composé selon la revendication 1 et choisi dans le groupe constitué par :
le 5-amino-1-(2-méthylphényl)-4-[3-pyridin-3-yl)benzoyl]pyrazole
le 5-amino-1-(2-méthylphényl)-4-[3-(N-oxydo-pyridin-3-yl)benzoyl]pyrazole,
le 5-amino-4-[3-(2,3-dihydroxypropoxy)benzoyl]-1-(4-fluorophényl)pyrazole,
le 5-amino-4-[3-(1,2-dihydroxyéthyl)benzoyl]-1-(4-fluorophényl)pyrazole,
le 5-amino-1-(4-fluorophényl)-4-[3-(sulfamoyl-benzoyl]pyrazole,
le 5-amino-1-(4-fluorophényl)-4-[3-(3-hydroxy-3-méthylbutyl)benzoyl]pyrazole,
le 5-amino-1-(4-fluorophényl)-4-[3-(2-(1-hydroxy-cyclopentyl)éthyl)benzoyl]pyrazole,
le 5-amino-4-[3-(2-méthylsulfonyléthyl)benzoyl]-1-(4-fluorophényl)pyrazole
et
le 5-amino-1-(2,4-difluorophényl)-4-[3-(2-hydroxy-éthylsulfonyl)benzoyl]pyrazole.

35. Composé selon la revendication 34, qui est :
le 5-amino-4-[3-{2(S),3-dihydroxypropoxy}benzoyl]-1-(4-fluorophényl)pyrazole.

36. Composé selon la revendication 34, qui est :
le 5-amino-4-[3-{2(R),3-dihydroxypropoxy)benzoyl]-1-(4-fluorophényl)pyrazole.

37. Procédé de préparation d'un composé de Formule (I) choisi parmi les composés selon la revendication 1, lequel procédé comprend les étapes consistant à :
(i) faire réagir un 2-céto-3-phénylaminoacrylonitrile de Formule 1 : 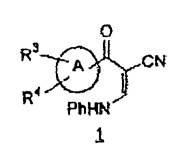 avec une hydrazine de Formule 2 : 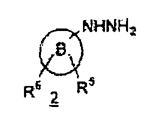 où R³, R⁴, R⁵ et R⁶ sont tels que définis dans la revendication 1 pour fournir un composé de Formule (I) où R¹ représente un atome d'hydrogène ; ou
(ii) faire réagir un 2-céto-3-phénylaminoacrylonitrile de Formule 3 : 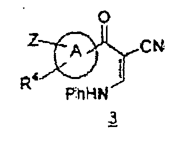 où Z représente un groupe soit hydroxy soit nitro soit halogéno et R⁴ est tel que défini dans la revendication 1 avec une hydrazine de Formule 2 pour fournir un composé de Formule 4 : 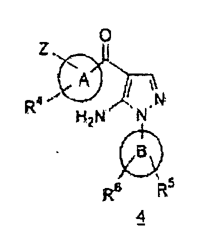 ceci suivi d'une conversion du groupe Z en groupe R³ souhaité pour fournir un composé de Formule (I) où R¹ représente un atome d'hydrogène ;
(iii) modifier éventuellement l'un quelconque des groupes R¹, R³, R⁴, R⁵ ou R⁶ ;
(iv) convertir éventuellement le composé de Formule (I) préparé dans les étapes (i), (ii) ou (iii) ci-dessus, en sel d'addition d'acide correspondant par traitement avec un acide ;
(v) convertir éventuellement le composé de Formule (I) préparé dans les étapes (i), (ii) ou (iii) ci-dessus en base libre correspondante par traitement avec une base ; et
(vi) séparer éventuellement un mélange de stéréo-isomères d'un composé de Formule (I) préparé dans les étapes (i) à (v) ci-dessus, pour donner un stéréo-isomère unique.

38. Procédé de préparation d'un composé de Formule (I) choisi parmi les composés selon la revendication 1, lequel procédé comprend la mise à réagir d'un composé de Formule 5 : 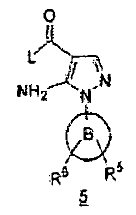 où R⁵ et R⁶ sont selon la revendication 1 et L représente un groupe libérable dans des conditions réactionnelles de déplacement organométallique
avec un réactif organométallique formule 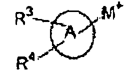 où R³ et R⁴ sont selon la revendication l et M représente un radical métallique pour fournir un composé de Formule (I) où R¹ représente un atome d'hydrogène ;
(ii) modifier éventuellement l'un quelconque des groupes R¹, R³, R⁴, R⁵ ou R⁶ ;
(iii) convertir éventuellement le composé de Formule (I) préparé dans les étapes (i) ou (ii) ci-dessus, en sel d'addition d'acide correspondant par traitement avec un acide ;
(iv) convertir éventuellement le composé de Formule (I) préparé dans les étapes (i) ou (ii) ci-dessus en base libre correspondante par traitement avec une base ; et
séparer éventuellement un mélange de stéréo-isomères d'un composé de Formule (I) préparé dans les étapes (i) à (iv) ci-dessus, pour donner un stéréo-isomère unique.

39. Composé de formule 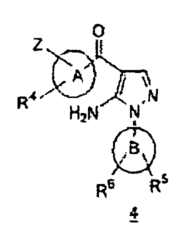 dans laquelle Z représente un groupe soit hydroxy soit nitro soit halogéno et A, B, R⁴, R⁵ et R⁶ sont tels que définis dans la revendication 1 ; les composés de cette formule où A représente un cycle phényle, R⁴ représente un atome d'hydrogène, Z représente un groupe p-chloro, o-chloro ou p-nitro, B représente un cycle phényle, R⁵ et R⁶ représentent un atome d'hydrogène ou R⁵ représente un atome d'hydrogène et R⁶ représente un groupe méthyle ou R⁵ représente un groupe méthyle et R⁶ représente un atome d'hydrogène étant exclus.

40. Composé selon l'une quelconque des revendications 1 à 36 pour une utilisation en tant que médicament.

41. Médicament contenant un composé selon l'une quelconque des revendications 1 à 36 et un excipient pharmaceutiquement acceptable.

42. Médicament selon la revendication 41 pour le contrôle ou la prévention de maladies inflammatoires.

43. Procédé de fabrication de médicaments, lequel procédé comprend les étapes consistant à mettre un composé selon l'une quelconque des revendications 1 à 36 conjointement avec un excipient pharmaceutiquement acceptable et amener le mélange sous une forme galénique d'administration.

44. Utilisation d'un composé selon l'une quelconque des revendications 1 à 36, pour la fabrication d'un médicament destiné au traitement et à la prophylaxie de maladies inflammatoires.

45. Composé selon l'une quelconque des revendications 1 à 36, à chaque fois préparé selon un procédé selon la revendication 37 ou la revendication 38.
